# EUROPEAN PATENT SPECIFICATION

(11) **EP 3 556 769 B1**
(45) Date of publication and mention of the grant of the patent: **14.06.2023**
(21) Application number: 17880389.6
(22) Date of filing: 15.12.2017
(51) Int. Cl.: C07K 7/62, A61K 38/12, A61P 31/04, A61K 38/00

(54) **POLYMYXIN DERIVATIVE, PREPARATION METHOD AND APPLICATION THEREOF**
POLYMYXINDERIVAT, HERSTELLUNGSVERFAHREN UND ANWENDUNG DAVON
DÉRIVÉ DE POLYXIMINE, PROCÉDÉ DE PRÉPARATION ET APPLICATION ASSOCIÉS

(30) Priority: 16.12.2016 CN 201611168114
(43) Date of publication of application: 23.10.2019
(73) Proprietor: Institute of Medicinal Biotechnology, Chinese Academy of Medical Sciences, Beijing 100050 (CN)
(72) Inventor: CUI, Along, Beijing 100050 (CN); LI, Zhuorong, Beijing 100050 (CN); JIN, Jie, Beijing 100050 (CN); GAO, Yan, Beijing 100050 (CN); HU, Xinxin, Beijing 100050 (CN); YOU, Xuefu, Beijing 100050 (CN); CHEN, Yang, Beijing 100050 (CN); HE, Qiyang, Beijing 100050 (CN)
(74) Representative: HGF
(86) International application number: PCT/CN2017/116484
(87) International publication number: WO 2018/108154

(56) References cited:
- WO-A1-2010/130007
- WO-A1-2015/149131
- WO-A1-2016/083531
- WO-A1-2016/113470
- CN-A- 101 010 336
- DE-A1- 2 204 887
- TW-A- 201 035 111
- US-A1- 2006 004 185
- US-A1- 2009 215 677
- US-A1- 2012 316 105
- TONY VELKOV ET AL: "Structure-Activity Relationships of Polymyxin Antibiotics", JOURNAL OF MEDICINAL CHEMISTRY, vol. 53, no. 5, 11 March 2010 (2010-03-11) , pages 1898-1916, XP055043339, ISSN: 0022-2623, DOI: 10.1021/jm900999h
- Bo Yun ET AL: "Cellular Uptake and Localization of Polymyxins in Renal Tubular Cells Using Rationally Designed Fluorescent Probes", Antimicrobial agents and chemotherapy, 1 December 2015 (2015-12-01), pages 7489-7496, XP055709300, United States DOI: 10.1128/AAC.01216-15 Retrieved from the Internet: URL:https://aac.asm.org/content/aac/59/12/ 7489.full.pdf [retrieved on 2020-06-26]
- Zahra Kassamali ET AL: "Microbiological Assessment of Polymyxin B Components Tested Alone and in Combination", Antimicrobial agents and chemotherapy, 1 December 2015 (2015-12-01), pages 7823-7825, XP055709307, United States DOI: 10.1128/AAC.01021-15 Retrieved from the Internet: URL:https://aac.asm.org/content/aac/59/12/ 7823.full.pdf [retrieved on 2020-06-26]
- MARTTI VAARA ET AL: "Polymyxins and their novel derivatives", CURRENT OPINION IN MICROBIOLOGY, vol. 13, no. 5, 1 October 2010 (2010-10-01), pages 574-581, XP055397178, GB ISSN: 1369-5274, DOI: 10.1016/j.mib.2010.09.002
- SAKURA N ET AL: "The contribution of the N-terminal structure of polymyxin B peptides to antimicrobial and lipopolysaccharide binding activity", BULLETIN OF THE CHEMICAL SOCIETY OF JAPAN, CHEMICAL SOCIETY OF JAPAN, TOKYO, JP, vol. 77, no. 10, 1 January 2004 (2004-01-01), pages 1915-1924, XP009094340, ISSN: 0009-2673, DOI: 10.1246/BCSJ.77.1915
- TONY VELKOV ET AL: "Teaching 'Old' Polymyxins New Tricks: New-Generation Lipopeptides Targeting Gram-Negative 'Superbugs'", ACS CHEMICAL BIOLOGY, vol. 9, no. 5, 16 May 2014 (2014-05-16), pages 1172-1177, XP055227897, ISSN: 1554-8929, DOI: 10.1021/cb500080r
- JUN''ICHI SHOJI ET AL: "The structures of two new polymyxin group antibiotics.", THE JOURNAL OF ANTIBIOTICS, vol. 30, no. 5, 1 January 1977 (1977-01-01), pages 427-429, XP055752261, GB ISSN: 0021-8820, DOI: 10.7164/antibiotics.30.427
- ALEJANDRA GALLARDO-GODOY ET AL: "Activity and Predicted Nephrotoxicity of Synthetic Antibiotics Based on Polymyxin B", JOURNAL OF MEDICINAL CHEMISTRY, vol. 59, no. 3, 6 January 2016 (2016-01-06), pages 1068-1077, XP055264468, ISSN: 0022-2623, DOI: 10.1021/acs.jmedchem.5b01593

## Description

### Technical field

The present invention relates to polymyxin derivatives and preparation methods thereof, and the use of the prepared compounds for the production of antibacterial agents, in particular for those with expended antibacterial spectra, increased antibacterial activities, as well as reduced nephrotoxicities, including the use in the preparation of antibacterial agents against "superbugs" carrying the NDM-1 gene, as well as pharmaceutical compositions containing such compounds as active ingredients. This invention belongs to the field of biomedicine.

### Background of the technique

Polymyxin was discovered in 1947, it is a general term for a series of cationic antibacterial peptides produced by Bacillus polymyxa. It has different types of structures, for example, types A, B, C, D, E, F, K, M, P, S and T. Their molecular weights are around 1200D. The common structural features of polymyxins are: consisting of a cyclic heptapeptide, a linear tripeptide, and a side acyl chain linked to the linear tripeptide, wherein the heptapeptide ring is composed of the position-4 amino acid L-Dab (α, γ-diaminobutyric acid), condensed with position-10 amino acid L-Thr (or L-Leu). The main difference between different types of structures lies in the difference of amino acids at the 3, 6, 7 or 10 positions. Their antibacterial spectra are similar. By changing the cell membrane permeability of Gram-negative bacteria, the leakage of intracellular substances leads to bactericidal action.

Polymyxins have narrow antibacterial spectra. They are only effective against Gram-negative bacteria, besides, they have certain nephrotoxicity. Especially after the emergence of new broad-spectrum antibacterial drugs for example, third-generation cephalosporins and carbapenems, their clinical use is gradually decreasing. Because in recent years, polymyxin has been found to be effective in the treatment of infections caused by multidrug-resistant Acinetobacter baumannii, Pseudomonas aeruginosa and Klebsiella pneumoniae, they received clinical attention.

Currently, polymyxin B and colistin (polymyxin E) are used clinically, both of which are multi-component mixtures obtained by bacterial fermentation. According to the Chinese Pharmacopoeia (2015 edition) specification of polymyxin B, the content of polymyxin B3 should not exceed 6.0%, the content of polymyxin B1-Ile should not exceed 15.0%. The total content of polymyxin B1, B2, B3 and B1-Ile shall not be less than 80.0%. At present, the compositions of polymyxin in clinical use is complex, the relative contents are uncertain, and they have certain nephrotoxicity and neurotoxicity, which brings safety hazards to clinical medication. Therefore, it is particularly urgent to prepare single-component polymyxins and polymyxin derivatives and to study the biological functions of the polymyxins and polymyxin derivatives.

Regarding the chemical preparation method of polymyxin compounds, only those for polymyxin B and E has been reported in the literature, those for other polymyxin compounds are first reported in the present invention. The chemical preparation method of polymyxin B reported in the literature adopted solid phase condensation and liquid phase cyclization strategy. (Sharma SK, Wu AD, Chandramouli N, et a1. Solid-phase total synthesis of polymyxin B1. J Pept Res , 1999, 53(5): 501-506, and Magee TV, Brown MF, Starr JT, et al. Discovery of Dap-3 polymyxin analogues for the treatment of multidrug-resistant Gram-negative nosocomial infections. J Med Chem, 2013, 56(12): 5079-5093). In the reported method, a large amount of solvent is required for liquid phase cyclization, the product is not easily separated and purified, and the yield is about 20%, and the yield in the actual synthesis process is even lower. The polymyxin B1 synthesized by solid phase condensation and solid phase cyclization using Kenner's safety catch method was reported in literature (de Visser PC, Kriek NM, van Hooft PA, et al. Solid-phase synthesis of polymyxin B1 and analogues via a safety-catch approach. J Pept Res, 2003, 61(6): 298-306), but the total yield was 1.5%. Preparation methods of polymyxin B2 and E2 using solid phase condensation and solid phase cyclization were reported in literature (Wei-Liang Xu, A-Long Cui, Xin-Xin Hu, et al. A new strategy for total solid-phase synthesis of polymyxins. Tetrahedron Letters, 2015, 56(33): 4796-4799.), with a yield of about 25%.

WO2013156977A1 reported a method for solid phase synthesis of insulin by a lysine side chain amino linking resin. This invention employs solid phase condensation and solid phase cyclization method by using a protected basic amino acid similar in structure to lysine in Fmoc-AA-OP side chain amino linking resin, to synthesize polymyxin derivatives. In literature (Wei-Liang Xu, A-Long Cui, Xin-Xin Hu, et al. A new strategy for total solid-phase synthesis of polymyxins. Tetrahedron Letters, 2015, 56(33): 4796-4799.) HCTU/DIEA is used as a condensing agent. In the condensation process, DIEA enolizes the β-carbonyl group of the side chain carboxylic acid CH₃(CH₂)ₙCOCH₂COOH of compound 3-7, which is prone to CH₃(CH₂)ₙCOCH₂COOH intermolecular condensation reaction, compound 3-7 could not be obtained. The present synthesis method uses DIC/HOBT as a condensing agent, and it is not easy to generate an intermolecular condensation reaction of CH₃(CH₂)ₙCOCH₂COOH, thereby being able to obtain compound 3-7 without addition of a base as a catalyst. The method has wide application range, avoids a large consmption of solvent by using liquid phase cyclization, is environmentally friendly, has high purity of crude polypeptide, the latter is easy to be separated and purified, the total yield is up to 40%.

Regarding the structural study of the natural components of polymyxins, the structural types of polymyxins A, B, D, E, M, P, S and T of natural origin are currently identified. The structure of many polymyxin natural products that have appeared in the literature has not been completely clarified, or the structure has been proved to be wrong. For example, the amino acid configuration of polymyxin C and F, as well as the structure of side chain acyl group of polymyxin K are all uncertain. Polymyxin A and M were originally thought to be compounds of the same structure. Later sdudy found that the position-3 amino acid configurations of polymyxin A and M were different, and so on (Terabe S, Konaka R, Shoji. J. Separation of polymyxins and octapeptins by high-performance liquid chromatography. J. Chromatogr. A. 1979, 173(2): 313-320. Shoji J, Hinoo H, Wakisaka Y, et al. Isolation of two new polymyxin group antibiotics. Studies on antibiotics from the genus Bacillus. XX). J Antibiot (Tokyo). 1977, 30(12): 1029-1034.). For the first time, this invention systematically synthesized single components with clarified structures in the polymyxin mixture of different structure types.

Regarding the study of the biological function of the single components of polymyxin antibiotics, the proportion of the main polymyxin components in clinical use is different among different brands. There are also dicrepancies of main component proportions even among the different batches of the same brand, resulting in instability of clinical efficacy (He J, Ledesma KR, Lam WY, et al. Variability of polymyxin B major components in commercial formulations. Int J Antimicrob Agents. 2010, 35(3): 308-310. He H, Li JC, Nation RL, et al. Pharmacokinetics of four different brands of colistimethate and formed colistin in rats. J Antimicrob Chemother. 2013, 68(10): 2311-2317.) The natural components of polymyxin are complex. Tam et al. obtained the polymyxin B1, B2, B3, B4 and B1-Ile by preparative liquid chromatography, and tested in vitro antibacterial activity of single components for the first time. (Tam VH, Cao H, Ledesma KR, et al. In vitro potency of various polymyxin B components. Antimicrob Agents Chemother. 2011, 55(9): 4490-4491.) Except for the main component B1, B2, E1, E2 in clinical use have reports of antibacterial activity and nephrotoxicity (Roberts KD, Azad MA, Wang J, et al. Antimicrobial Activity and Toxicity of the Major Lipopeptide Components of Polymyxin B and Colistin: Last-Line Antibiotics against Multidrug-Resistant Gram-Negative Bacteria. ACS Infect. Dis. 2015, 1(11): 568-575.), research on other components is mostly limited to reports of material discovery, for some components, even the structures were not very certain, systematic studies of the biological functions of each components are missing. The present invention is the first to study the biological function of single components of polymyxin antibiotics, in order to guide the rational and safe use of polymyxins in clinical treatment of bacterial infections.

Regarding the preparation of new derivatives of polymyxin, the present invention has for the first time prepared new derivatives with increased or decreased hydrophobicity of the side acyl chain (altering R₀) by changing the length and volume of the side acyl chain, new derivatives with basic or polar amino acid replacing position-1 and/or-3 amino acids (altering R₁ and/or R₃), new derivatives with a hydrophobic amino acid or a polar amino acid replacing position-2 and/or-10 amino acids (altering R₂ and/or R₉), new derivatives with a hydrophobic or a basic or a polar amino acid replacing position-5 and/or-8 and/or-9 amino acids (altering R₄, R₇, R₈), new derivatives with a hydrophobic amino acid or a polar amino acid replacing positions-6 and/or-7 amino acids ( Changing R₅, R₆). By changing the number of the amino groups or hydrophobicity of the polymyxin molecules, the antibacterial spectrum is increased or the antibacterial activity increased or the nephrotoxicity lowered.

Regarding the biological function of polymyxin derivatives, the present invention studies the antibacterial activity and nephrotoxicity of polymyxin derivatives. In comparison with some positive controls, some polymyxin derivatives have higher antibacterial activities against Gram-positive bacteria, some have increased antibacterial activities against Gram-negative bacteria, some show reduced nephrotoxicity.

### Summary of the invention

The invention relates to polymyxin derivatives and a preparation method thereof, in particular to a method for preparing a polymyxin derivative by solid phase condensation and solid phase cyclization. The invention also relates to polymyxin derivatives and their use as antibacterial agents, in particular as antibacterial agents with expanded antibacterial spectra, increased antibacterial activity and decreased nephrotoxicity, including their use as antibacterial drugs against "superbug" carrying the NDM-1 gene, as defined by the appended set of claims.

In the present invention, the polymyxin derivative is compound 116. Compounds 1 to 115 and 117 to 152 are disclosed, but not claimed.
(1) 6-methoxyhexanoyl-Dab-Thr-Dab-ring(4-10)[Dab-Dab-D-Phe-Leu-Dab-Dab-Thr] 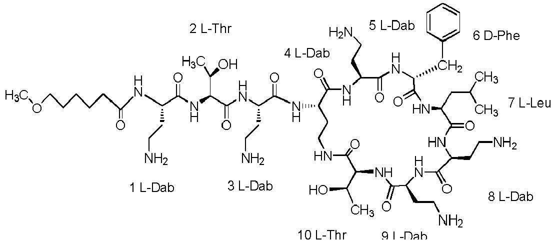
*(2) N, N*-dimethylaminovaleryl-Dab-Thr-Dab-ring(4-10)[Dab-Dab-D-Phe-Leu-Dab-Dab-Thr] 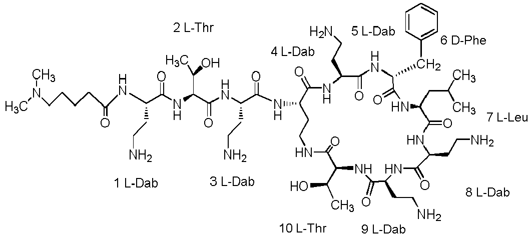
(3) 3-oxohexanoyl-Dab-Thr-Dab-ring(4-10)[Dab-Dab-D-Phe-Leu-Dab-Dab-Thr] 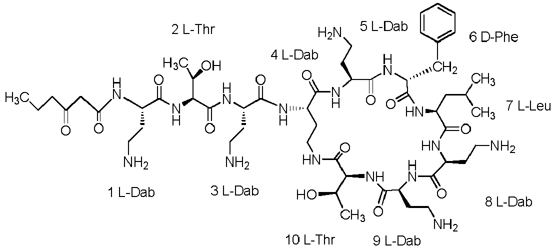
(4) 3-oxoheptanoyl-Dab-Thr-Dab-ring(4-10)[Dab-Dab-D-Phe-Leu-Dab-Dab-Thr] 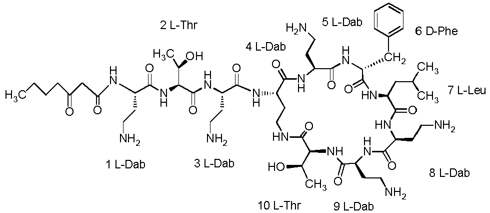
(5) 3-oxooctanoyl-Dab-Thr-Dab-ring(4-10)[Dab-Dab-D-Phe-Leu-Dab-Dab-Thr] 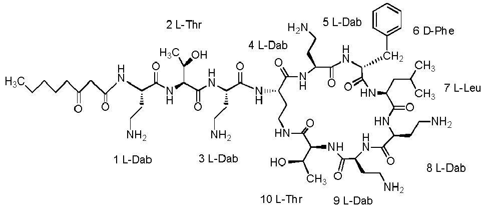
(6) 3-oxononanoyl-Dab-Thr-Dab-ring(4-10)[Dab-Dab-D-Phe-Leu-Dab-Dab-Thr] 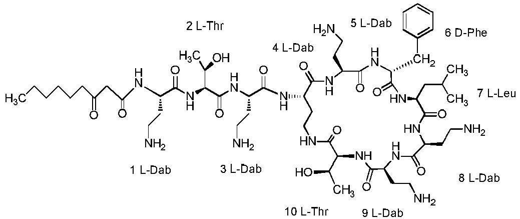
(7) 3-oxodecanoyl-Dab-Thr-Dab-ring(4-10)[Dab-Dab-D-Phe-Leu-Dab-Dab-Thr] 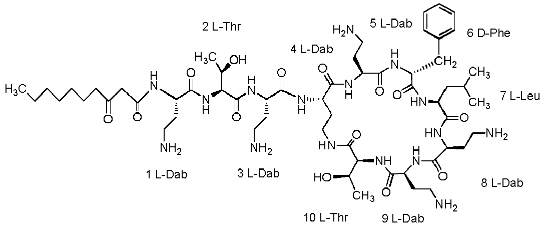
(8) 4-phenoxybenzoyl-Dab-Thr-Dab-ring(4-10)[Dab-Dab-D-Phe-Leu-Dab-Dab-Thr] 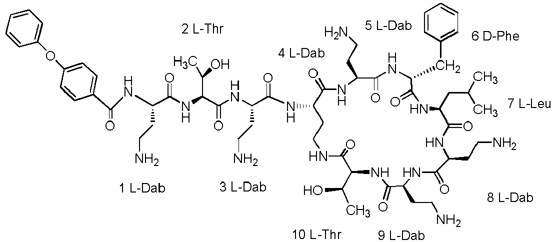
(9) 4-(p-methylphenoxy)benzoyl-Dab-Thr-Dab-ring(4-10)[Dab-Dab-D-Phe-Leu-Dab-Dab-Thr] 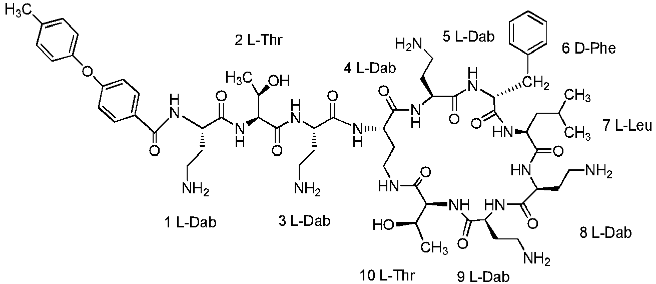
(10) 3-hydroxyl-6-methyloctanoyl-Dab-Thr-Dab-ring(4-10)[Dab-Dab-D-Phe-Leu-Dab-Dab-Thr] 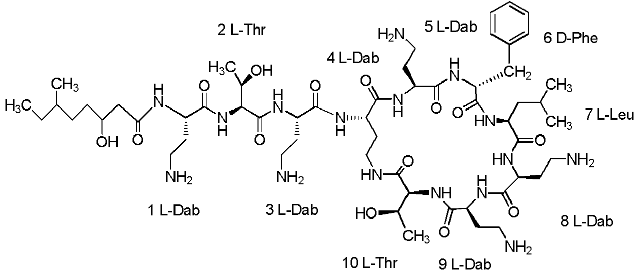
(11) (S)-6-methyloctanoyl-Dab-Thr-Dab-ring(4-10)[Dab-Dab-D-Phe(4-NH₂)-Leu-Dab-Dab-Thr] 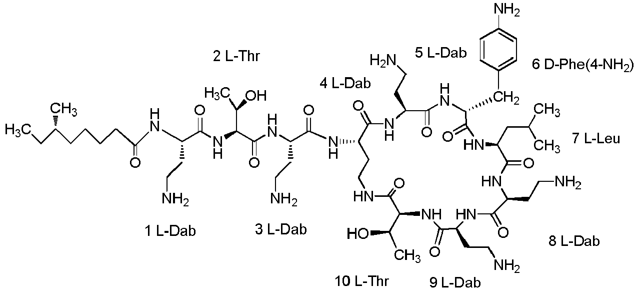
(12) (S)-6-methyloctanoyl-Dab-Thr-Dab-ring(4-10)[Dab-Dab-D-Tyr-Leu-Dab-Dab-Thr] 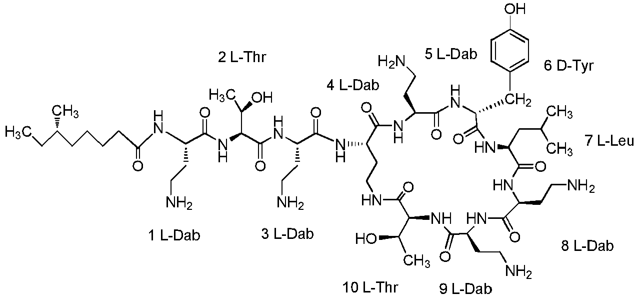
(13) (S)-6-methyloctanoyl-Dab-Thr-Dab-ring(4-10)[Dab-Dab-D-Phe(4-CN)-Leu-Dab-Dab-Thr] 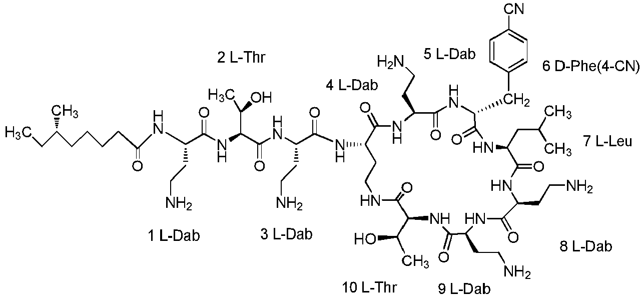
(14) (S)-6-methyloctanoyl-Dab-Thr-Dab-ring(4-10)[Dab-Dab-D-Phe(4-NO₂)-Leu-Dab-Dab-Thr] 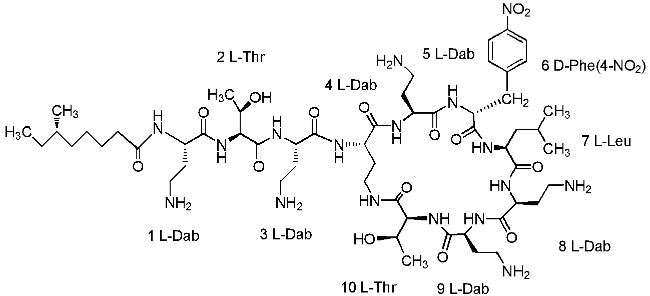
(15) (S)-6-methyloctanoyl-Dab-Thr-Dab-ring(4-10)[Dab-Dab-D-Phe(4-F)-Leu-Dab-Dab-Thr] 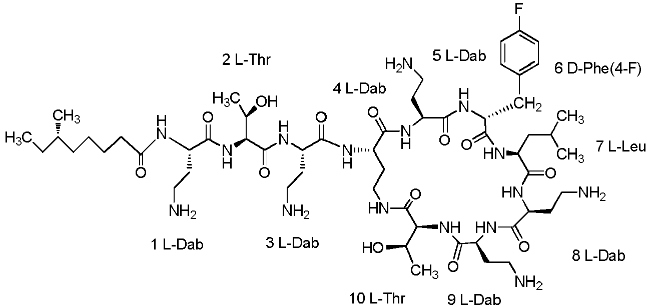
(16) (S)-6-methyloctanoyl-Dab-Thr-Dab-ring(4-10)[Dab-Dab-D-Phe(4-Cl)-Leu-Dab-Dab-Thr] 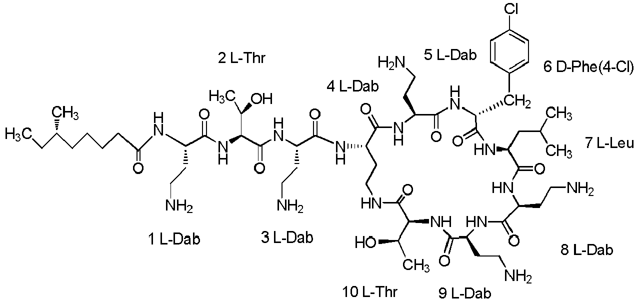
(17) (S)-6-methyloctanoyl-Dab-Thr-Dab-ring(4-10)[Dab-Dab-D-Phe(4-Br)-Leu-Dab-Dab-Thr] 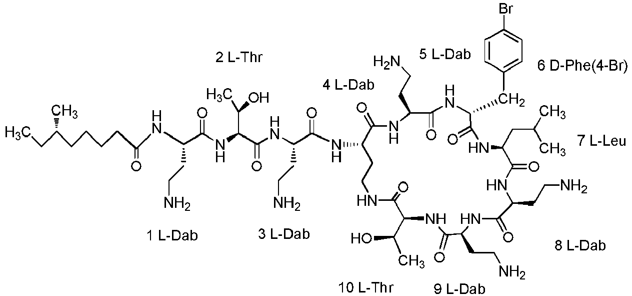
(18) (S)-6-methyloctanoyl-Dab-Thr-Dab-ring(4-10)[Dab-Dab-D-Phe(2-Cl)-Leu-Dab-Dab-Thr] 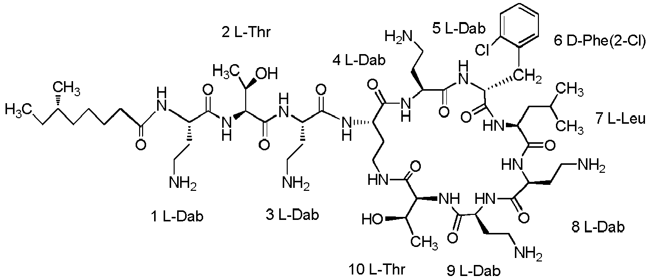
(19) (S)-6-methyloctanoyl-Dab-Thr-Dab-ring(4-10)[Dab-Dab-D-Phe(3-Cl)-Leu-Dab-Dab-Thr] 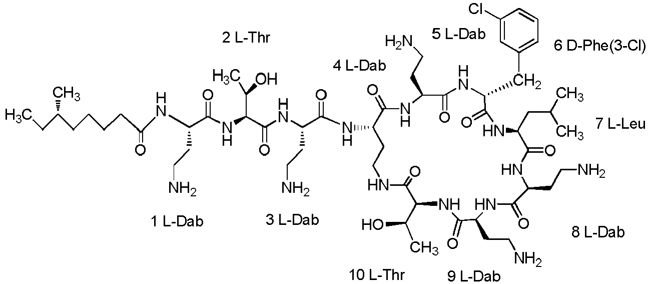
(20) (S)-6-methyloctanoyl-Dab-Thr-Dab-ring(4-10)[Dab-Dab-D-Phe(2,4-dichloro)-Leu-Dab-Dab-Thr] 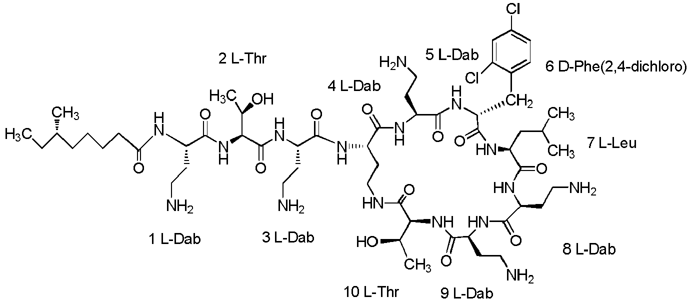
(21) (S)-6-methyloctanoyl-Dab-Thr-Dab-ring(4-10)[Dab-Dab-D-Phe(2,3-dichloro)-Leu-Dab-Dab-Thr] 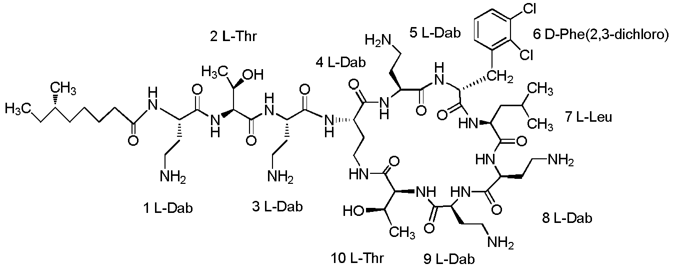
(22) (S)-6-methyloctanoyl-Dab-Thr-Dab-ring(4-10)[Dab-Dab-D-Phe(3,4-dichloro)-Leu-Dab-Dab-Thr] 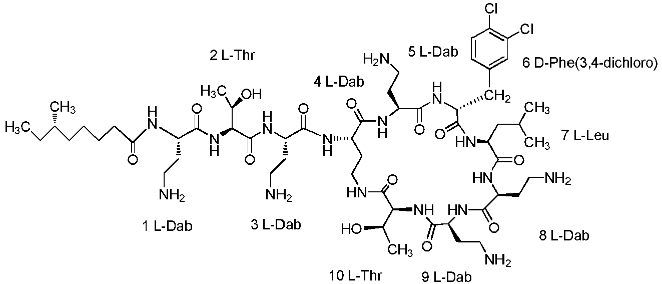
(23) (S)-6-methyloctanoyl-Dab-Thr-Dab-ring(4-10)[Dab-Dab-D-Phe(4-CF₃)-Leu-Dab-Dab-Thr] 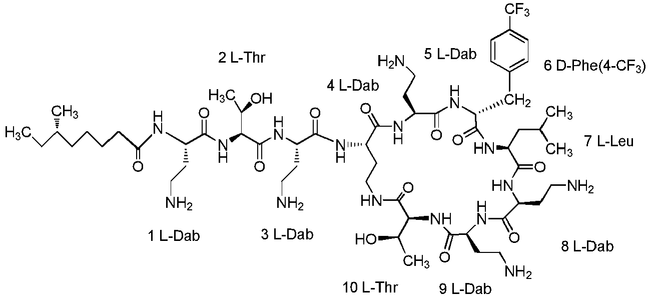
(24) (S)-6-methyloctanoyl-Dab-Thr-Dab-ring(4-10)[Dab-Dab-D-Phe(4-OCH₃)-Leu-Dab-Dab-Thr] 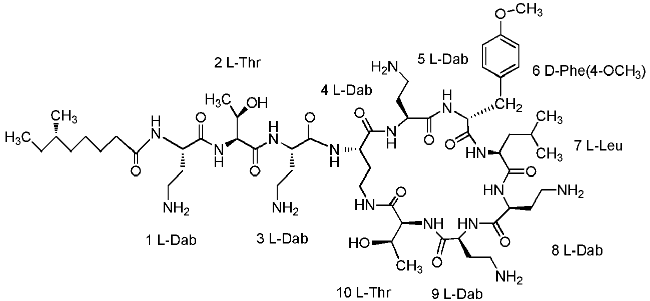
(25) (S)-6-methyloctanoyl-Dab-Thr-Dab-ring(4-10)[Dab-Dab-D-Phe(4-OEt)-Leu-Dab-Dab-Thr] 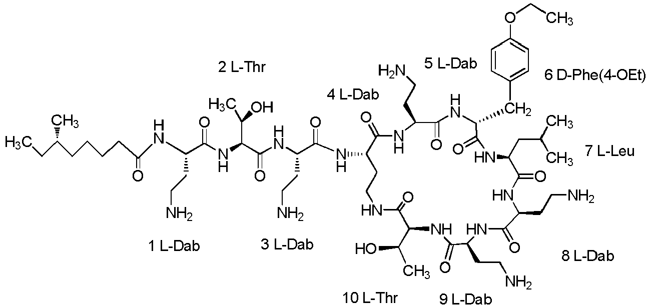
(26) (S)-6-methyloctanoyl-Dab-Thr-Dab-ring(4-10)[Dab-Dab-D-Phe(4-CH₃)-Leu-Dab-Dab-Thr] 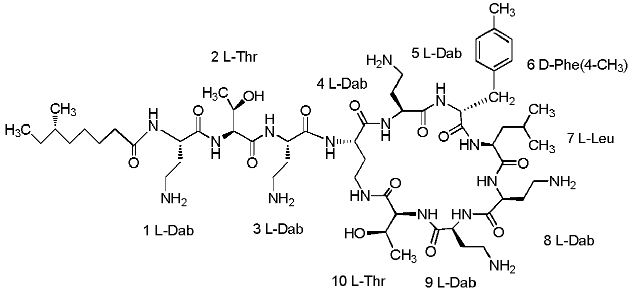
(27) (S)-6-methyloctanoyl-Dab-Thr-Dab-ring(4-10)[Dab-Dab-D-Phe(4-tBu)-Leu-Dab-Dab-Thr] 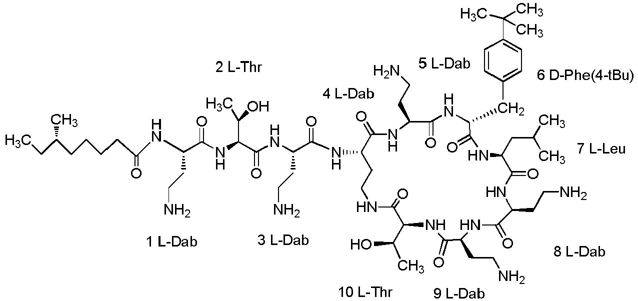
(28) (S)-6-methyloctanoyl-Dab-Thr-Dab-ring(4-10)[Dab-Dab-D-Phe(4-benzyl)-Leu-Dab-Dab-Thr] 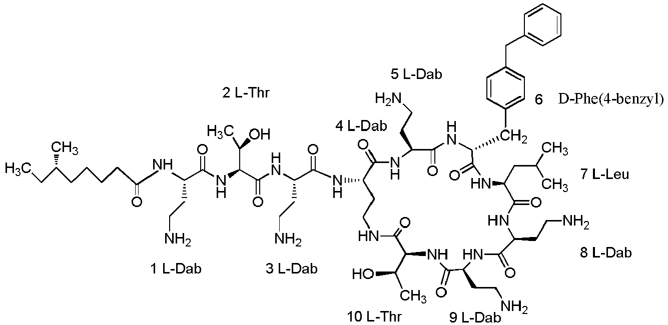
(29) (S)-6-methyloctanoyl-Dab-Thr-Dab-ring(4-10)[Dab-Dab-D-Phe(4-benzoyl)-Leu-Dab-Dab-Thr] 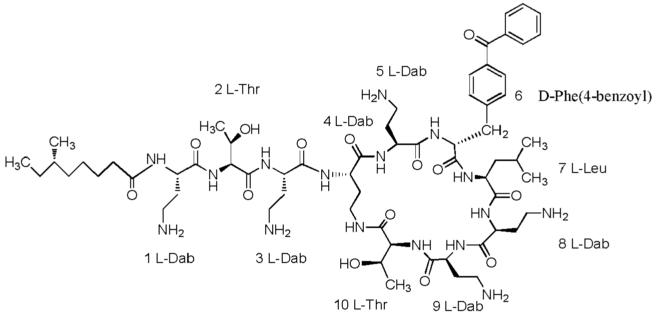
(30) (S)-6-methyloctanoyl-Dab-Thr-D-Dab-ring(4-10)[Dab-Dab-D-Leu-Thr-Dab-Dab-Thr] 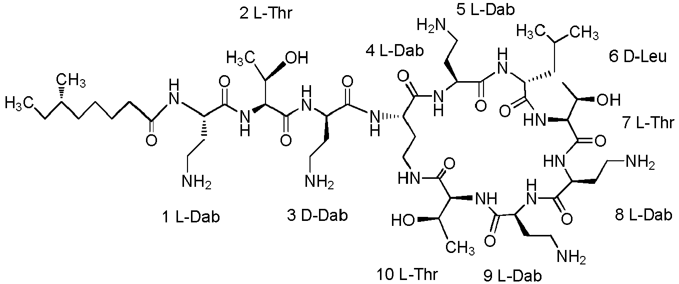
(31) 6-methylheptanoyl-Dab-Thr-D-Dab-ring(4-10)[Dab-Dab-D-Leu-Thr-Dab-Dab-Thr] 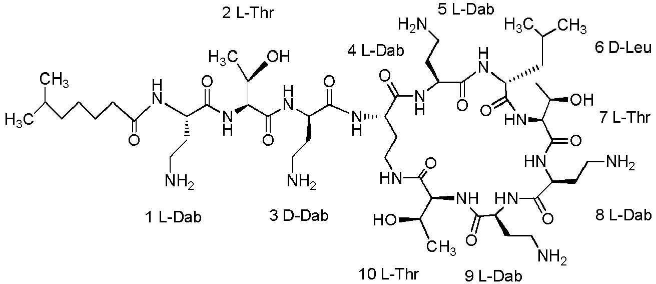
(32) Octanoyl-Dab-Thr-D-Dab-ring(4-10)[Dab-Dab-D-Leu-Thr-Dab-Dab-Thr] 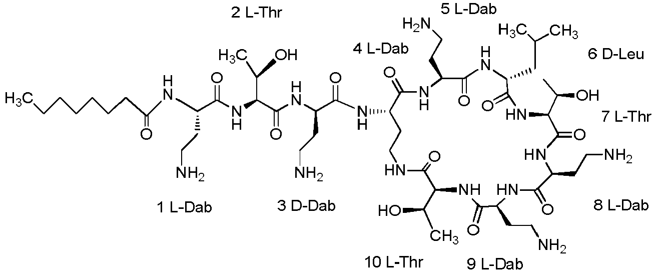
(33) Heptanoyl-Dab-Thr-D-Dab-ring(4-10)[Dab-Dab-D-Leu-Thr-Dab-Dab-Thr] 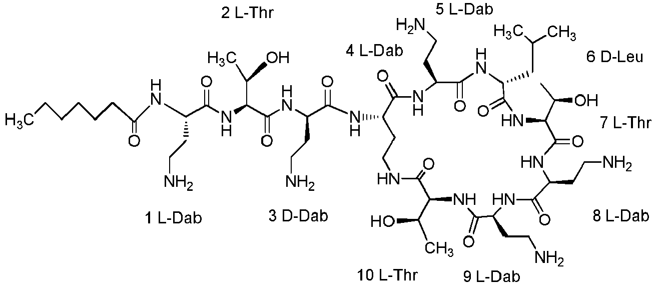
(34) Nonanoyl-Dab-Thr-D-Dab-ring(4-10)[Dab-Dab-D-Leu-Thr-Dab-Dab-Thr] 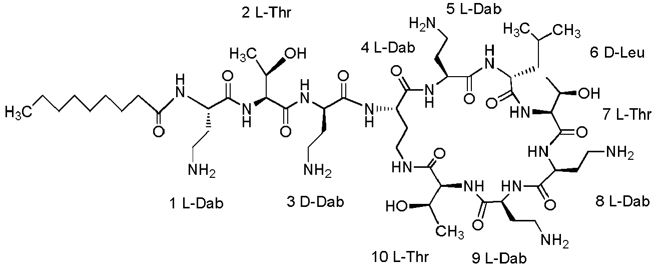
(35) Octanoyl-Dab-Ser-D-Dab-ring(4-10)[Dab-Dab-D-Leu-Thr-Dab-Dab-Thr] 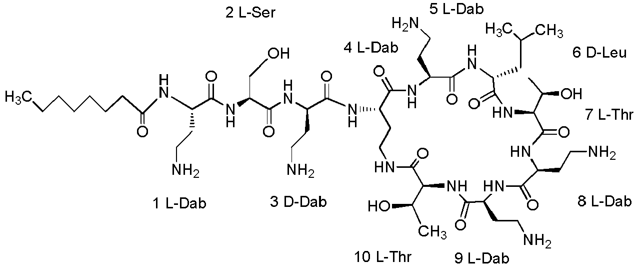
(36) Octanoyl-Dab-Thr-D-Dab-ring(4-10)[Dab-Dab-D-Leu-Ser-Dab-Dab-Thr] 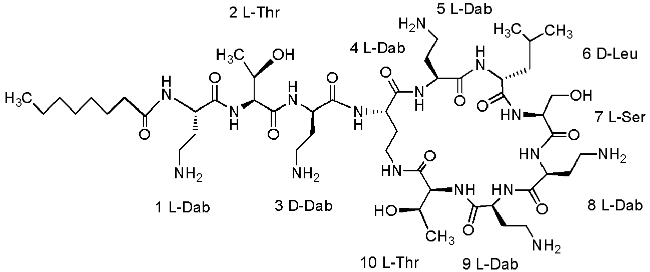
(37) Octanoyl-Dab-Thr-D-Dab-ring(4-10)[Dab-Dab-Thr-Thr-Dab-Dab-Thr] 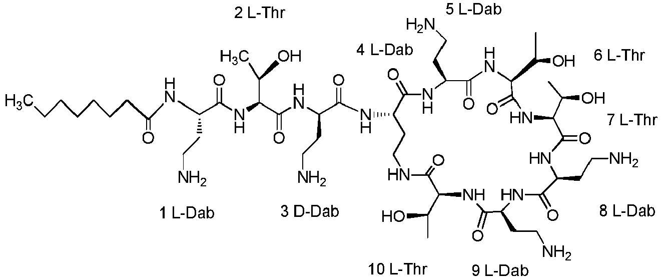
(38) Octanoyl-Ser-Thr-D-Dab-ring(4-10)[Dab-Dab-D-Leu-Thr-Dab-Dab-Thr] 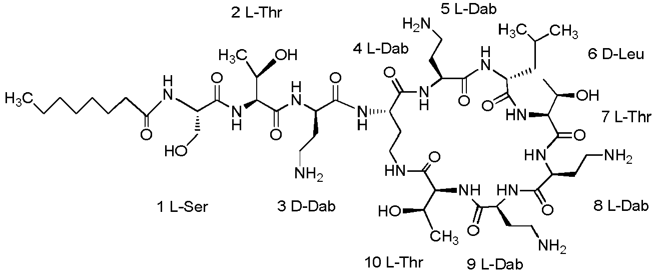
(39) Octanoyl-Dab-Thr-D-Dab-ring(4-10)[Dab-Ser-D-Leu-Thr-Dab-Dab-Thr] 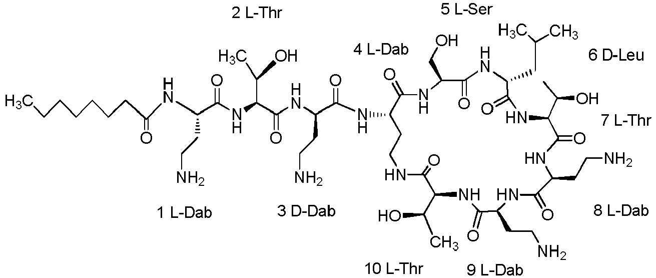
(40) Octanoyl-Dab-Thr-D-Dab-ring(4-10)[Dab-Dab-D-Leu-Thr-Ser-Dab-Thr] 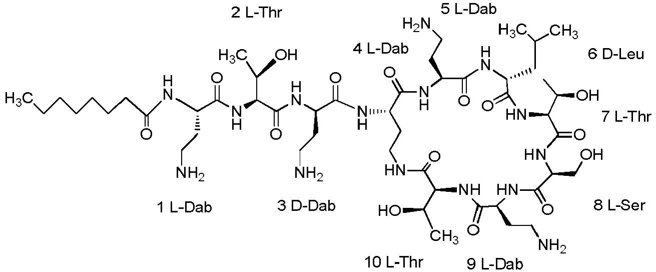
(41) Octanoyl-Dab-Thr-D-Dab-ring(4-10)[Dab-Dab-D-Leu-Thr-Dab-Ser-Thr] 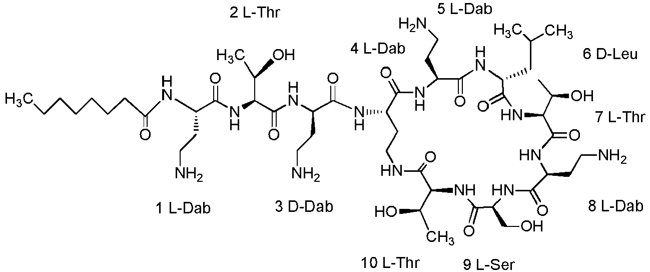
(42) (S)-6-methyloctanoyl-Dab-Thr-Dab-ring(4-10)[Dab-Dab-D-Phe-Leu-Dab-Dab-Thr] 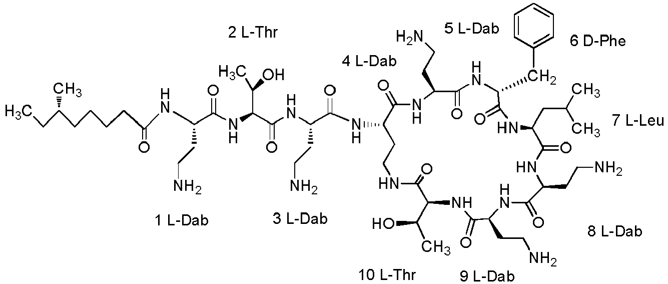
(43) 6-methylheptanoyl-Dab-Thr-Dab-ring(4-10)[Dab-Dab-D-Phe-Leu-Dab-Dab-Thr] 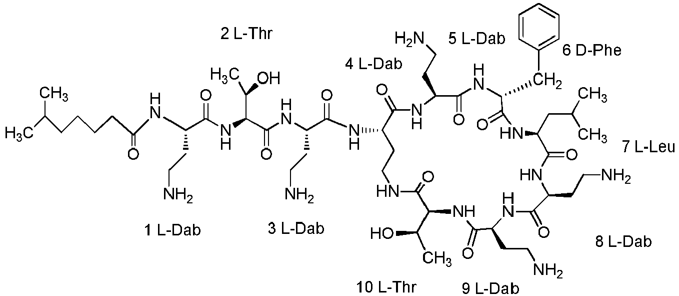
(44) Octanoyl-Dab-Thr-Dab-ring(4-10)[Dab-Dab-D-Phe-Leu-Dab-Dab-Thr] 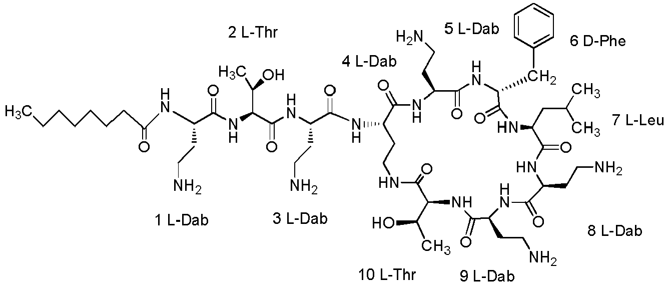
(45) Heptanoyl-Dab-Thr-Dab-ring(4-10)[Dab-Dab-D-Phe-Leu-Dab-Dab-Thr] 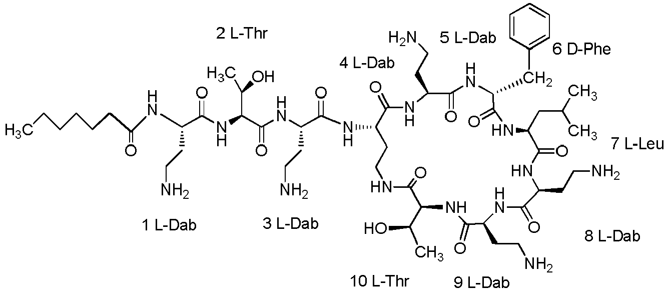
(46) Nonanoyl-Dab-Thr-Dab-ring(4-10)[Dab-Dab-D-Phe-Leu-Dab-Dab-Thr] 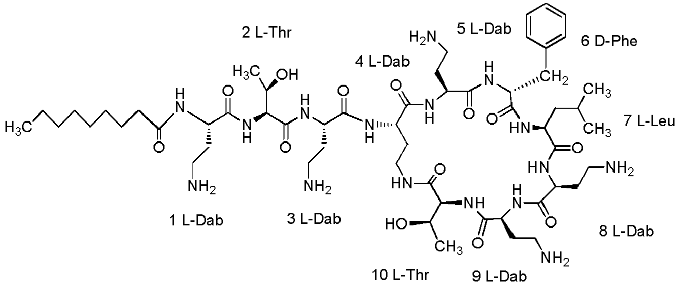
(47) (S)-6-methyloctanoyl-Dab-Thr-Dab-ring(4-10)[Dab-Dab-D-Phe-Ile-Dab-Dab-Thr] 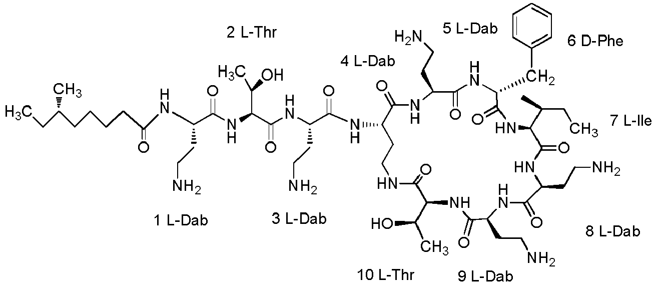
(48) (S)-6-methyloctanoyl-Dab-Thr-Dab-ring(4-10)[Dab-Dab-Thr-Leu-Dab-Dab-Thr] 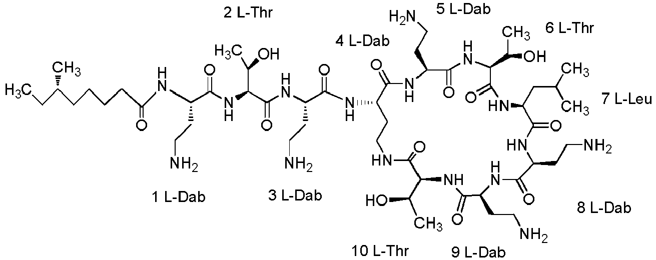
(49) Octanoyl-Dab-Ser-Dab-ring(4-10)[Dab-Dab-D-Phe-Leu-Dab-Dab-Thr] 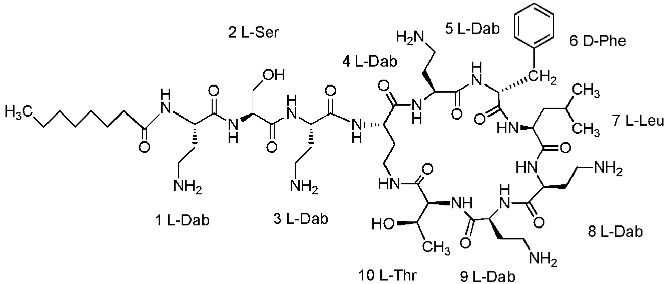
(50) Octanoyl-Dab-Thr-Dab-ring(4-10)[Dab-Dab-D-Phe-Leu-Dab-Dab-Ser] 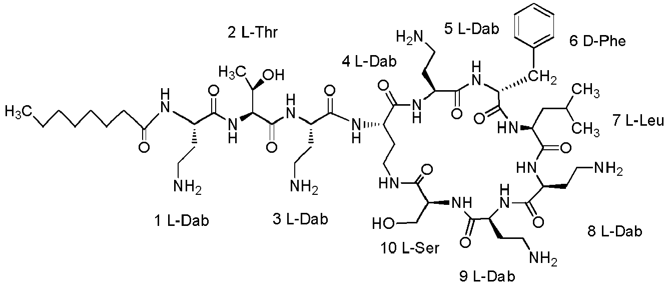
(51) Octanoyl-Dab-Ser-Dab-ring(4-10)[Dab-Dab-D-Phe-Ile-Dab-Dab-Thr] 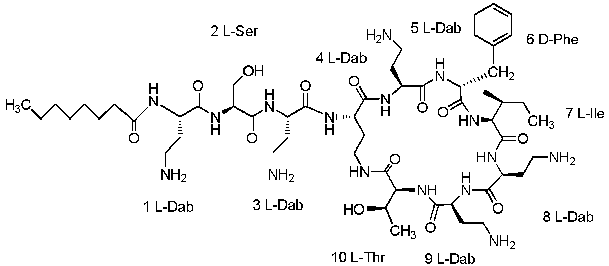
(52) Octanoyl-Dab-Thr-Dab-ring(4-10)[Dab-Dab-D-Phe-Ile-Dab-Dab-Ser] 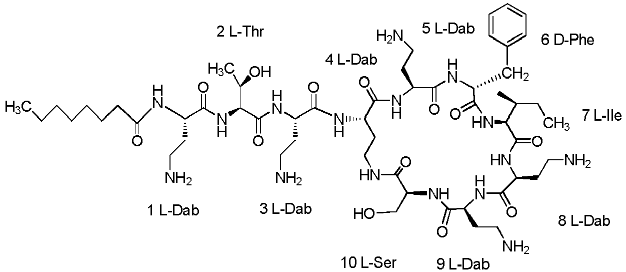
(53) Octanoyl-Ser-Thr-Dab-ring(4-10)[Dab-Dab-D-Phe-Leu-Dab-Dab-Thr] 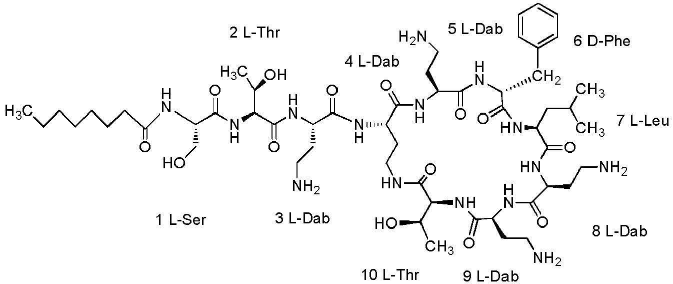
(54) Octanoyl-Dab-Thr-Ser-ring(4-10)[Dab-Dab-D-Phe-Leu-Dab-Dab-Thr] 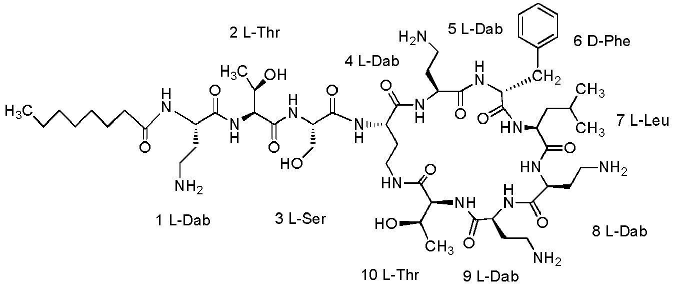
(55) Octanoyl-Dab-Thr-Dab-ring(4-10)[Dab-Ser-D-Phe-Leu-Dab-Dab-Thr] 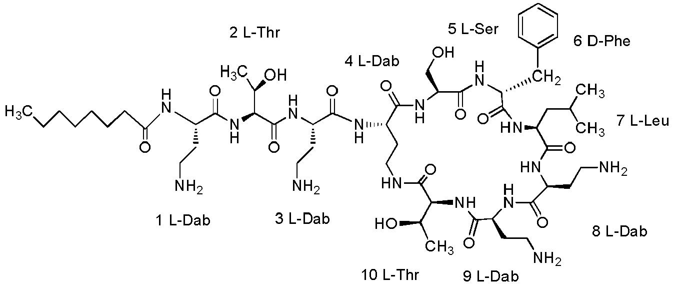
(56) Octanoyl-Dab-Thr-Dab-ring(4-10)[Dab-Dab-D-Phe-Leu-Ser-Dab-Thr] 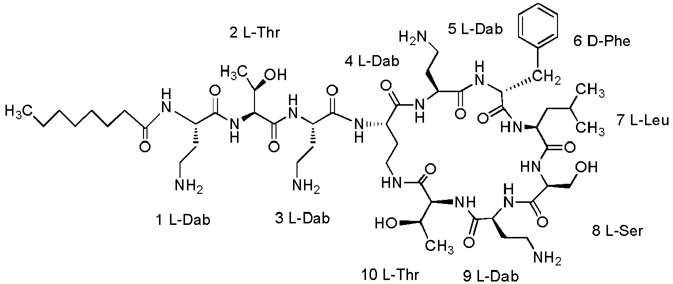
(57) Octanoyl-Dab-Thr-Dab-ring(4-10)[Dab-Dab-D-Phe-Leu-Dab-Ser-Thr] 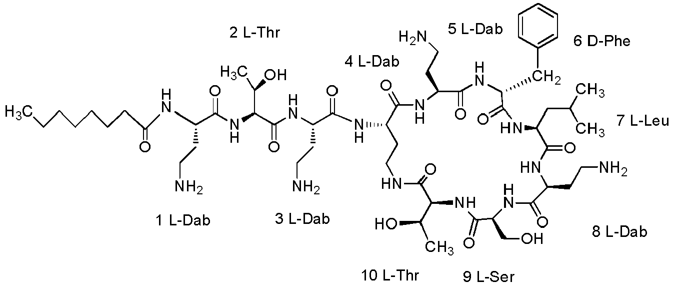
(58) (S)-6-methyloctanoyl-Dab-Thr-D-Ser-ring(4-10)[Dab-Dab-D-Leu-Thr-Dab-Dab-Thr] 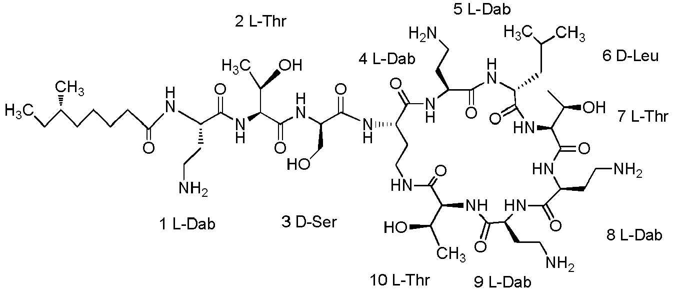
(59) 6-methylheptanoyl-Dab-Thr-D-Ser-ring(4-10)[Dab-Dab-D-Leu-Thr-Dab-Dab-Thr] 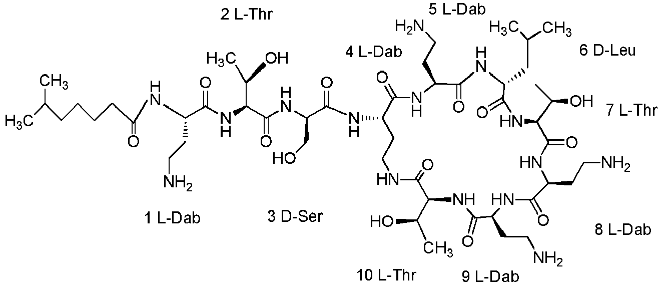
(60) Octanoyl-Dab-Thr-D-Ser-ring(4-10)[Dab-Dab-D-Leu-Thr-Dab-Dab-Thr] 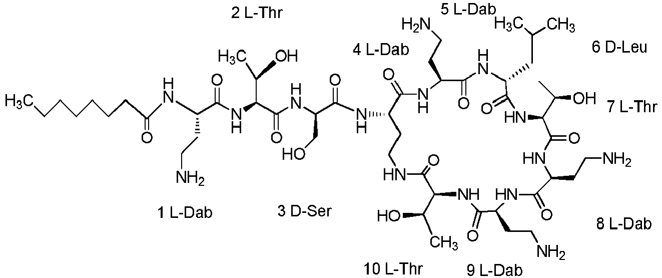
(61) Heptanoyl-Dab-Thr-D-Ser-ring(4-10)[Dab-Dab-D-Leu-Thr-Dab-Dab-Thr] 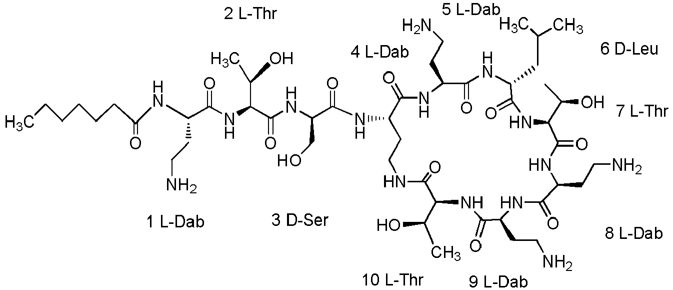
(62) Nonanoyl-Dab-Thr-D-Ser-ring(4-10)[Dab-Dab-D-Leu-Thr-Dab-Dab-Thr] 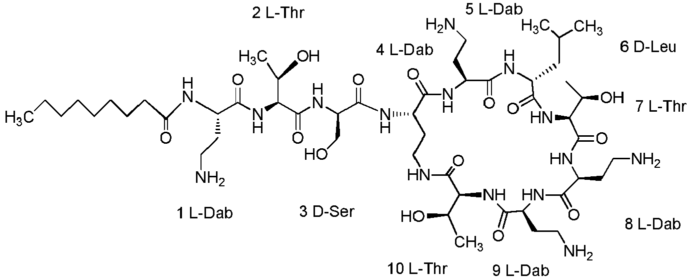
(63) Octanoyl-Dab-Ser-D-Ser-ring(4-10)[Dab-Dab-D-Leu-Thr-Dab-Dab-Thr] 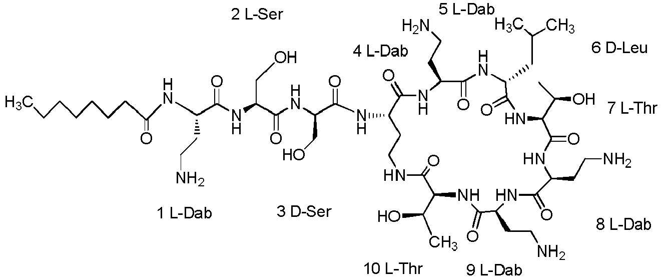
(64) Octanoyl-Dab-Thr-D-Ser-ring(4-10)[Dab-Dab-D-Leu-Ser-Dab-Dab-Thr] 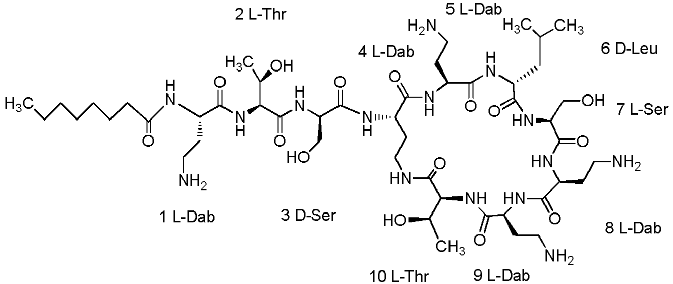
(65) Octanoyl-Dab-Thr-D-Ser-ring(4-10)[Dab-Dab-D-Leu-Thr-Dab-Dab-Ser] 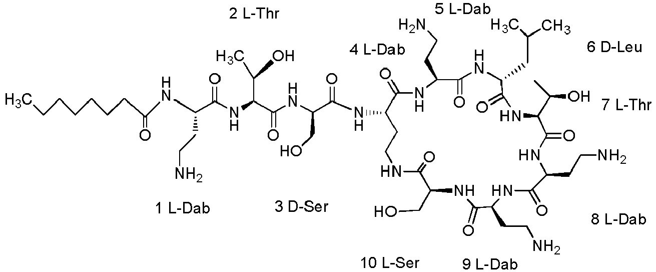
(66) Octanoyl-Ser-Thr-D-Ser-ring(4-10)[Dab-Dab-D-Leu-Thr-Dab-Dab-Thr] 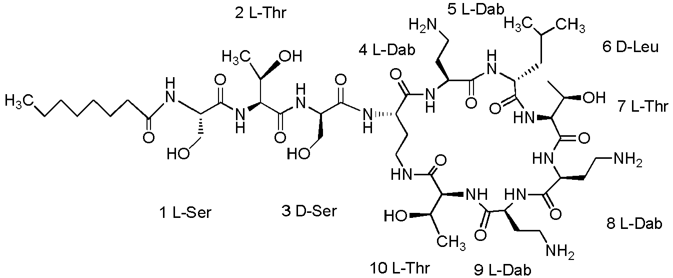
(67) Octanoyl-Dab-Thr-D-Ser-ring(4-10)[Dab-Ser-D-Leu-Thr-Dab-Dab-Thr] 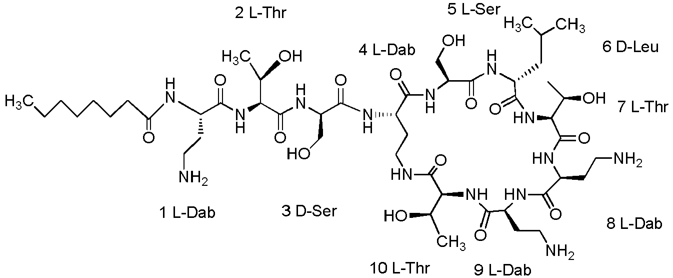
(68) Octanoyl-Dab-Thr-D-Ser-ring(4-10)[Dab-Dab-D-Leu-Thr-Ser-Dab-Thr] 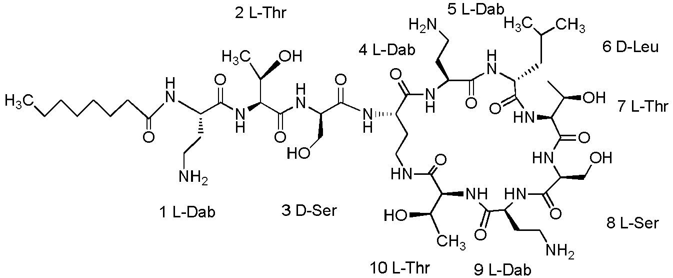
(69) Octanoyl-Dab-Thr-D-Ser-ring(4-10)[Dab-Dab-D-Leu-Thr-Dab-Ser-Thr] 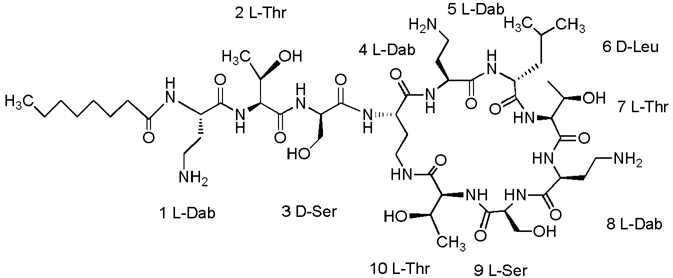
(70) (S)-6-methyloctanoyl-Dab-Thr-Dab-ring(4-10)[Dab-Dab-D-Leu-Leu-Dab-Dab-Thr] 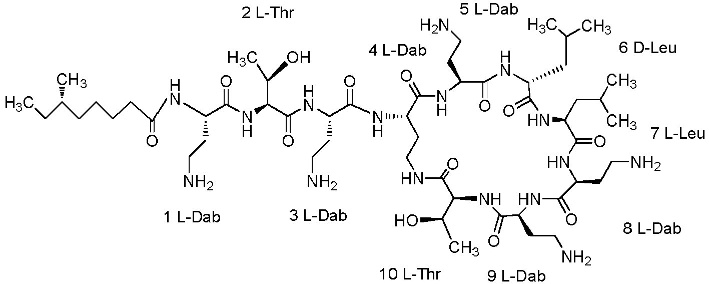
(71) (S)-6-methyloctanoyl-Dab-Thr-Dab-ring(4-10)[Dab-Dab-D-Leu-Ile-Dab-Dab-Thr] 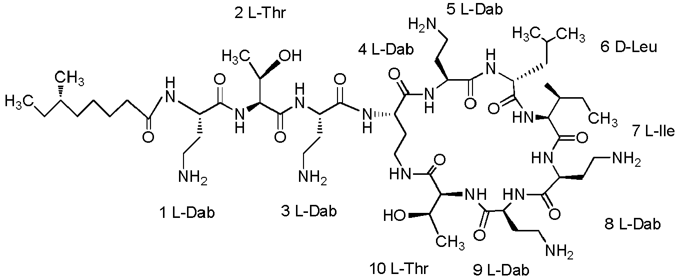
(72) (S)-6-methyloctanoyl-Dab-Thr-Dab-ring(4-10)[Dab-Dab-D-Leu-Val-Dab-Dab-Thr] 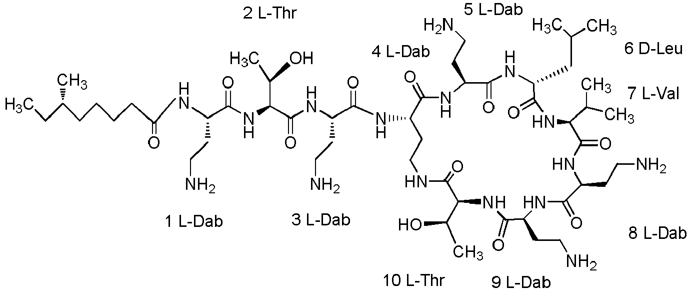
(73) (S)-6-methyloctanoyl-Dab-Thr-Dab-ring(4-10)[Dab-Dab-D-Leu-Nva-Dab-Dab-Thr] 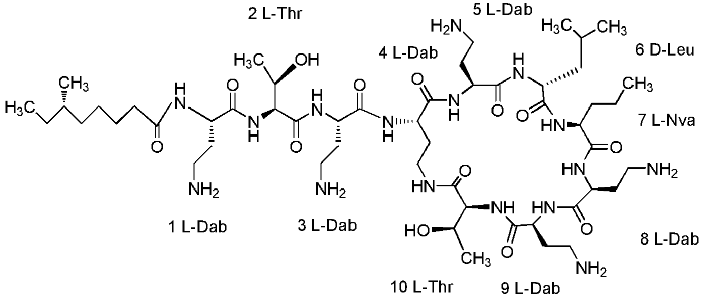
(74) 6-methylheptanoyl-Dab-Thr-Dab-ring(4-10)[Dab-Dab-D-Leu-Leu-Dab-Dab-Thr] 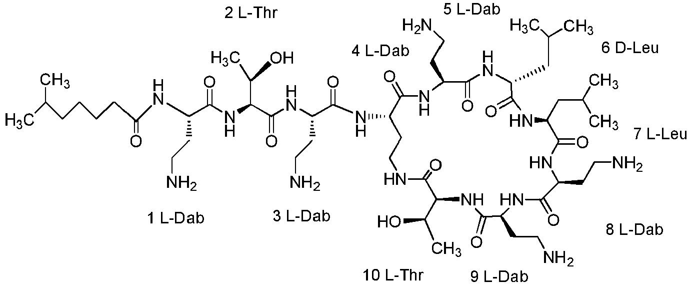
(75) 6-methylheptanoyl-Dab-Thr-Dab-ring(4-10)[Dab-Dab-D-Leu-Ile-Dab-Dab-Thr] 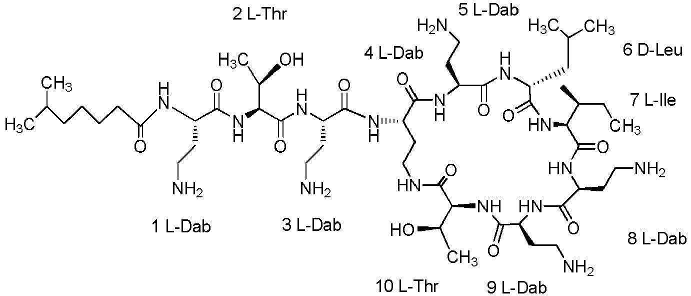
(76) 6-methylheptanoyl-Dab-Thr-Dab-ring(4-10)[Dab-Dab-D-Leu-Val-Dab-Dab-Thr] 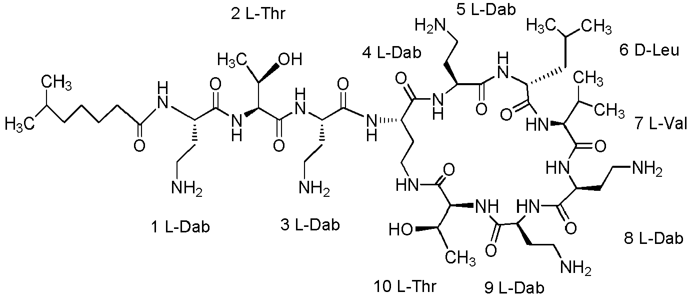
(77) 6-methylheptanoyl-Dab-Thr-Dab-ring(4-10)[Dab-Dab-D-Leu-Nva-Dab-Dab-Thr] 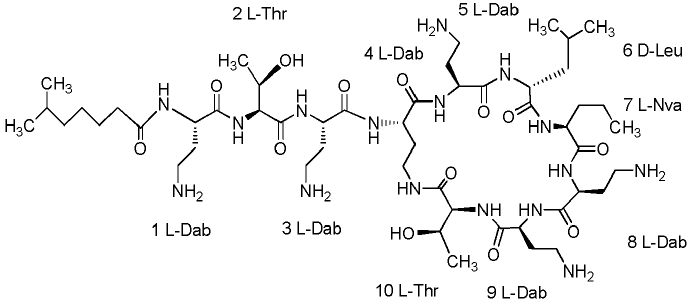
(78) Octanoyl-Dab-Thr-Dab-ring(4-10)[Dab-Dab-D-Leu-Leu-Dab-Dab-Thr] 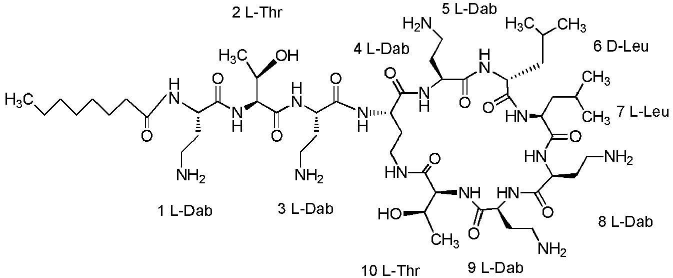
(79) Octanoyl-Dab-Thr-Dab-ring(4-10)[Dab-Dab-D-Leu-Ile-Dab-Dab-Thr] 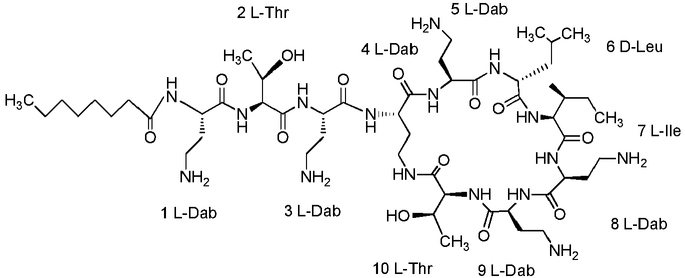
(80) Octanoyl-Dab-Thr-Dab-ring(4-10)[Dab-Dab-D-Leu-Val-Dab-Dab-Thr] 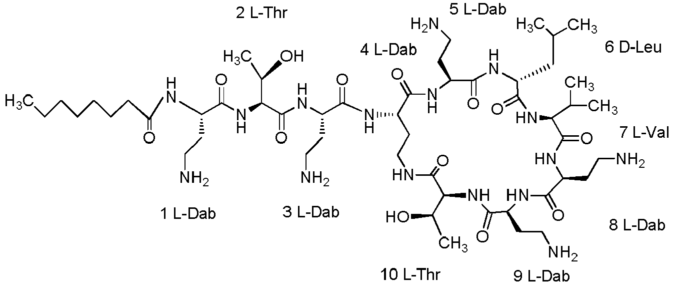
(81) Octanoyl-Dab-Thr-Dab-ring(4-10)[Dab-Dab-D-Leu-Nva-Dab-Dab-Thr] 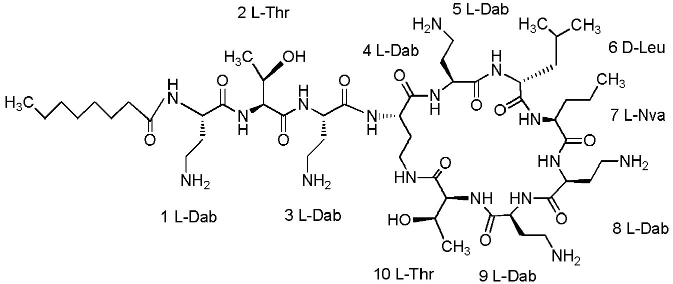
(82) Heptanoyl-Dab-Thr-Dab-ring(4-10)[Dab-Dab-D-Leu-Leu-Dab-Dab-Thr] 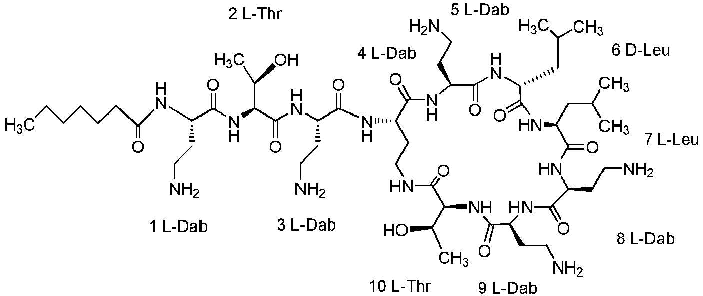
(83) Heptanoyl-Dab-Thr-Dab-ring(4-10)[Dab-Dab-D-Leu-Ile-Dab-Dab-Thr] 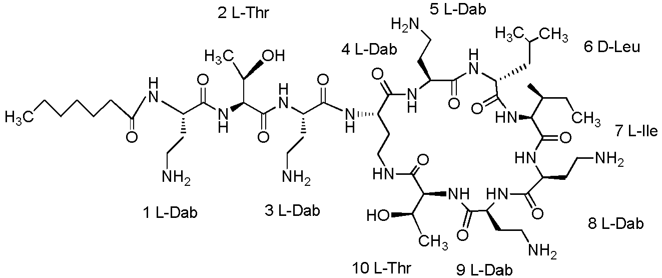
(84) Heptanoyl-Dab-Thr-Dab-ring(4-10)[Dab-Dab-D-Leu-Val-Dab-Dab-Thr] 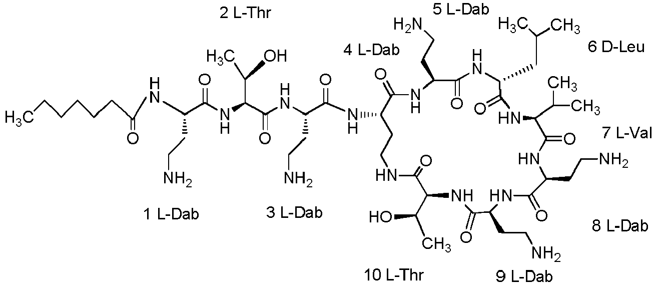
(85) Heptanoyl-Dab-Thr-Dab-ring(4-10)[Dab-Dab-D-Leu-Nva-Dab-Dab-Thr] 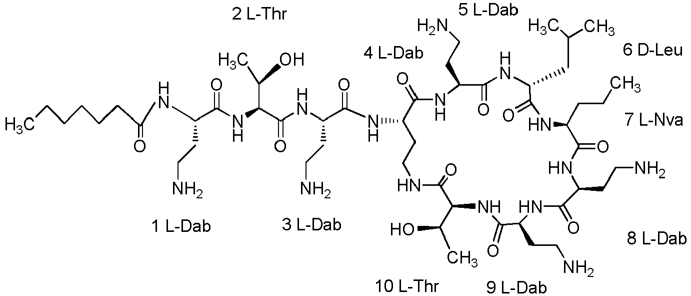
(86) 7-methyloctanoyl-Dab-Thr-Dab-ring(4-10)[Dab-Dab-D-Leu-Leu-Dab-Dab-Thr] 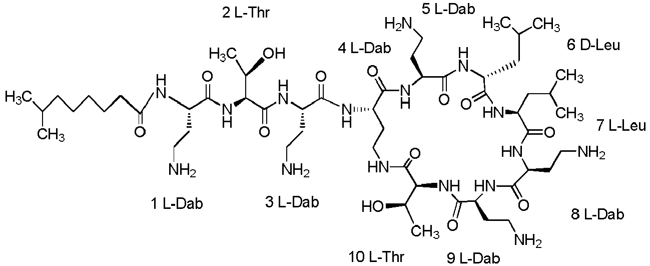
(87) Octanoyl-Dab-Ser-Dab-ring(4-10)[Dab-Dab-D-Leu-Leu-Dab-Dab-Thr] 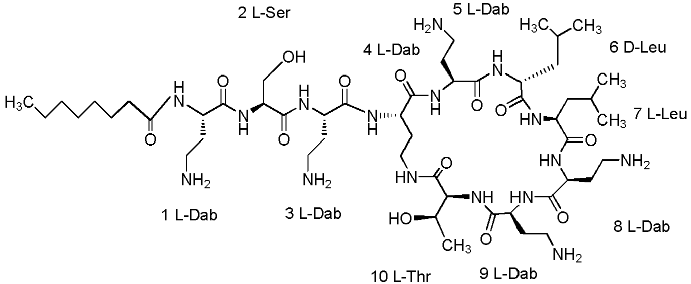
(88) Octanoyl-Dab-Thr-Dab-ring(4-10)[Dab-Dab-D-Leu-Leu-Dab-Dab-Ser] 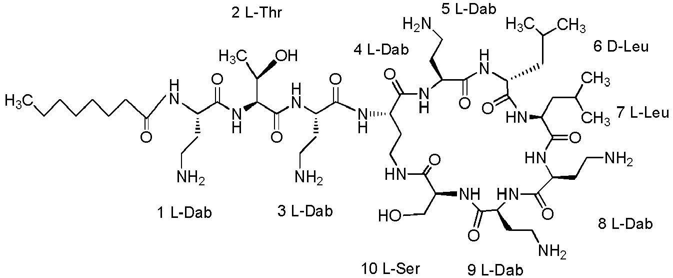
(89) Octanoyl-Dab-Ser-Dab-ring(4-10)[Dab-Dab-D-Leu-Ile-Dab-Dab-Thr] 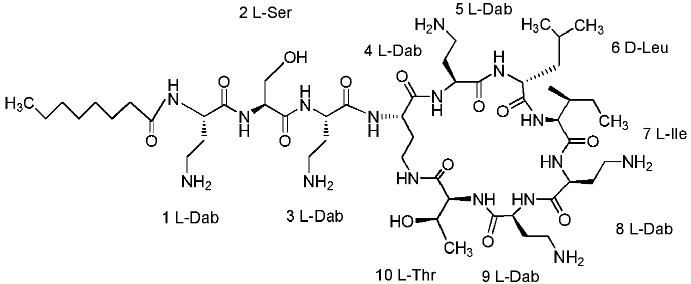
(90) Octanoyl-Dab-Thr-Dab-ring(4-10)[Dab-Dab-D-Leu-Ile-Dab-Dab-Ser] 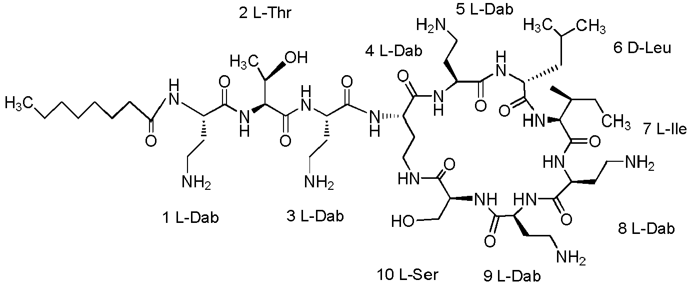
(91) Octanoyl-Dab-Ser-Dab-ring(4-10)[Dab-Dab-D-Leu-Val-Dab-Dab-Thr] 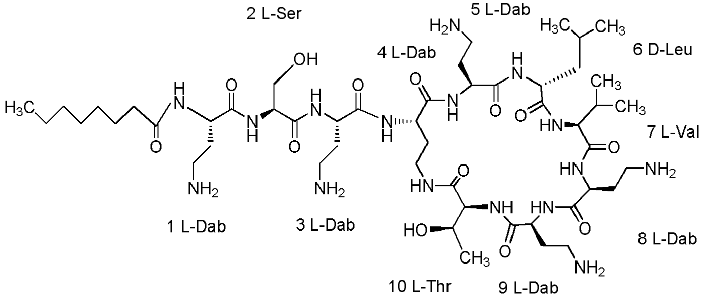
(92) Octanoyl-Dab-Thr-Dab-ring(4-10)[Dab-Dab-D-Leu-Val-Dab-Dab-Ser] 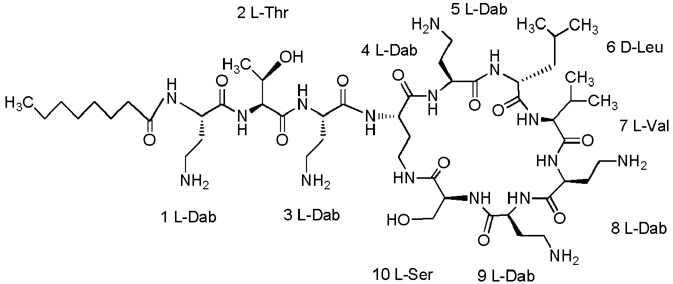
(93) Octanoyl-Dab-Ser-Dab-ring(4-10)[Dab-Dab-D-Leu-Nva-Dab-Dab-Thr] 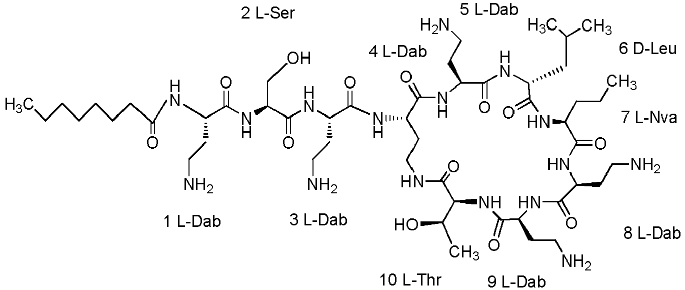
(94) Octanoyl-Dab-Thr-Dab-ring(4-10)[Dab-Dab-D-Leu-Nva-Dab-Dab-Ser] 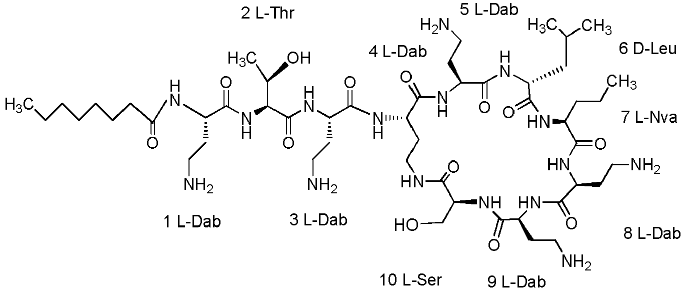
(95) 7-methyloctanoyl-Dab-Ser-Dab-ring(4-10)[Dab-Dab-D-Leu-Leu-Dab-Dab-Thr] 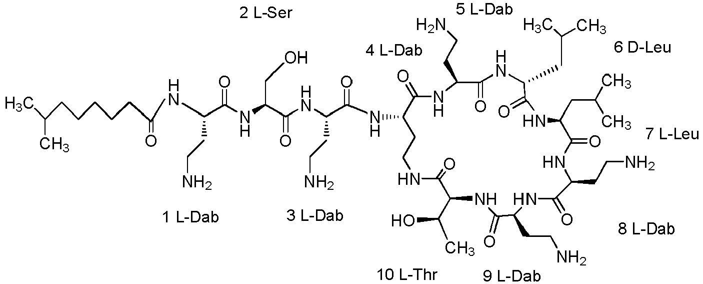
(96) 7-methyloctanoyl-Dab-Thr-Dab-ring(4-10)[Dab-Dab-D-Leu-Leu-Dab-Dab-Ser] 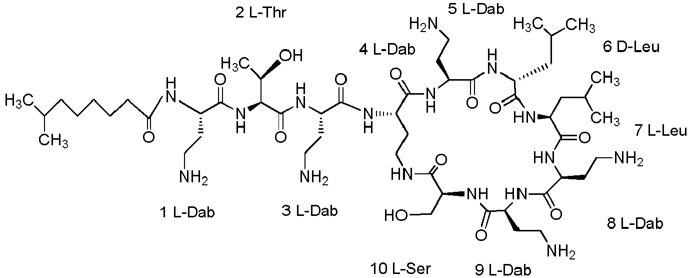
(97) Octanoyl-Ser-Thr-Dab-ring(4-10)[Dab-Dab-D-Leu-Leu-Dab-Dab-Thr] 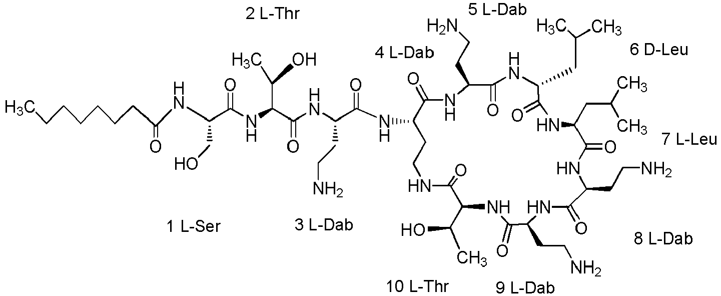
(98) Octanoyl-Dab-Thr-Ser-ring(4-10)[Dab-Dab-D-Leu-Leu-Dab-Dab-Thr] 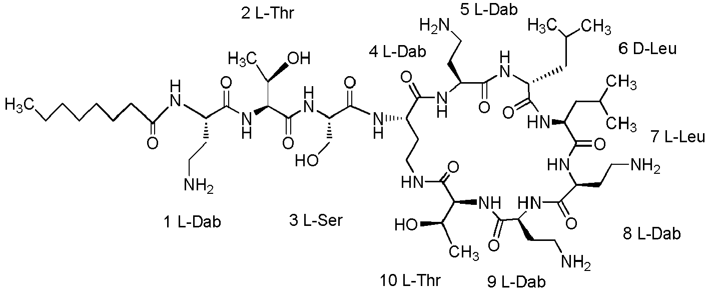
(99) Octanoyl-Dab-Thr-Dab-ring(4-10)[Dab-Ser-D-Leu-Leu-Dab-Dab-Thr] 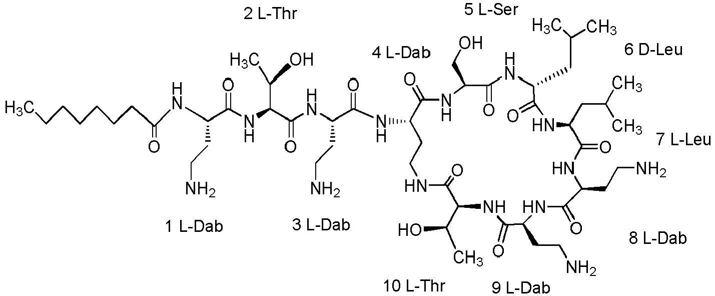
(100) Octanoyl-Dab-Thr-Dab-ring(4-10)[Dab-Dab-D-Leu-Leu-Ser-Dab-Thr] 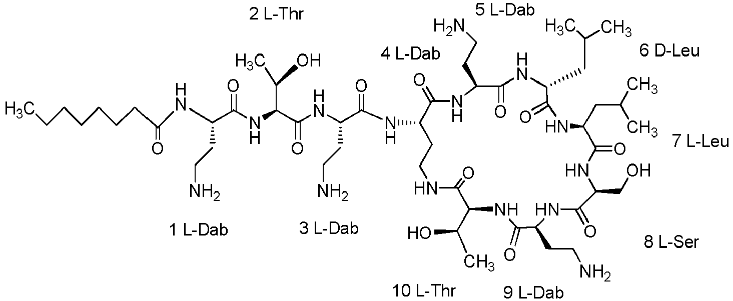
(101) Octanoyl-Dab-Thr-Dab-ring(4-10)[Dab-Dab-D-Leu-Leu-Dab-Ser-Thr] 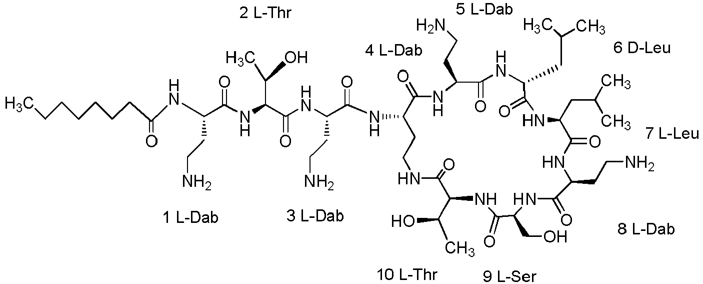
(102) (S)-6-methyloctanoyl-Dab-Thr-Dab-ring(4-10)[Dab-Dab-D-Leu-Thr-Dab-Dab-Thr] 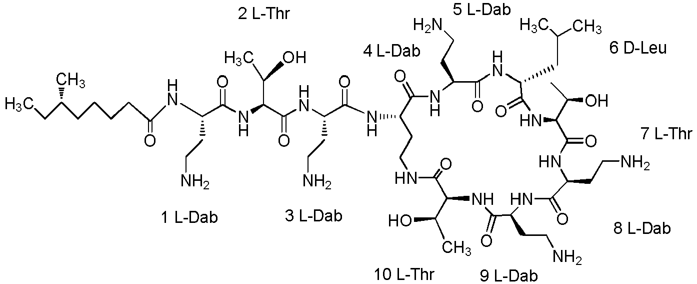
(103) 6-methylheptanoyl-Dab-Thr-Dab-ring(4-10)[Dab-Dab-D-Leu-Thr-Dab-Dab-Thr] 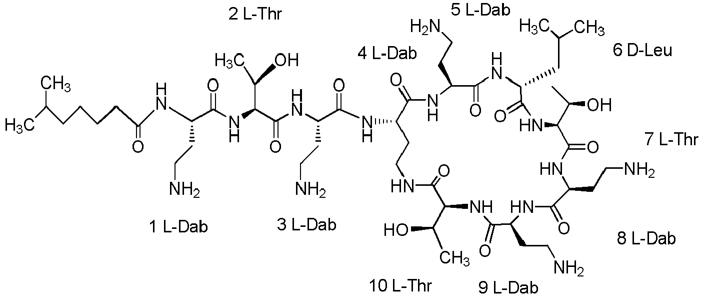
(104) Octanoyl-Dab-Thr-Dab-ring(4-10)[Dab-Dab-D-Leu-Thr-Dab-Dab-Thr] 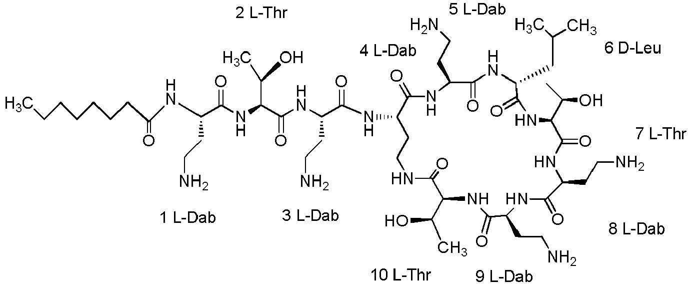
(105) Heptanoyl-Dab-Thr-Dab-ring(4-10)[Dab-Dab-D-Leu-Thr-Dab-Dab-Thr] 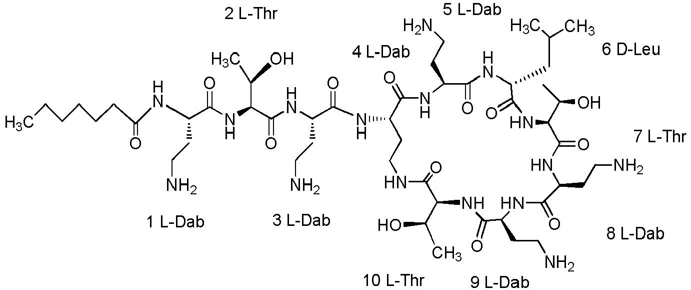
(106) Nonanoyl-Dab-Thr-Dab-ring(4-10)[Dab-Dab-D-Leu-Thr-Dab-Dab-Thr] 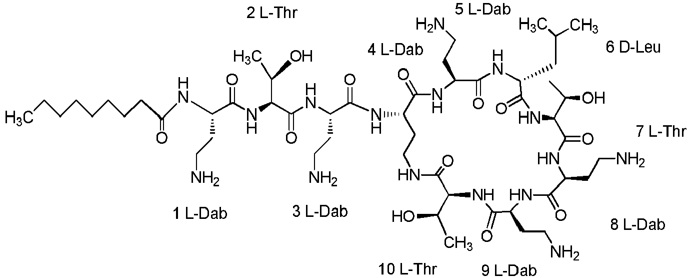
(107) Octanoyl-Dab-Ser-Dab-ring(4-10)[Dab-Dab-D-Leu-Thr-Dab-Dab-Thr] 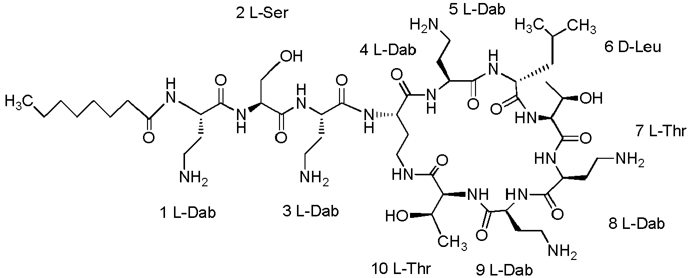
(108) Octanoyl-Dab-Thr-Dab-ring(4-10)[Dab-Dab-D-Leu-Ser-Dab-Dab-Thr] 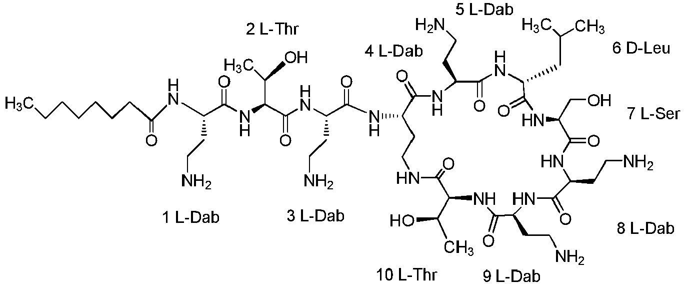
(109) Octanoyl-Dab-Thr-Dab-ring(4-10)[Dab-Dab-D-Leu-Thr-Dab-Dab-Ser] 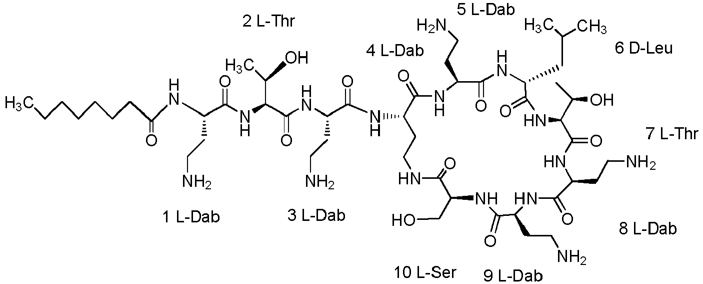
(110) Octanoyl-Ser-Thr-Dab-ring(4-10)[Dab-Dab-D-Leu-Thr-Dab-Dab-Thr] 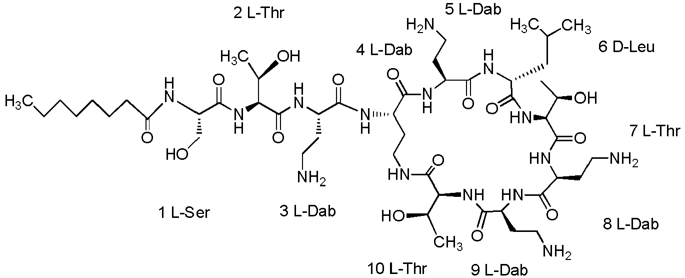
(111) Octanoyl-Dab-Thr-Ser-ring(4-10)[Dab-Dab-D-Leu-Thr-Dab-Dab-Thr] 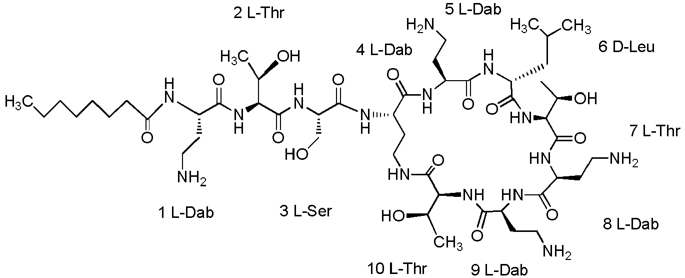
(112) Octanoyl-Dab-Thr-Dab-ring(4-10)[Dab-Ser-D-Leu-Thr-Dab-Dab-Thr] 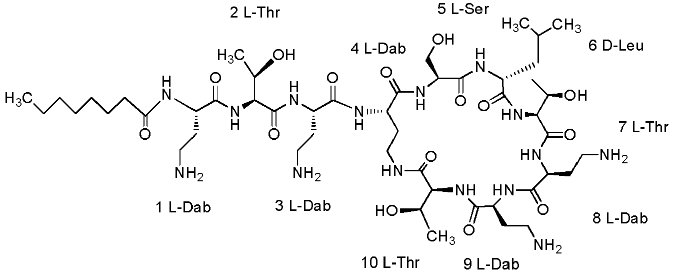
(113) Octanoyl-Dab-Thr-Dab-ring(4-10)[Dab-Dab-D-Leu-Thr-Ser-Dab-Thr] 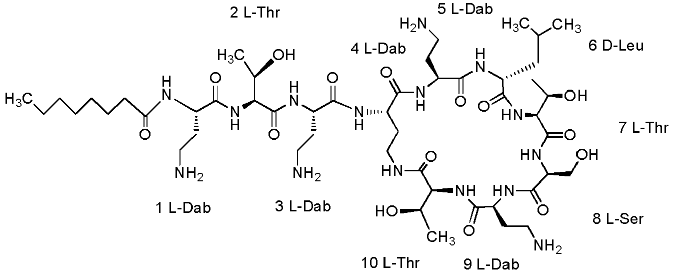
(114) Octanoyl-Dab-Thr-Dab-ring(4-10)[Dab-Dab-D-Leu-Thr-Dab-Ser-Thr] 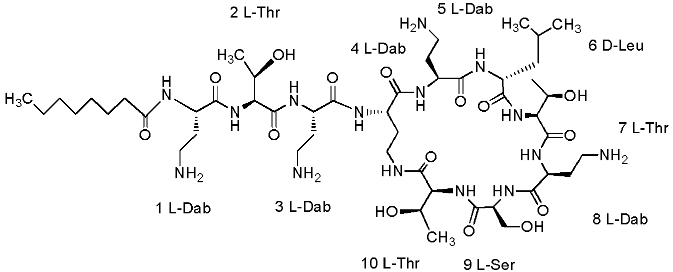
(115) (S)-6-methyloctanoyl-Dab-Thr-D-Ser-ring(4-10)[Dab-Dab-D-Phe-Thr-Dab-Dab-Thr] 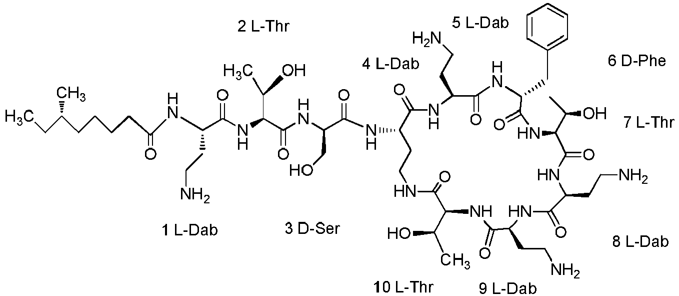
(116) 6-methylheptanoyl-Dab-Thr-D-Ser-ring(4-10)[Dab-Dab-D-Phe-Thr-Dab-Dab-Thr] 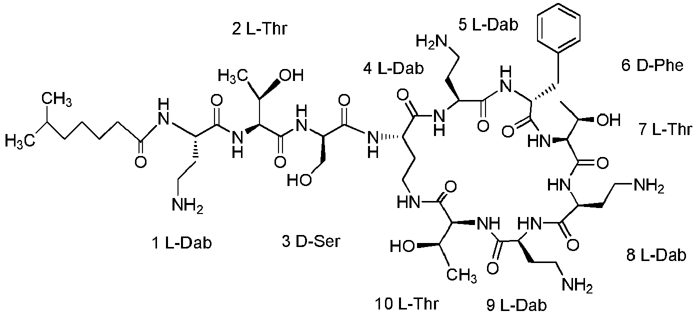
(117) Octanoyl-Dab-Thr-D-Ser-ring(4-10)[Dab-Dab-D-Phe-Thr-Dab-Dab-Thr] 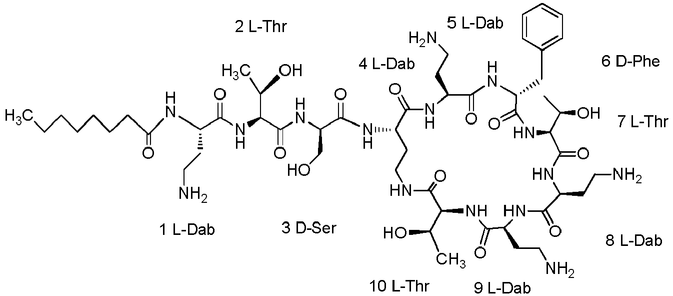
(118) Heptanoyl-Dab-Thr-D-Ser-ring(4-10)[Dab-Dab-D-Phe-Thr-Dab-Dab-Thr] 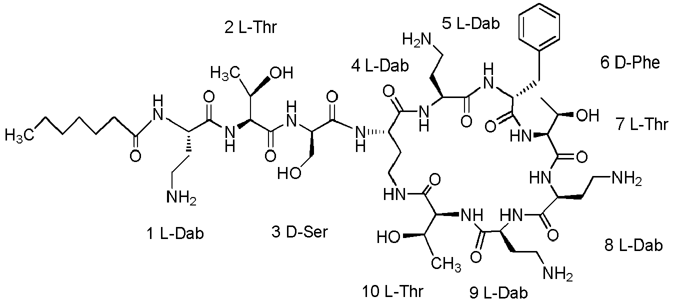
(119) Nonanoyl-Dab-Thr-D-Ser-ring(4-10)[Dab-Dab-D-Phe-Thr-Dab-Dab-Thr] 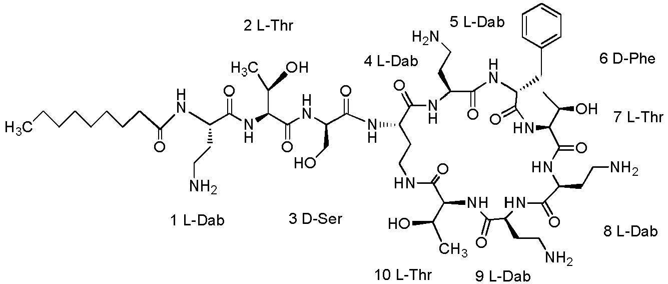
(120) Octanoyl-Dab-Ser-D-Ser-ring(4-10)[Dab-Dab-D-Phe-Thr-Dab-Dab-Thr] 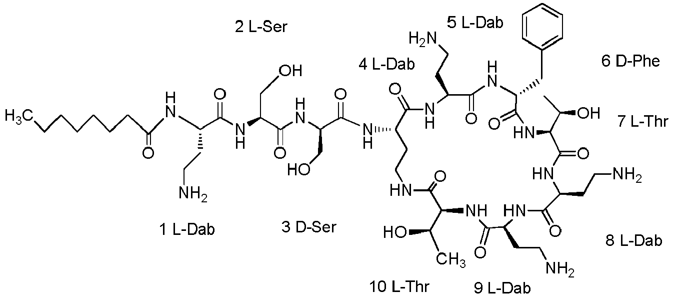
(121) Octanoyl-Dab-Thr-D-Ser-ring(4-10)[Dab-Dab-D-Phe-Ser-Dab-Dab-Thr] 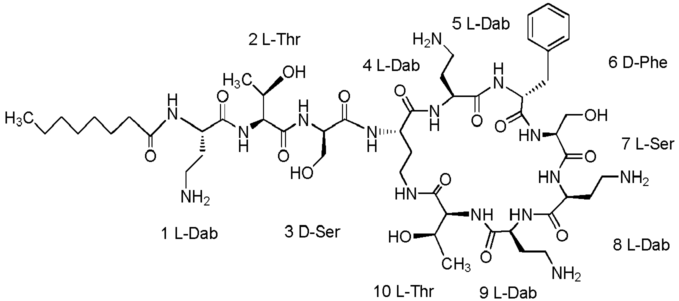
(122) Octanoyl-Dab-Thr-D-Ser-ring(4-10)[Dab-Dab-D-Phe-Thr-Dab-Dab-Ser] 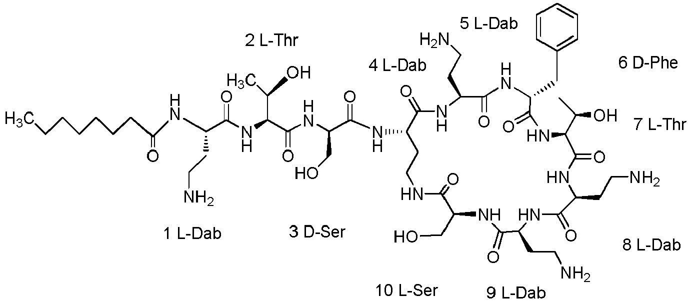
(123) Octanoyl-Ser-Thr-D-Ser-ring(4-10)[Dab-Dab-D-Phe-Thr-Dab-Dab-Thr] 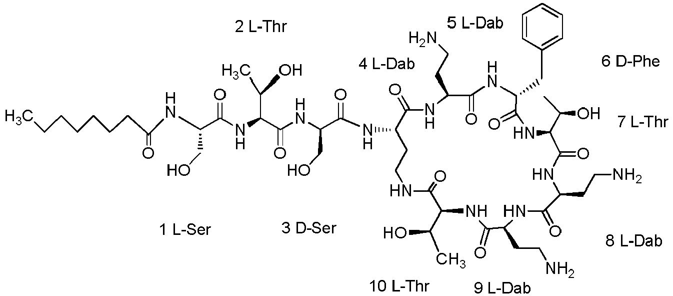
(124) Octanoyl-Dab-Thr-D-Ser-ring(4-10)[Dab-Ser-D-Phe-Thr-Dab-Dab-Thr] 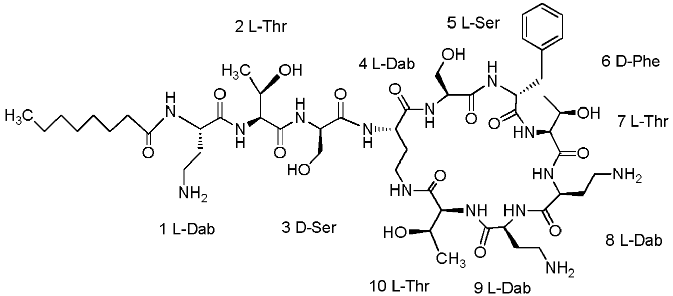
(125) Octanoyl-Dab-Thr-D-Ser-ring(4-10)[Dab-Dab-D-Phe-Thr-Ser-Dab-Thr] 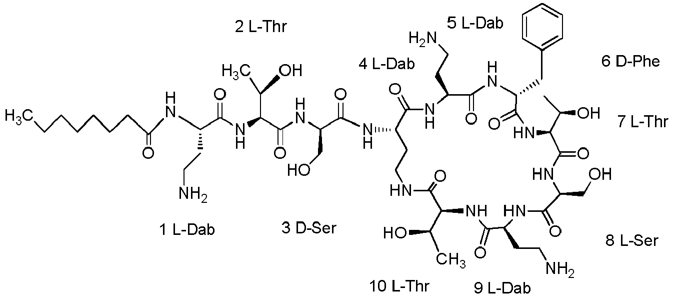
(126) Octanoyl-Dab-Thr-D-Ser-ring(4-10)[Dab-Dab-D-Phe-Thr-Dab-Ser-Thr] 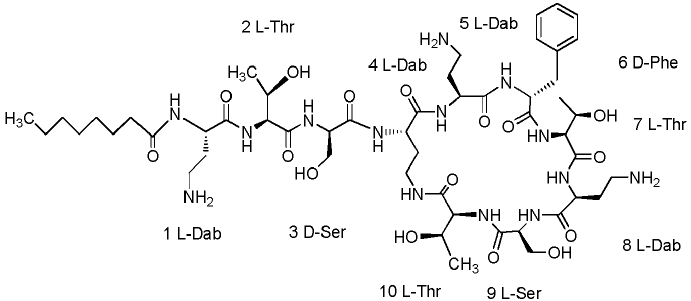
(127) (S)-6-methyloctanoyl-Dab-Thr-Dab-ring(4-10)[Dab-Dab-D-Phe-Leu-Dab-Dab-Leu] 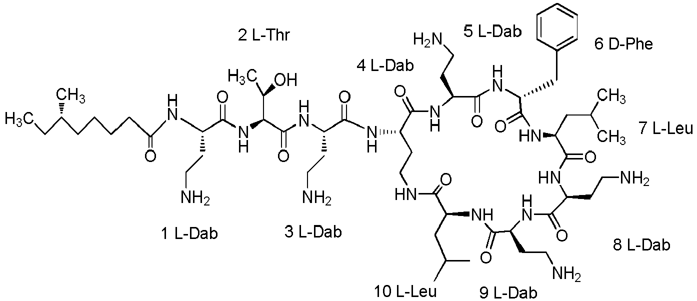
(128) 6-methylheptanoyl-Dab-Thr-Dab-ring(4-10)[Dab-Dab-D-Phe-Leu-Dab-Dab-Leu] 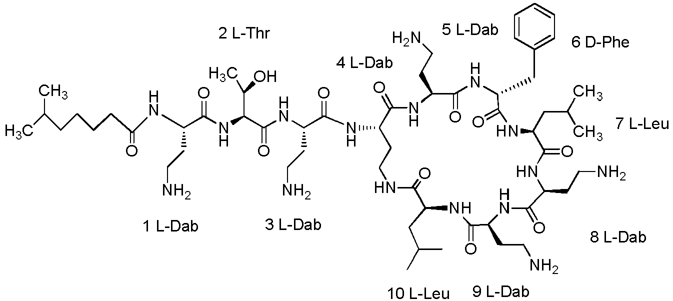
(129) Octanoyl-Dab-Thr-Dab-ring(4-10)[Dab-Dab-D-Phe-Leu-Dab-Dab-Leu] 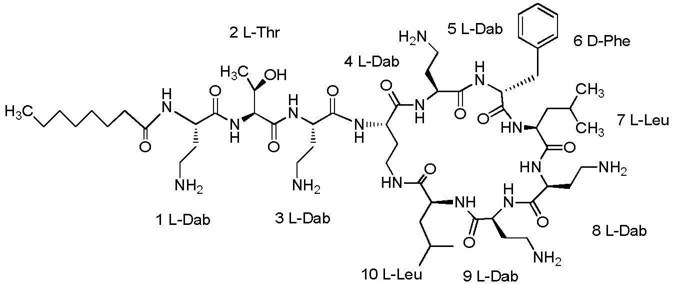
(130) Heptanoyl-Dab-Thr-Dab-ring(4-10)[Dab-Dab-D-Phe-Leu-Dab-Dab-Leu] 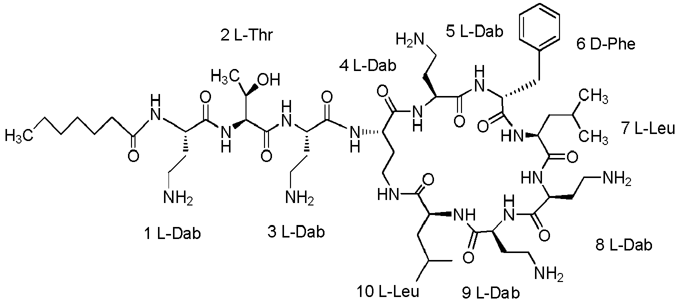
(131) Nonanoyl-Dab-Thr-Dab-ring(4-10)[Dab-Dab-D-Phe-Leu-Dab-Dab-Leu] 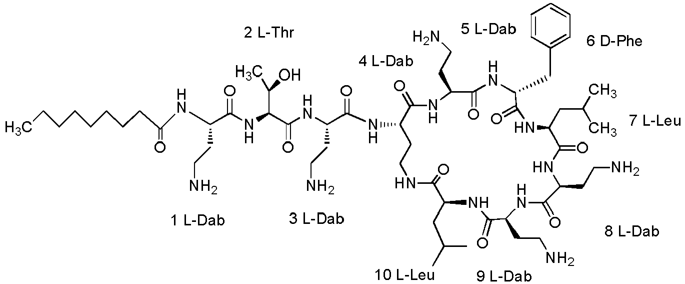
(132) Octanoyl-Dab-Ser-Dab-ring(4-10)[Dab-Dab-D-Phe-Leu-Dab-Dab-Leu] 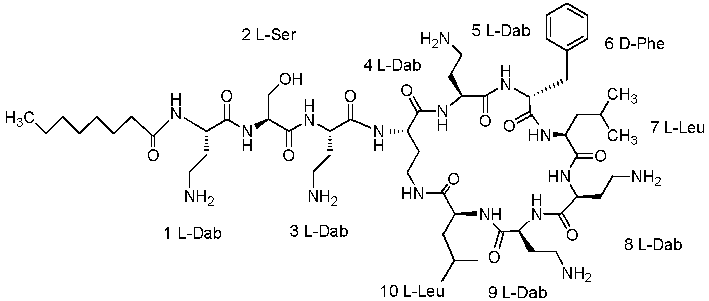
(133) Octanoyl-Ser-Thr-Dab-ring(4-10)[Dab-Dab-D-Phe-Leu-Dab-Dab-Leu] 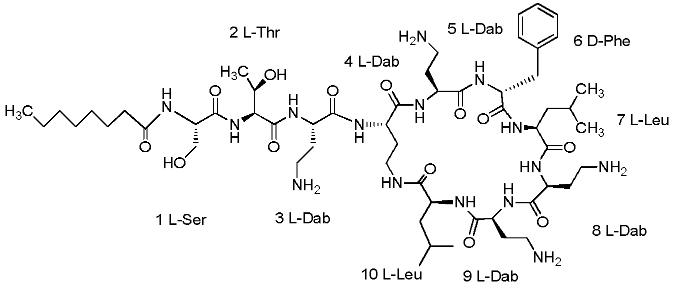
(134) Octanoyl-Dab-Thr-Ser-ring(4-10)[Dab-Dab-D-Phe-Leu-Dab-Dab-Leu] 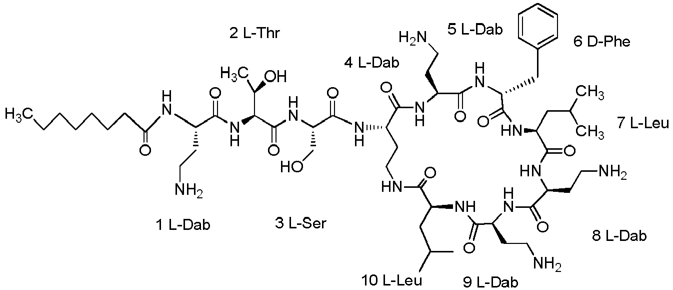
(135) Octanoyl-Dab-Thr-Dab-ring(4-10)[Dab-Ser-D-Phe-Leu-Dab-Dab-Leu] 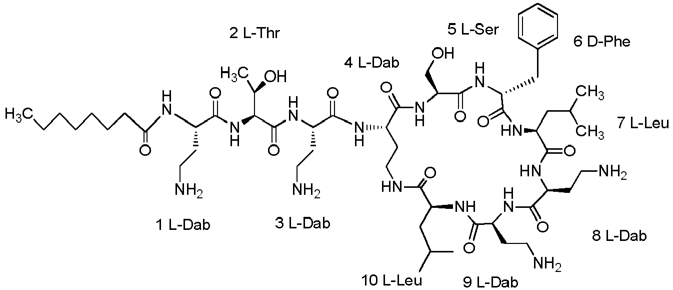
(136) Octanoyl-Dab-Thr-Dab-ring(4-10)[Dab-Dab-D-Phe-Leu-Ser-Dab-Leu] 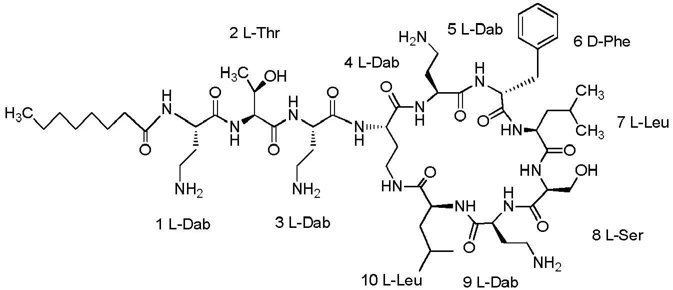
(137) Octanoyl-Dab-Thr-Dab-ring(4-10)[Dab-Dab-D-Phe-Leu-Dab-Ser-Leu] 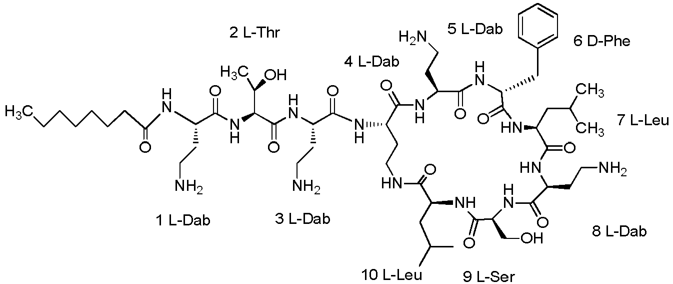
(138) (S)-6-methyloctanoyl-Dab-Thr-Dab-ring(4-10) [Dab-Dab-Phe-Thr-Dab-Dab-Thr] 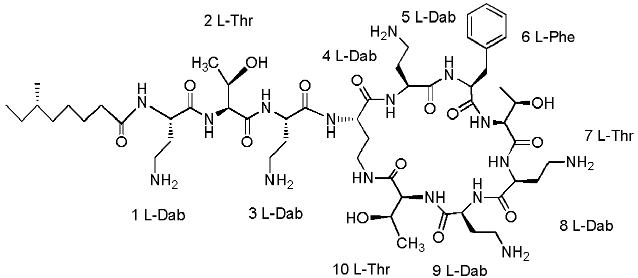
(139) 6-methylheptanoyl-Dab-Thr-Dab-ring(4-10)[Dab-Dab-Phe-Thr-Dab-Dab-Thr] 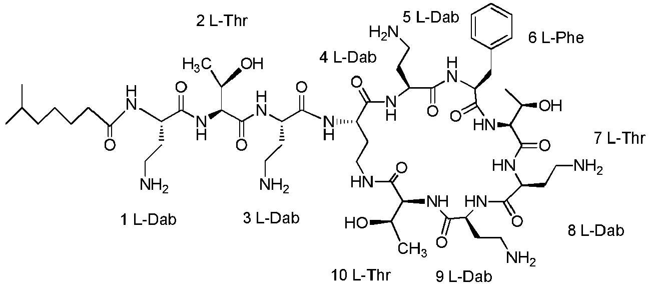
(140) Octanoyl-Dab-Thr-Dab-ring(4-10)[Dab-Dab-Phe-Thr-Dab-Dab-Thr] 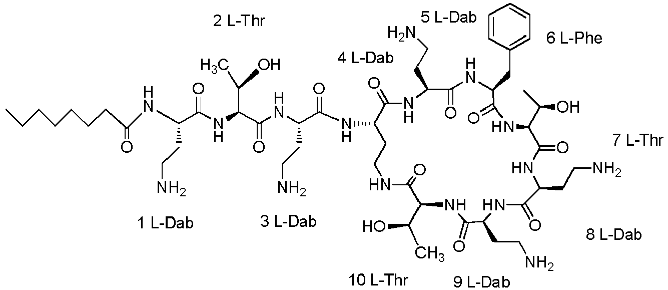
(141) Heptanoyl-Dab-Thr-Dab-ring(4-10)[Dab-Dab-Phe-Thr-Dab-Dab-Thr] 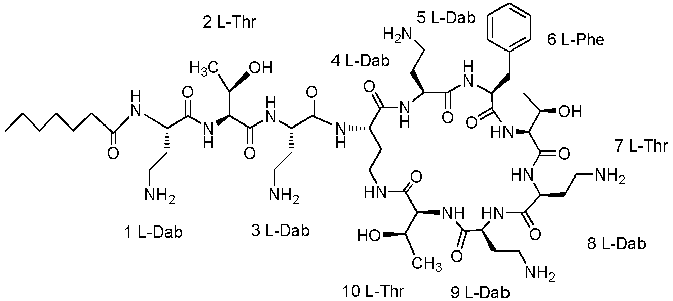
(142) Nonanoyl-Dab-Thr-Dab-ring(4-10)[Dab-Dab-Phe-Thr-Dab-Dab-Thr] 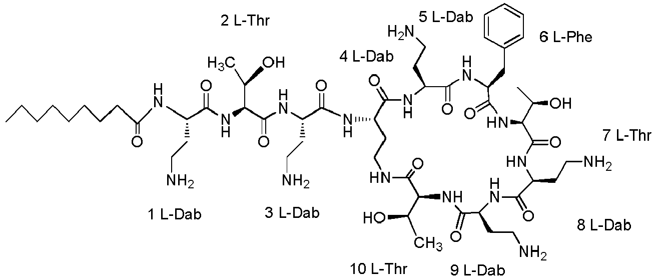
(143) (S)-6-methyloctanoyl-Dab-Thr-D-Dab-ring(4-10)[Dab-Dab-D-Phe-Thr-Dab-Dab-Thr] 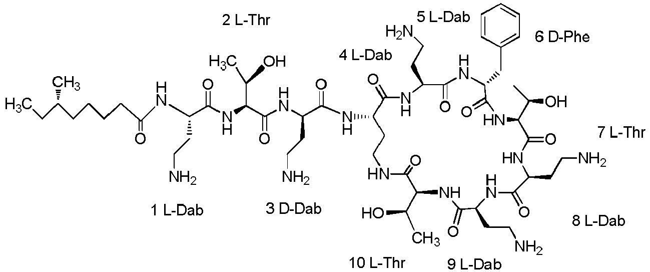
(144) 6-methylheptanoyl-Dab-Thr-D-Dab-ring(4-10)[Dab-Dab-D-Phe-Thr-Dab-Dab-Thr] 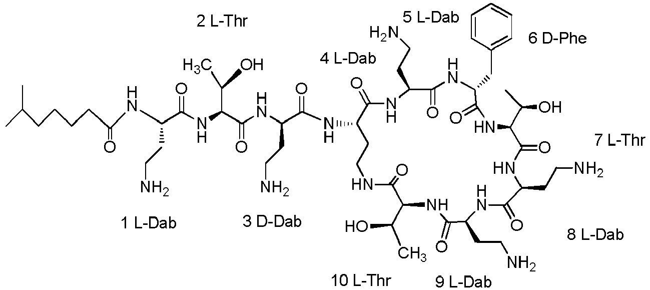
(145) Octanoyl-Dab-Thr-D-Dab-ring(4-10)[Dab-Dab-D-Phe-Thr-Dab-Dab-Thr] 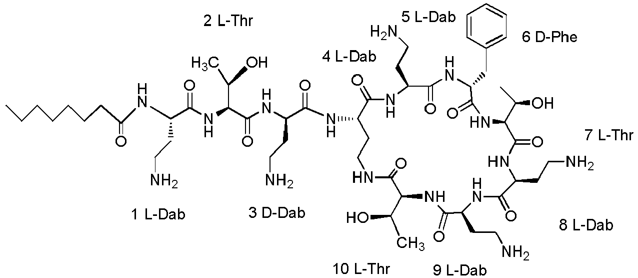
(146) Heptanoyl-Dab-Thr-D-Dab-ring(4-10)[Dab-Dab-D-Phe-Thr-Dab-Dab-Thr] 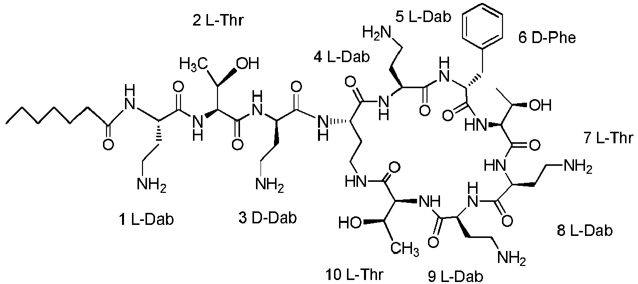
(147) Nonanoyl-Dab-Thr-D-Dab-ring(4-10)[Dab-Dab-D-Phe-Thr-Dab-Dab-Thr] 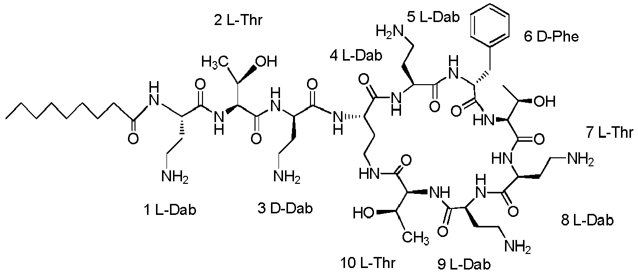
(148) Octanoyl-Thr-Thr-Thr-ring(4-10)[Dab-Dab-D-Phe-Leu-Dab-Dab-Thr] 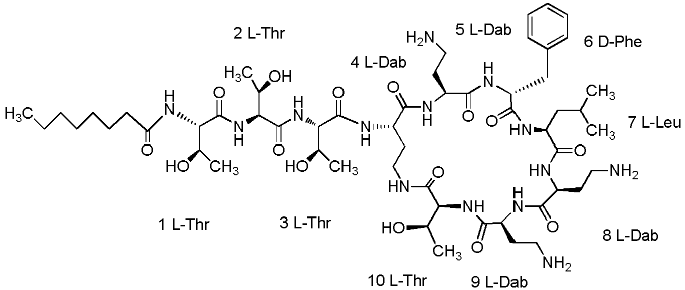
(149) Octanoyl-Dap-Thr-Dab-ring(4-10)[Dab-Dab-D-Phe-Leu-Dab-Dab-Thr] 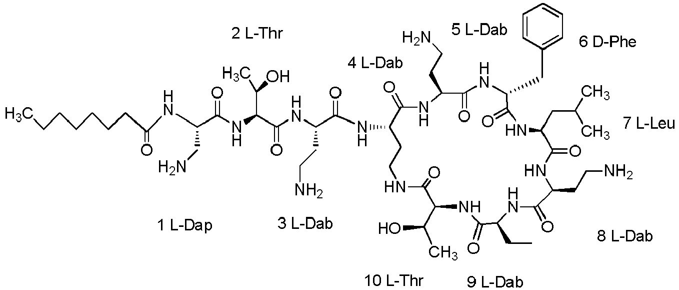
(150) Octanoyl-Arg-Thr-Dab-ring(4-10)[Dab-Dab-D-Phe-Leu-Dab-Dab-Thr] 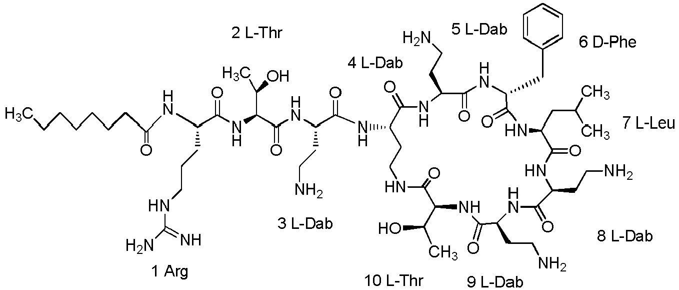
(151) Octanoyl-Dab-Thr-Met-ring(4-10)[Dab-Dab-D-Phe-Leu-Dab-Dab-Thr] 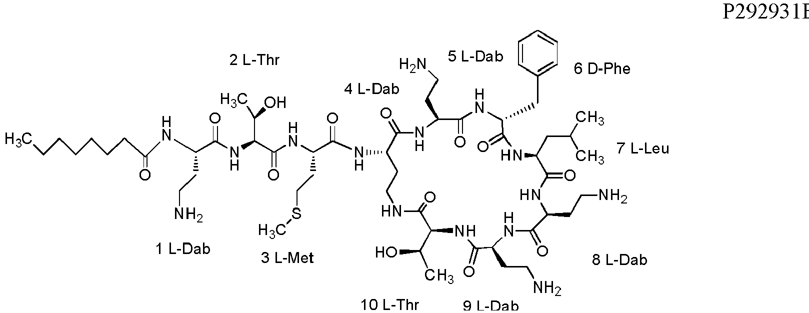
(152) Octanoyl-Dab-Thr-Dab-ring(4-10)[Dab-Dap-D-Phe-Leu-Dap-Dap-Thr]_{∘}

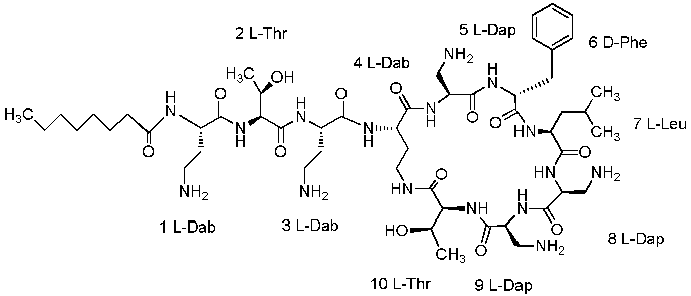

The term "ring (4-10)" refers to a heptapeptide ring which is formed by a terminal carboxyl group at position-10 bonded to the side chain amino group of the position-4 basic amino acid via an amide bond, and has a structure as shown in Formula-I and-II.

The configuration of D amino acid is indicated by D. When no configuration is mentioned, it can be understood that the amino acid is L-configuration. Dab represents α,γ-diaminobutyric acid, Nva represents norvaline, and Dap represents α,β-diaminopropionic acid.

In this invention, the pharmaceutically acceptable salts of compound (116) denote the salts of said compound with acids, said acids being selected from the group consisting of inorganic or organic acids, wherein the mineral acids, is selected from perchloricc acid, hydroiodic acid, hydrobromic acid, hydrochloric acid, sulfuric acid, nitric acid or phosphoric acid; said organic acid being slelcted from acetic acid, trifluoroacetic acid, lactic acid, succinic acid, fumaric acid, maleic acid, citric acid, benzoic acid, methanesulfonic acid acid and *p*-toluenesulfonic acid.

The present invention also provides a novel method for solid phase synthesis of a polymyxin derivative or a pharmaceutically acceptable salt thereof. The method comprises the steps of: solid phase condensation, solid phase cyclization to prepare a polymyxin derivative or a pharmaceutically acceptable salt thereof. Following are the steps:
(1) The free amino group in the protected basic amino acid Fmoc-AA-OP side chain is reacted with a halogenated resin to obtain Fmoc-AA-OP-resin; wherein P is a carboxyl protecting group, for example, allyl, benzyl (Bn); when Fmoc-AA-OP is Fmoc-Dab-OP, its structure is as shown in Formula III: When Fmoc-AA-OP is Fmoc-Dap-OP, its structure is as shown in Formula IV: 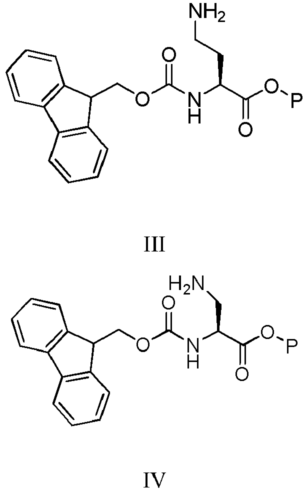
(2) Fmoc-AA-OP-resin is coupled one by one to obtain a linear polypeptide-resin;
(3) Selectively removing the protecting group from linear polypeptide-resin, and via solid-phase cyclizing to obtain a cyclic polypeptide-resin;
(4) The cyclic polypeptide-resin is acid-decomposed to obtain a crude cyclic polypeptide;
(5) The crude cyclic polypeptide is purified and/or salified, and lyophilized to obtain a pure cyclic polypeptide.

### I regarding the step (1)

The halogenated resin described in the step (1) is selected from the group consisting of trityl chloride resin, 4-methyltrityl chloride resin, 4-methoxytrityl chloride resin, 2-chlorotrityl chloride resin, bromo-(4-methylphenyl)-methyl resin or bromo-(4-methoxyphenyl)-methyl resin, for example, the resin is 2-chlorotrityl chloride resin.

The degree of substitution of the halogenated resin is from 0.1 to 1.6 mmol/g, for example, the degree of substitution is from 0.5 to 1.0 mmol/g.

The amount of each Fmoc-protected amino acid charged is from 1.2 to 6 times of the total moles of the resin charged, for example from 2.0 to 3.5 times.

The base is selected from the group consisting of at least one of following: *N*, *N*-diisopropylethylamine (DIEA), triethylamine (TEA), and pyridine, for example, DIEA; the molar amount of the base is 1.5-3 times the molar amount of the Fmoc-protected amino acid, for example, twice the molar amount of the Fmoc-protected amino acid.

The substitution reaction time is 1-12 h, for example, 2-3 h.

### II regarding step (2)

The reagent for removing the α-amino Fmoc protecting group in the step (2) includes, but is not limited to, a solution of 10-30% piperidine (PIP) in DMF, for example, PIP (20% concentration) in DMF. The deprotecting agent is used in an amount of 5 to 15 mL per gram of the resin to be charged, for example, 10 mL per gram of the resin. The deprotection reaction time is 10-60 min, for example, 10-20 min. The reagent for removing the position-4 amino acid side chain amino group ivDde or Dde protecting group includes, but is not limited to, a solution of hydrazine hydrate in DMF at a concentration of 1-10%, for example, at a concentration of 2%. The deprotecting agent is used in an amount of 5 to 15 mL per gram of the resin to be charged, for example, 10 mL per gram of the resin. The deprotection reaction time is 30-100 min, for example, 30-60 min.

The coupling agent in the coupling reaction is selected from the group consisting of *N, N*-diisopropylcarbodiimide (DIC), *N, N*-dicyclohexylcarbodiimide (DCC), 1-ethyl-(3-dimethylaminopropyl)carbodiimide hydrochloride (EDC), benzotriazole-N,N,N',N'-tetramethyluronium hexafluorophosphate (HBTU), 6-Chlorobenzotriazole-1,1,3,3-tetramethylurea hexafluorophosphate (HCTU), 2-(7-azobenzotriazole)-N,N,N',N'-Tetramethylurea hexafluorophosphate (HATU), O-benzotriazole-N,N,N',N'-tetramethyluronium tetrafluoroborate (TBTU), benzotriazole-1-yl-oxy-trispyrrolidinophosphonium hexafluorophosphate (PyBOP), for example, *N*, *N*-diisopropylcarbodiimide (DIC).

The moles of the coupling agent used is from 1.2 to 6 times the total moles of the charged resin, for example from 2.0 to 3.5 times.

The activator is selected from the group consisting of 1-hydroxybenzotriazole (HOBT), 6-chloro-1-hydroxybenzotriazole(Cl-HOBT), 1-hydroxy-7-azobenzotriazine (HOAT), for example, is 1-hydroxybenzotriazole (HOBT).

The activator is used in a molar amount of from 1.2 to 6 times of the total moles of the charged resin, for example from 2.0 to 3.5 times.

The coupling reaction time is 60-300 min, for example, 60-120 min.

In the coupling reaction, for a part of the coupling agent a catalyst needs to be added in. The catalyst is an organic base selected from the group consisting of *N*,*N*-diisopropylethylamine (DIEA), triethylamine (TEA), *N*-methylmorpholine (NMM), for example, *N*,*N*-diisopropylethylamine (DIEA),

The solvent is an aprotic polar solvent selected from the group consisting of dimethylformamide (DMF) or N-methylpyrrolidone (NMP) or mixtures thereof, for example, DMF.

### III regarding step (3)

The reagent for removing the allyl protecting group of the carboxyl group in the step (3) is a solution of tetrakis(triphenylphosphine)palladium/phenylsilane in DCM and DMF (DCM: DMF mixed solution having a volume ratio of 5:5). The tetrakis(triphenylphosphine)palladium is used in a molar amount of 0.1 to 2 times of the total moles of the charged resin, for example, 0.1 to 0.3 times. The phenylsilane molar amount is 2 to 10 times of the total moles of the resin to be charged, for example, 3-5 times. The deprotecting agent is used in an amount of 10 to 30 mL per gram of the resin to be charged, for example, 20 mL per gram of the resin. The deprotection reaction time is 60-300 min, for example, 60-120 min. The reagent for deprotection of the carboxyl benzyl protecting group is H2, 10% Pd/C ethanol solution, and the 10% Pd/C molar amount is 0.1-2 times of the total moles of the charged resin, for example, 0.1-0.3 times. The deprotection reaction time is 30-100 min, for example, 30-60 min.

The solid phase cyclization coupling reagent is selected from the group consisting of: (7-azabenzotriazol-1-yloxy)tripyrrolidinophosphonium
hexafluorophosphate (PyAOP), benzotriazole-1-yl-oxy-trispyrrolidinophosphonium hexafluorophosphate (PyBOP), for example, (7-azabenzotriazol-1-yloxy)tripyrrolidinophosphonium
hexafluorophosphate (PyAOP);

The coupling agent is used from 1.2 to 6 times the total moles of the charged resin, for example from 2.0 to 3.5 times.

The activator is selected from the group consisting of 1-hydroxybenzotriazole (HOBT), 1-hydroxy-7-azobenzotriazole (HOAT), for example, 1-hydroxy-7-azobenzotriazole (HOAT).

The activator is used from 1.2 to 6 times the total moles of the charged resin, for example from 2.0 to 3.5 times.

The cyclization reaction time is from 1 to 20 h, for example, from 1 to 3 h.

The catalyst is an organic base selected from the group consisting of *N, N*-diisopropylethylamine (DIEA), triethylamine (TEA), N-methylmorpholine (NMM), for example, N-methyl Morpholine (NMM).

The solvent is an aprotic polar solvent selected from the group consisting of dimethylformamide (DMF) or N-methylpyrrolidone (NMP) or mixtures thereof, for example, DMF.

### IV regarding the step (4)

The acidolysis solution in the step (4) is a solution containing hydrofluoric acid (HF) or trifluoroacetic acid (TFA), for example, trifluoroacetic acid.

The amount of the acid solution is 5-30 mL per gram of the resin to be charged, for example, 10 mL per gram of the resin. The acidolysis solution comprises trifluoroacetic acid and a side chain protecting group remover.

The concentration of trifluoroacetic acid is 80%-95%, the rest is a side chain protecting group remover.

The side chain protecting group remover is selected from the group consisting of thioanisole, triisopropylsilane, phenol, water, 1,2-ethanedithiol, for example, water.

The acidolysis time is 60-300 min, for example, 100-120 min.

The acid hydrolyzed solution containing the polypeptide was added to cold ether (the ratio of the acid hydrolyzate to cold diethyl ether is 1:20), the peptide is precipitated, centrifuged, and dried to obtain a crude peptide.

### V regarding the step (5)

The crude peptide from step (5) is dissolved in water, filtered through a 0.22 µm pore size filter, purified by preparative high performance liquid chromatography, the mobile phase A 0.1% : TFA/water solution, mobile phase B: 0.1% TFA/acetonitrile solution, using gradient elution, detection wavelength 215 nm, drying the product by lyophilization. The final purity achievable by this method is greater than 95%, for example greater than 99%.

In the step (1), the preparation of the Fmoc-AA-OP-resin is carried out, for example, by adding a halogenated resin to the polypeptide solid phase synthesis tube, adding DCM to swell, when swelling is completed, washing the resin three times with DMF, then washing three times with DCM. The protected starting amino acid Fmoc-AA-OP and DIEA are dissolved in DCM and added to the peptide synthesis tube. The reaction is carried out for 2 h at room temperature. Draw out the reaction solution by vacuum. The resin is washed three times with DMF and three times with DCM to give Fmoc-AA-OP-resin.

In the step (2), the coupling synthesis method is as follows: Fmoc-AA-OP-resin obtained by the reaction of the step (1) is treated with 20% piperidine/DMF (2 times, 10 minutes each time) to remove the α-amino Fmoc protector. The resin is washed three times with DMF and three times with DCM, respectively. The amino acid or side chain carboxylic acid (R₀-COOH), DIC and HOBT are dissolved in DMF and added to the peptide synthesis tube. The reaction is carried out for 120 min at room temperature, and the reaction solution was drawn out by vacuum. With DMF wash the tube three times then with DCM three times. The starting amino acid (i.e., the amino acid at the position-x, x is 5 or 8 or 9) is coupled one after the other, after finishingcoupling with the amino acids, the side chain carboxylic acid is then coupled to the protected polypeptide-resin. The ivDde or Dde protecting group of the amino acid side chain amino group at position-4 was removed with 2% hydrazine hydrate/DMF solution (30 min), washed three times with DMF, and washed three times with DCM, couple the amino acid carboxyl group at position-10 to the amino acid side chain amino group at position-4; Coupling from the 10 amino acid one by one to the former amino acid (x+1 amino acid) of the starting amino acid to obtain a linear fully protected polypeptide-resin. Said one-by-one coupling sequence comprises two parts, the first part being the starting amino acid (ie the amino acid at position-x, x being 5 or 8 or 9) to the amino acid at position-1 and then to the side chain carboxylic acid; the second part being from amino acid 10 to amino acid (x+1). If x is 9, then the first part is in the order of amino acid 8 to amino acid 1, then to the side chain carboxylic acid, and the second part is only the amino acid 10; if x is 8, then the first part is in the order of amino acid 7 to amino acid 1, then to the side chain carboxylic acid, the second part of the sequence is from amino acid 10 to amino acid 9; if x is 5, then the first part is in the order of amino acid 4 to amino acid 1, then to the side chain carboxylic acid, the second part is in the order of amino acid 10 to amino acid 6.

In the step (3), the specific method for selectively removing the protecting group and solid phase cycling is for example shown in the following operation: treating the linear wholly protected polypeptide-resin in the step (2) with 20% piperidine/DMF (2 times, each for 10 min), to remove the α-amino Fmoc protecting group, wash three times with DMF and then with DCM to free the amino group; using a solution of tetrakis (triphenylphosphine) palladium/phenylsilane in DCM/DMF mixed solvent (DCM:DMF = 5:5, volume ratio) to deprotect the carboxyallyl protecting group (120 min) to free the carboxyl group. Dissolve PyAOP and HOAT in DMF, add to NMM, add to the peptide synthesis tube, reacte at room temperature for 3 h, and draw out the reaction solution by vacuum, wash three times with DMF then three times with DCM to obtain a protected cyclic polypeptide-resin.

In the step (4), the specific method to prepare the crude cyclic basic polypeptide by acid hydrolysis is for example as follows: add an acidolysis solution (TFA:H₂O=95:5, volume ratio) to the polypeptide synthesis tube, carry out the acidolysis reaction at room temperature for 120 minutes. Add the acidolysis solution to cold ether (the ratio of TFA lysate to cold ether was 1:20), precipitate the peptide, centrifuge to dry the precipitate to obtain the crude peptide.

In the step (5), the specific method to purify the crude product, form a salt, and lyophilize the product is for example as follows: dissolve the crude product in water, filter through a 0.22 µm pore size filter, and purify by preparative high performance liquid chromatography, the chromatographic packing is 10 µm reversed C₁₈, mobile phase A: 0.1% TFA/aqueous solution, mobile phase B: 0.1% TFA/acetonitrile solution, column dimentions: 22mm × 250mm, flow rate: 10mL/min, detection wavelength: 215 nm, using gradient elution and cycle injection purification. Inject crude product solution to the column, collect the fraction corresponding to the main peak in the chromatogram, and evaporate acetonitrile in the fraction to obtain an aqueous solution of the polymyxin derivative, lyophilize the solution to obtain the product.

The final purity achievable by this method is greater than 95.0%, for example greater than 99.0%. The yield was greater than 40.0% based on the charged resin.

The present invention prepares a new derivative of polymyxin molecular with different amino groups or hydrophobicities, the products of the present invention are easily prepared according to the chemical synthesis methods described above, whereas the polymyxin B and colistin (polymyxin) E) currently in clinic use is a multi-component mixture obtained by a bacterial fermentation process.

**Table 1 Structure of part of the disclosed compounds**

| No. | Structural formula | Molecular formula | MW |
|---|---|---|---|
| 1 | 6-methoxycaproyl-Dab-Thr-Dab-ring(4-10)[Dab-Dab-D-Phe-Leu-Dab-Dab-Thr] | C₅₄H₉₄N₁₆O₁₄ | 1191.42 |
| 2 | *N*,*N*-dimethylaminovaleryl-Dab-Thr-Dab-ring(4-10)[Dab-Dab-D-Phe-Leu-Dab-Dab-Thr] | C₅₄H₉₅N₁₇O₁₃ | 1190.44 |
| 3 | 3-oxocaproyl-Dab-Thr-Dab-ring(4-10)[Dab-Dab-D-Phe-Leu-Dab-Dab-Thr] | C₅₃H₉₀N₁₆O₁₄ | 1175.38 |
| 4 | 3-oxoheptanoyl-Dab-Thr-Dab-ring(4-10)[Dab-Dab-D-Phe-Leu-Dab-Dab-Thr] | C₅₄H₉₂N₁₆O₁₄ | 1189.41 |
| 5 | 3-oxooctanoyl-Dab-Thr-Dab-ring(4-10)[Dab-Dab-D-Phe-Leu-Dab-Dab-Thr] | C₅₅H₉₄N₁₆O₁₄ | 1203.43 |
| 6 | 3-oxononanoyl-Dab-Thr-Dab-ring(4-10)[Dab-Dab-D-Phe-Leu-Dab-Dab-Thr] | C₅₆H₉₆N₁₆O₁₄ | 1217.46 |
| 7 | 3-oxodecanoyl-Dab-Thr-Dab-ring(4-10)[Dab-Dab-D-Phe-Leu-Dab-Dab-Thr] | C₅₇H₉₈N₁₆O₁₄ | 1231.49 |
| 8 | 4-(phenoxy)benzoyl-Dab-Thr-Dab-ring(4-10)[Dab-Dab-D-Phe-Leu-Dab-Dab-Thr] | C₆₀H₉₀N₁₆O₁₄ | 1259.46 |
| 9 | 4-(*p*-methylphenoxy)benzoyl-Dab-Thr-Dab-ring(4-10)[Dab-Dab-D-Phe-Leu-Dab-Dab-Thr] | C₆₁H₉₂N₁₆O₁₄ | 1273.48 |
| 10 | 3-hydroxyl-6-methyloctanoyl-Dab-Thr-Dab-ring(4-10)[Dab-Dab-D-Phe-Leu-Dab-Dab-Thr] | C₅₆H₉₈N₁₆O₁₄ | 1219.48 |
| 11 | (S)-6-methyloctanoyl-Dab-Thr-Dab-ring(4-10)[Dab-Dab-D-Phe(4-NH₂)-Leu-Dab-Dab-Thr] | C₅₆H₉₉N₁₇O₁₃ | 1218.49 |
| 12 | (S)-6-methyloctanoyl-Dab-Thr-Dab-ring(4-10)[Dab-Dab-D-Tyr-Leu-Dab-Dab-Thr] | C₅₆H₉₈N₁₆O₁₄ | 1219.48 |
| 13 | (S)-6-methyloctanoyl-Dab-Thr-Dab-ring(4-10)[Dab-Dab-D-Phe(4-CN)-Leu-Dab-Dab-Thr] | C₅₇H₉₇N₁₇O₁₃ | 1228.49 |
| 14 | (S)-6-methyloctanoyl-Dab-Thr-Dab-ring(4-10)[Dab-Dab-D-Phe(4-NO₂)-Leu-Dab-Dab-Thr] | C₅₆H₉₇N₁₇O₁₅ | 1248.47 |
| 15 | (S)-6-methyloctanoyl-Dab-Thr-Dab-ring(4-10)[Dab-Dab-D-Phe(4-F)-Leu-Dab-Dab-Thr] | C₅₆H₉₇FN₁₆O₁₃ | 1221.47 |
| 16 | (S)-6-methyloctanoyl-Dab-Thr-Dab-ring(4-10)[Dab-Dab-D-Phe(4-Cl)-Leu-Dab-Dab-Thr] | C₅₆H₉₇ClN₁₆O₁₃ | 1237.92 |
| 17 | (S)-6-methyloctanoyl-Dab-Thr-Dab-ring(4-10)[Dab-Dab-D-Phe(4-Br)-Leu-Dab-Dab-Thr] | C₅₆H₉₇BrN₁₆O₁₃ | 1282.37 |
| 18 | (S)-6-methyloctanoyl-Dab-Thr-Dab-ring(4-10)[Dab-Dab-D-Phe(2-Cl)-Leu-Dab-Dab-Thr] | C₅₆H₉₇ClN₁₆O₁₃ | 1237.92 |
| 19 | (S)-6-methyloctanoyl-Dab-Thr-Dab-ring(4-10)[Dab-Dab-D-Phe(3-Cl)-Leu-Dab-Dab-Thr] | C₅₆H₉₇ClN₁₆O₁₃ | 1237.92 |
| 20 | (S)-6-methyloctanoyl-Dab-Thr-Dab-ring(4-10)[Dab-Dab-D-Phe(2,4-dichloro)-Leu-Dab-Dab-Thr] | C₅₆H₉₆Cl₂N₁₆O₁₃ | 1272.37 |
| 21 | (S)-6-methyloctanoyl-Dab-Thr-Dab-ring(4-10)[Dab-Dab-D-Phe(2,3-dichloro)-Leu-Dab-Dab-Thr] | C₅₆H₉₆Cl₂N₁₆O₁₃ | 1272.37 |
| 22 | (S)-6-methyloctanoyl-Dab-Thr-Dab-ring(4-10)[Dab-Dab-D-Phe(3,4-dichloro)-Leu-Dab-Dab-Thr] | C₅₆H₉₆Cl₂N₁₆O₁₃ | 1272.37 |
| 23 | (S)-6-methyloctanoyl-Dab-Thr-Dab-ring(4-10)[Dab-Dab-D-Phe(4-CF₃)-Leu-Dab-Dab-Thr] | C₅₇H₉₇F₃N₁₆O₁₃ | 1271.47 |
| 24 | (S)-6-methyloctanoyl-Dab-Thr-Dab-ring(4-10)[Dab-Dab-D-Phe(4-OCH₃)-Leu-Dab-Dab-Thr] | C₅₇H₁₀₀N₁₆O₁₄ | 1233.50 |
| 25 | (S)-6-methyloctanoyl-Dab-Thr-Dab-ring(4-10)[Dab-Dab-D-Phe(4-OEt)-Leu-Dab-Dab-Thr] | C₅₈H₁₀₂N₁₆O₁₄ | 1247.53 |
| 26 | (S)-6-methyloctanoyl-Dab-Thr-Dab-ring(4-10)[Dab-Dab-D-Phe(4-CH₃)-Leu-Dab-Dab-Thr] | C₅₇H₁₀₀N₁₆O₁₃ | 1217.50 |
| 27 | (S)-6-methyloctanoyl-Dab-Thr-Dab-ring(4-10)[Dab-Dab-D-Phe(4-tBu)-Leu-Dab-Dab-Thr] | C₆₀H₁₀₆N₁₆O₁₃ | 1259.58 |
| 28 | (S)-6-methyloctanoyl-Dab-Thr-Dab-ring(4-10)[Dab-Dab-D-Phe(4-benzyl)-Leu-Dab-Dab-Thr] | C₆₃H₁₀₄N₁₆O₁₃ | 1293.60 |
| 29 | (S)-6-methyloctanoyl-Dab-Thr-Dab-ring(4-10)[Dab-Dab-D-Phe(4-benzoyl)-Leu-Dab-Dab-Thr] | C₆₃H₁₀₂N₁₆O₁₄ | 1307.58 |
| 30 | (S)-6-methyloctanoyl-Dab-Thr-D-Dab-ring(4-10)[Dab-Dab-D-Leu-Thr-Dab-Dab-Thr] | C₅₁H₉₆N₁₆O₁₄ | 1157.41 |
| 31 | 6-methylheptanoyl-Dab-Thr-D-Dab-ring(4-10)[Dab-Dab-D-Leu-Thr-Dab-Dab-Thr] | C₅₀H₉₄N₁₆O₁₄ | 1143.38 |
| 32 | octanoyl-Dab-Thr-D-Dab-ring(4-10)[Dab-Dab-D-Leu-Thr-Dab-Dab-Thr] | C₅₀H₉₄N₁₆O₁₄ | 1143.38 |
| 33 | heptanoyl-Dab-Thr-D-Dab-ring(4-10)[Dab-Dab-D-Leu-Thr-Dab-Dab-Thr] | C₄₉H₉₂N₁₆O₁₄ | 1129.35 |
| 34 | nonanoyl-Dab-Thr-D-Dab-ring(4-10)[Dab-Dab-D-Leu-Thr-Dab-Dab-Thr] | C₅₁H₉₆N₁₆O₁₄ | 1157.41 |
| 35 | octanoyl-Dab-Ser-D-Dab-ring(4-10)[Dab-Dab-D-Leu-Thr-Dab-Dab-Thr] | C₄₉H₉₂N₁₆O₁₄ | 1129.35 |
| 36 | octanoyl-Dab-Thr-D-Dab-ring(4-10)[Dab-Dab-D-Leu-Ser-Dab-Dab-Thr] | C₄₉H₉₂N₁₆O₁₄ | 1129.35 |
| 37 | octanoyl-Dab-Thr-D-Dab-ring(4-10)[Dab-Dab-Thr-Thr-Dab-Dab-Thr] | C₄₈H₉₀N₁₆O₁₅ | 1131.33 |
| 38 | octanoyl-Ser-Thr-D-Dab-ring(4-10)[Dab-Dab-D-Leu-Thr-Dab-Dab-Thr] | C₄₉H₉₁N₁₅O₁₅ | 1130.34 |
| 39 | octanoyl-Dab-Thr-D-Dab-ring(4-10)[Dab-Ser-D-Leu-Thr-Dab-Dab-Thr] | C₄₉H₉₁N₁₅O₁₅ | 1130.34 |
| 40 | octanoyl-Dab-Thr-D-Dab-ring(4-10)[Dab-Dab-D-Leu-Thr-Ser-Dab-Thr] | C₄₉H₉₁N₁₅O₁₅ | 1130.34 |
| 41 | octanoyl-Dab-Thr-D-Dab-ring(4-10)[Dab-Dab-D-Leu-Thr-Dab-Ser-Thr] | C₄₉H₉₁N₁₅O₁₅ | 1130.34 |
| 42 | (S)-6-methyloctanoyl-Dab-Thr-Dab-ring(4-10)[Dab-Dab-D-Phe-Leu-Dab-Dab-Thr] | C₅₆H₉₈N₁₆O₁₃ | 1203.48 |
| 43 | 6-methylheptanoyl-Dab-Thr-Dab-ring(4-10)[Dab-Dab-D-Phe-Leu-Dab-Dab-Thr] | C₅₅H₉₆N₁₆O₁₃ | 1189.45 |
| 44 | octanoyl-Dab-Thr-Dab-ring(4-10)[Dab-Dab-D-Phe-Leu-Dab-Dab-Thr] | C₅₅H₉₆N₁₆O₁₃ | 1189.45 |
| 45 | heptanoyl-Dab-Thr-Dab-ring(4-10)[Dab-Dab-D-Phe-Leu-Dab-Dab-Thr] | C₅₄H₉₄N₁₆O₁₃ | 1175.42 |
| 46 | nonanoyl-Dab-Thr-Dab-ring(4-10)[Dab-Dab-D-Phe-Leu-Dab-Dab-Thr] | C₅₆H₉₈N₁₆O₁₃ | 1203.48 |
| 47 | (S)-6-methyloctanoyl-Dab-Thr-Dab-ring(4-10)[Dab-Dab-D-Phe-Ile-Dab-Dab-Thr] | C₅₆H₉₈N₁₆O₁₃ | 1203.48 |
| 48 | (S)-6-methyloctanoyl-Dab-Thr-Dab-ring(4-10)[Dab-Dab-Thr-Leu-Dab-Dab-Thr] | C₅₁H₉₆N₁₆O₁₄ | 1157.41 |
| 49 | octanoyl-Dab-Ser-Dab-ring(4-10)[Dab-Dab-D-Phe-Leu-Dab-Dab-Thr] | C₅₄H₉₄N₁₆O₁₃ | 1175.42 |
| 50 | octanoyl-Dab-Thr-Dab-ring(4-10)[Dab-Dab-D-Phe-Leu-Dab-Dab-Ser] | C₅₄H₉₄N₁₆O₁₃ | 1175.42 |
| 51 | octanoyl-Dab-Ser-Dab-ring(4-10)[Dab-Dab-D-Phe-Ile-Dab-Dab-Thr] | C₅₄H₉₄N₁₆O₁₃ | 1175.42 |
| 52 | octanoyl-Dab-Thr-Dab-ring(4-10)[Dab-Dab-D-Phe-Ile-Dab-Dab-Ser] | C₅₄H₉₄N₁₆O₁₃ | 1175.42 |
| 53 | octanoyl-Ser-Thr-Dab-ring(4-10)[Dab-Dab-D-Phe-Leu-Dab-Dab-Thr] | C₅₄H₉₃N₁₅O₁₄ | 1176.41 |
| 54 | octanoyl-Dab-Thr-Ser-ring(4-10)[Dab-Dab-D-Phe-Leu-Dab-Dab-Thr] | C₅₄H₉₃N₁₅O₁₄ | 1176.41 |
| 55 | octanoyl-Dab-Thr-Dab-ring(4-10)[Dab-Ser-D-Phe-Leu-Dab-Dab-Thr] | C₅₄H₉₃N₁₅O₁₄ | 1176.41 |
| 56 | octanoyl-Dab-Thr-Dab-ring(4-10)[Dab-Dab-D-Phe-Leu-Ser-Dab-Thr] | C₅₄H₉₃N₁₅O₁₄ | 1176.41 |
| 57 | octanoyl-Dab-Thr-Dab-ring(4-10)[Dab-Dab-D-Phe-Leu-Dab-Ser-Thr] | C₅₄H₉₃N₁₅O₁₄ | 1176.41 |
| 58 | (S)-6-methyloctanoyl-Dab-Thr-D-Ser-ring(4-10)[Dab-Dab-D-Leu-Thr-Dab-Dab-Thr] | C₅₀H₉₃N₁₅O₁₅ | 1144.36 |
| 59 | 6-methylheptanoyl-Dab-Thr-D-Ser-ring(4-10)[Dab-Dab-D-Leu-Thr-Dab-Dab-Thr] | C₄₉H₉₁N₁₅O₁₅ | 1130.34 |
| 60 | octanoyl-Dab-Thr-D-Ser-ring(4-10)[Dab-Dab-D-Leu-Thr-Dab-Dab-Thr] | C₄₉H₉₁N₁₅O₁₅ | 113034 |
| 61 | heptanoyl-Dab-Thr-D-Ser-ring(4-10)[Dab-Dab-D-Leu-Thr-Dab-Dab-Thr] | C₄₈H₈₉N₁₅O₁₅ | 1116.31 |
| 62 | nonanoyl-Dab-Thr-D-Ser-ring(4-10)[Dab-Dab-D-Leu-Thr-Dab-Dab-Thr] | C₅₀H₉₃N₁₅O₁₅ | 1144.36 |
| 63 | octanoyl-Dab-Ser-D-Ser-ring(4-10)[Dab-Dab-D-Leu-Thr-Dab-Dab-Thr] | C₄₈H₈₉N₁₅O₁₅ | 1116.31 |
| 64 | octanoyl-Dab-Thr-D-Ser-ring(4-10)[Dab-Dab-D-Leu-Ser-Dab-Dab-Thr] | C₄₈H₈₉N₁₅O₁₅ | 1116.31 |
| 65 | octanoyl-Dab-Thr-D-Ser-ring(4-10)[Dab-Dab-D-Leu-Thr-Dab-Dab-Ser] | C₄₈H₈₉N₁₅O₁₅ | 1116.31 |
| 66 | octanoyl-Ser-Thr-D-Ser-ring(4-10)[Dab-Dab-D-Leu-Thr-Dab-Dab-Thr] | C₄₈H₈₈N₁₄O₁₆ | 1117.30 |
| 67 | octanoyl-Dab-Thr-D-Ser-ring(4-10)[Dab-Ser-D-Leu-Thr-Dab-Dab-Thr] | C₄₈H₈₈N₁₄O₁₆ | 1117.30 |
| 68 | octanoyl-Dab-Thr-D-Ser-ring(4-10)[Dab-Dab-D-Leu-Thr-Ser-Dab-Thr] | C₄₈H₈₈N₁₄O₁₆ | 1117.30 |
| 69 | octanoyl-Dab-Thr-D-Ser-ring(4-10)[Dab-Dab-D-Leu-Thr-Dab-Ser-Thr] | C₄₈H₈₈N₁₄O₁₆ | 1117.30 |
| 70 | (S)-6-methyloctanoyl-Dab-Thr-Dab-ring(4-10)[Dab-Dab-D-Leu-Leu-Dab-Dab-Thr] | C₅₃H₁₀₀N₁₆O₁₃ | 1169.46 |
| 71 | (S)-6-methyloctanoyl-Dab-Thr-Dab-ring(4-10)[Dab-Dab-D-Leu-Ile-Dab-Dab-Thr] | C₅₃H₁₀₀N₁₆O₁₃ | 1169.46 |
| 72 | (S)-6-methyloctanoyl-Dab-Thr-Dab-ring(4-10)[Dab-Dab-D-Leu-Val-Dab-Dab-Thr] | C₅₂H₉₈N₁₆O₁₃ | 1155.43 |
| 73 | (S)-6-methyloctanoyl-Dab-Thr-Dab-ring(4-10)[Dab-Dab-D-Leu-Nva-Dab-Dab-Thr] | C₅₂H₉₈N₁₆O₁₃ | 1155.43 |
| 74 | 6-methylheptanoyl-Dab-Thr-Dab-ring(4-10)[Dab-Dab-D-Leu-Leu-Dab-Dab-Thr] | C₅₂H₉₈N₁₆O₁₃ | 1155.43 |
| 75 | 6-methylheptanoyl-Dab-Thr-Dab-ring(4-10)[Dab-Dab-D-Leu-Ile-Dab-Dab-Thr] | C₅₂H₉₈N₁₆O₁₃ | 1155.43 |
| 76 | 6-methylheptanoyl-Dab-Thr-Dab-ring(4-10)[Dab-Dab-D-Leu-Val-Dab-Dab-Thr] | C₅₁H₉₆N₁₆O₁₃ | 1141.41 |
| 77 | 6-methylheptanoyl-Dab-Thr-Dab-ring(4-10)[Dab-Dab-D-Leu-Nva-Dab-Dab-Thr] | C₅₁H₉₆N₁₆O₁₃ | 1141.41 |
| 78 | octanoyl-Dab-Thr-Dab-ring(4-10)[Dab-Dab-D-Leu-Leu-Dab-Dab-Thr] | C₅₂H₉₈N₁₆O₁₃ | 1155.43 |
| 79 | octanoyl-Dab-Thr-Dab-ring(4-10)[Dab-Dab-D-Leu-Ile-Dab-Dab-Thr] | C₅₂H₉₈N₁₆O₁₃ | 1155.43 |
| 80 | octanoyl-Dab-Thr-Dab-ring(4-10)[Dab-Dab-D-Leu-Val-Dab-Dab-Thr] | C₅₁H₉₆N₁₆O₁₃ | 1141.41 |
| 81 | octanoyl-Dab-Thr-Dab-ring(4-10)[Dab-Dab-D-Leu-Nva-Dab-Dab-Thr] | C₅₁H₉₆N₁₆O₁₃ | 1141.41 |
| 82 | heptanoyl-Dab-Thr-Dab-ring(4-10)[Dab-Dab-D-Leu-Leu-Dab-Dab-Thr] | C₅₁H₉₆N₁₆O₁₃ | 1141.41 |
| 83 | heptanoyl-Dab-Thr-Dab-ring(4-10)[Dab-Dab-D-Leu-Ile-Dab-Dab-Thr] | C₅₁H₉₆N₁₆O₁₃ | 1141.41 |
| 84 | heptanoyl-Dab-Thr-Dab-ring(4-10)[Dab-Dab-D-Leu-Val-Dab-Dab-Thr] | C₅₀H₉₄N₁₆O₁₃ | 1127.38 |
| 85 | heptanoyl-Dab-Thr-Dab-ring(4-10)[Dab-Dab-D-Leu-Nva-Dab-Dab-Thr] | C₅₀H₉₄N₁₆O₁₃ | 1127.38 |
| 86 | 7-methyloctanoyl-Dab-Thr-Dab-ring(4-10)[Dab-Dab-D-Leu-Leu-Dab-Dab-Thr] | C₅₃H₁₀₀N₁₆O₁₃ | 1169.46 |
| 87 | octanoyl-Dab-Ser-Dab-ring(4-10)[Dab-Dab-D-Leu-Leu-Dab-Dab-Thr] | C₅₁H₉₆N₁₆O₁₃ | 1141.41 |
| 88 | octanoyl-Dab-Thr-Dab-ring(4-10)[Dab-Dab-D-Leu-Leu-Dab-Dab-Ser] | C₅₁H₉₆N₁₆O₁₃ | 1141.41 |
| 89 | octanoyl-Dab-Ser-Dab-ring(4-10)[Dab-Dab-D-Leu-Ile-Dab-Dab-Thr] | C₅₁H₉₆N₁₆O₁₃ | 1141.41 |
| 90 | octanoyl-Dab-Thr-Dab-ring(4-10)[Dab-Dab-D-Leu-Ile-Dab-Dab-Ser] | C₅₁H₉₆N₁₆O₁₃ | 1141.41 |
| 91 | octanoyl-Dab-Ser-Dab-ring(4-10)[Dab-Dab-D-Leu-Val-Dab-Dab-Thr] | C₅₀H₉₄N₁₆O₁₃ | 1127.38 |
| 92 | octanoyl-Dab-Thr-Dab-ring(4-10)[Dab-Dab-D-Leu-Val-Dab-Dab-Ser] | C₅₀H₉₄N₁₆O₁₃ | 1127.38 |
| 93 | octanoyl-Dab-Ser-Dab-ring(4-10)[Dab-Dab-D-Leu-Nva-Dab-Dab-Thr] | C₅₀H₉₄N₁₆O₁₃ | 1127.38 |
| 94 | octanoyl-Dab-Thr-Dab-ring(4-10)[Dab-Dab-D-Leu-Nva-Dab-Dab-Ser] | C₅₀H₉₄N₁₆O₁₃ | 1127.38 |
| 95 | 7-methyloctanoyl-Dab-Ser-Dab-ring(4-10)[Dab-Dab-D-Leu-Leu-Dab-Dab-Thr] | C₅₂H₉₈N₁₆O₁₃ | 1155.43 |
| 96 | 7-methyloctanoyl-Dab-Thr-Dab-ring(4-10)[Dab-Dab-D-Leu-Leu-Dab-Dab-Ser] | C₅₂H₉₈N₁₆O₁₃ | 1155.43 |
| 97 | octanoyl-Ser-Thr-Dab-ring(4-10)[Dab-Dab-D-Leu-Leu-Dab-Dab-Thr] | C₅₁H₉₅N₁₅O₁₄ | 1142.39 |
| 98 | octanoyl-Dab-Thr-Ser-ring(4-10)[Dab-Dab-D-Leu-Leu-Dab-Dab-Thr] | C₅₁H₉₅N₁₅O₁₄ | 1142.39 |
| 99 | octanoyl-Dab-Thr-Dab-ring(4-10)[Dab-Ser-D-Leu-Leu-Dab-Dab-Thr] | C₅₁H₉₅N₁₅O₁₄ | 1142.39 |
| 100 | octanoyl-Dab-Thr-Dab-ring(4-10)[Dab-Dab-D-Leu-Leu-Ser-Dab-Thr] | C₅₁H₉₅N₁₅O₁₄ | 1142.39 |
| 101 | octanoyl-Dab-Thr-Dab-ring(4-10)[Dab-Dab-D-Leu-Leu-Dab-Ser-Thr] | C₅₁H₉₅N₁₅O₁₄ | 1142.39 |
| 102 | (S)-6-methyloctanoyl-Dab-Thr-Dab-ring(4-10)[Dab-Dab-D-Leu-Thr-Dab-Dab-Thr] | C₅₁H₉₆N₁₆O₁₄ | 1157.41 |
| 103 | 6-methylheptanoyl-Dab-Thr-Dab-ring(4-10)[Dab-Dab-D-Leu-Thr-Dab-Dab-Thr] | C₅₀H₉₄N₁₆O₁₄ | 1143.38 |
| 104 | octanoyl-Dab-Thr-Dab-ring(4-10)[Dab-Dab-D-Leu-Thr-Dab-Dab-Thr] | C₅₀H₉₄N₁₆O₁₄ | 1143.38 |
| 105 | heptanoyl-Dab-Thr-Dab-ring(4-10)[Dab-Dab-D-Leu-Thr-Dab-Dab-Thr] | C₄₉H₉₂N₁₆O₁₄ | 1129.35 |
| 106 | nonanoyl-Dab-Thr-Dab-ring(4-10)[Dab-Dab-D-Leu-Thr-Dab-Dab-Thr] | C₅₁H₉₆N₁₆O₁₄ | 1157.41 |
| 107 | octanoyl-Dab-Ser-Dab-ring(4-10)[Dab-Dab-D-Leu-Thr-Dab-Dab-Thr] | C₄₉H₉₂N₁₆O₁₄ | 1129.35 |
| 108 | octanoyl-Dab-Thr-Dab-ring(4-10)[Dab-Dab-D-Leu-Ser-Dab-Dab-Thr] | C₄₉H₉₂N₁₆O₁₄ | 1129.35 |
| 109 | octanoyl-Dab-Thr-Dab-ring(4-10)[Dab-Dab-D-Leu-Thr-Dab-Dab-Ser] | C₄₉H₉₂N₁₆O₁₄ | 1129.35 |
| 110 | octanoyl-Ser-Thr-Dab-ring(4-10)[Dab-Dab-D-Leu-Thr-Dab-Dab-Thr] | C₄₉H₉₁N₁₅O₁₅ | 113034 |
| 111 | octanoyl-Dab-Thr-Ser-ring(4-10)[Dab-Dab-D-Leu-Thr-Dab-Dab-Thr] | C₄₉H₉₁N₁₅O₁₅ | 1130.34 |
| 112 | octanoyl-Dab-Thr-Dab-ring(4-10)[Dab-Ser-D-Leu-Thr-Dab-Dab-Thr] | C₄₉H₉₁N₁₅O₁₅ | 1130.34 |
| 113 | octanoyl-Dab-Thr-Dab-ring(4-10)[Dab-Dab-D-Leu-Thr-Ser-Dab-Thr] | C₄₉H₉₁N₁₅O₁₅ | 1130.34 |
| 114 | octanoyl-Dab-Thr-Dab-ring(4-10)[Dab-Dab-D-Leu-Thr-Dab-Ser-Thr] | C₄₉H₉₁N₁₅O₁₅ | 1130.34 |
| 115 | (S)-6-methyloctanoyl-Dab-Thr-D-Ser-ring(4-10)[Dab-Dab-D-Phe-Thr-Dab-Dab-Thr] | C₅₃H₉₁N₁₅O₁₅ | 1178.38 |
| 116 | 6-methylheptanoyl-Dab-Thr-D-Ser-ring(4-10)[Dab-Dab-D-Phe-Thr-Dab-Dab-Thr] | C₅₂H₈₉N₁₅O₁₅ | 1164.35 |
| 117 | octanoyl-Dab-Thr-D-Ser-ring(4-10)[Dab-Dab-D-Phe-Thr-Dab-Dab-Thr] | C₅₂H₈₉N₁₅O₁₅ | 1164.35 |
| 118 | heptanoyl-Dab-Thr-D-Ser-ring(4-10)[Dab-Dab-D-Phe-Thr-Dab-Dab-Thr] | C₅₁H₈₇N₁₅O₁₅ | 1150.33 |
| 119 | nonanoyl-Dab-Thr-D-Ser-ring(4-10)[Dab-Dab-D-Phe-Thr-Dab-Dab-Thr] | C₅₃H₉₁N₁₅O₁₅ | 1178.38 |
| 120 | octanoyl-Dab-Ser-D-Ser-ring(4-10)[Dab-Dab-D-Phe-Thr-Dab-Dab-Thr] | C₅₁H₈₇N₁₅O₁₅ | 1150.33 |
| 121 | octanoyl-Dab-Thr-D-Ser-ring(4-10)[Dab-Dab-D-Phe-Ser-Dab-Dab-Thr] | C₅₁H₈₇N₁₅O₁₅ | 1150.33 |
| 122 | octanoyl-Dab-Thr-D-Ser-ring(4-10)[Dab-Dab-D-Phe-Thr-Dab-Dab-Ser] | C₅₁H₈₇N₁₅O₁₅ | 1150.33 |
| 123 | octanoyl-Ser-Thr-D-Ser-ring(4-10)[Dab-Dab-D-Phe-Thr-Dab-Dab-Thr] | C₅₁H₈₆N₁₄O₁₆ | 1151.31 |
| 124 | octanoyl-Dab-Thr-D-Ser-ring(4-10)[Dab-Ser-D-Phe-Thr-Dab-Dab-Thr] | C₅₁H₈₆N₁₄O₁₆ | 1151.31 |
| 125 | octanoyl-Dab-Thr-D-Ser-ring(4-10)[Dab-Dab-D-Phe-Thr-Ser-Dab-Thr] | C₅₁H₈₆N₁₄O₁₆ | 1151.31 |
| 126 | octanoyl-Dab-Thr-D-Ser-ring(4-10)[Dab-Dab-D-Phe-Thr-Dab-Ser-Thr] | C₅₁H₈₆N₁₄O₁₆ | 1151.31 |
| 127 | (S)-6-methyloctanoyl-Dab-Thr-Dab-ring(4-10)[Dab-Dab-D-Phe-Leu-Dab-Dab-Leu] | C₅₈H₁₀₂N₁₆O₁₂ | 1215.53 |
| 128 | 6-methylheptanoyl-Dab-Thr-Dab-ring(4-10)[Dab-Dab-D-Phe-Leu-Dab-Dab-Leu] | C₅₇H₁₀₀N₁₆O₁₂ | 1201.50 |
| 129 | octanoyl-Dab-Thr-Dab-ring(4-10)[Dab-Dab-D-Phe-Leu-Dab-Dab-Leu] | C₅₇H₁₀₀N₁₆O₁₂ | 1201.50 |
| 130 | heptanoyl-Dab-Thr-Dab-ring(4-10)[Dab-Dab-D-Phe-Leu-Dab-Dab-Leu] | C₅₆H₉₈N₁₆O₁₂ | 1187.48 |
| 131 | nonanoyl-Dab-Thr-Dab-ring(4-10)[Dab-Dab-D-Phe-Leu-Dab-Dab-Leu] | C₅₈H₁₀₂N₁₆O₁₂ | 1215.53 |
| 132 | octanoyl-Dab-Ser-Dab-ring(4-10)[Dab-Dab-D-Phe-Leu-Dab-Dab-Leu] | C₅₆H₉₈N₁₆O₁₂ | 1187.48 |
| 133 | octanoyl-Ser-Thr-Dab-ring(4-10)[Dab-Dab-D-Phe-Leu-Dab-Dab-Leu] | C₅₆H₉₇N₁₅O₁₃ | 1188.46 |
| 134 | octanoyl-Dab-Thr-Ser-ring(4-10)[Dab-Dab-D-Phe-Leu-Dab-Dab-Leu] | C₅₆H₉₇N₁₅O₁₃ | 1188.46 |
| 135 | octanoyl-Dab-Thr-Dab-ring(4-10)[Dab-Ser-D-Phe-Leu-Dab-Dab-Leu] | C₅₆H₉₇N₁₅O₁₃ | 1188.46 |
| 136 | octanoyl-Dab-Thr-Dab-ring(4-10)[Dab-Dab-D-Phe-Leu-Ser-Dab-Leu] | C₅₆H₉₇N₁₅O₁₃ | 1188.46 |
| 137 | octanoyl-Dab-Thr-Dab-ring(4-10)[Dab-Dab-D-Phe-Leu-Dab-Ser-Leu] | C₅₆H₉₇N₁₅O₁₃ | 1188.46 |
| 138 | (S)-6-methyloctanoyl-Dab-Thr-Dab-ring(4-10)[Dab-Dab-Phe-Thr-Dab-Dab-Thr] | C₅₄H₉₄N₁₆O₁₄ | 1191.42 |
| 139 | 6-methylheptanoyl-Dab-Thr-Dab-ring(4-10)[Dab-Dab-Phe-Thr-Dab-Dab-Thr] | C₅₃H₉₂N₁₆O₁₄ | 1177.40 |
| 140 | octanoyl-Dab-Thr-Dab-ring(4-10)[Dab-Dab-Phe-Thr-Dab-Dab-Thr] | C₅₃H₉₂N₁₆O₁₄ | 1177.40 |
| 141 | heptanoyl-Dab-Thr-Dab-ring(4-10)[Dab-Dab-Phe-Thr-Dab-Dab-Thr] | C₅₂H₉₀N₁₆O₁₄ | 1163.37 |
| 142 | nonanoyl-Dab-Thr-Dab-ring(4-10)[Dab-Dab-Phe-Thr-Dab-Dab-Thr] | C₅₄H₉₄N₁₆O₁₄ | 1191.42 |
| 143 | (S)-6-methyloctanoyl-Dab-Thr-D-Dab-ring(4-10)[Dab-Dab-D-Phe-Thr-Dab-Dab-Thr] | C₅₄H₉₄N₁₆O₁₄ | 1191.42 |
| 144 | 6-methylheptanoyl-Dab-Thr-D-Dab-ring(4-10)[Dab-Dab-D-Phe-Thr-Dab-Dab-Thr] | C₅₃H₉₂N₁₆O₁₄ | 1177.40 |
| 145 | octanoyl-Dab-Thr-D-Dab-ring(4-10)[Dab-Dab-D-Phe-Thr-Dab-Dab-Thr] | C₅₃H₉₂N₁₆O₁₄ | 1177.40 |
| 146 | heptanoyl-Dab-Thr-D-Dab-ring(4-10)[Dab-Dab-D-Phe-Thr-Dab-Dab-Thr] | C₅₂H₉₀N₁₆O₁₄ | 1163.37 |
| 147 | nonanoyl-Dab-Thr-D-Dab-ring(4-10)[Dab-Dab-D-Phe-Thr-Dab-Dab-Thr] | C₅₄H₉₄N₁₆O₁₄ | 1191.42 |
| 148 | octanoyl-Thr-Thr-Thr-ring(4-10)[Dab-Dab-D-Phe-Leu-Dab-Dab-Thr] | C₅₅H₉₄N₁₄O₁₅ | 1191.42 |
| 149 | octanoyl-Dap-Thr-Dab-ring(4-10)[Dab-Dab-D-Phe-Leu-Dab-Dab-Thr] | C₅₄H₉₄N₁₆O₁₃ | 1175.42 |
| 150 | octanoyl-Arg-Thr-Dab-ring(4-10)[Dab-Dab-D-Phe-Leu-Dab-Dab-Thr] | C₅₇H₁₀₀N₁₈O₁₃ | 1245.52 |
| 151 | octanoyl-Dab-Thr-Met-ring(4-10)[Dab-Dab-D-Phe-Leu-Dab-Dab-Thr] | C₅₆H₉₇N₁₅O₁₃S | 1220.53 |
| 152 | octanoyl-Dab-Thr-Dab-ring(4-10)[Dab-Dap-D-Phe-Leu-Dap-Dap-Thr] | C₅₂H₉₀N₁₆O₁₃ | 1147.370 |

The present invention also provides said polymyxin derivatives described therein for use as an antibacterial agent against Gram-negative bacteria and Gram-positive bacteria of, or a pharmaceutically acceptable salt thereof. Pharmaceutical-related Gram-negative bacteria include Escherichia coli, Klebsiella pneumoniae, Pseudomonas aeruginosa, Acinetobacter baumanii, Salmonella, Moraxella, Helicobacter, Legionella, Haemophilus influenzae, Enterobacter cloacae, Enterobacter aerogenes, sticky Serratia marcescens, Morganella morganii, Providentia rettgeri, Proteus vulgaris, Proteus mirabilis, Stenotrophomonas maltophilia, Citrobacter freundii, and the like. Pharmaceutical-related Gram-positive bacteria include Staphylococcus epidermidis, Staphylococcus aureus, Enterococcus faecalis, Enterococcus faecium, and the like.

Gram-negative bacteria for example, Escherichia coli, Klebsiella pneumoniae, Pseudomonas aeruginosa, and Acinetobacter baumanii. Gram-positive bacteria for example, Staphylococcus epidermidis and Staphylococcus aureus.

The present invention also provides a polymyxin derivative, or a pharmaceutically acceptable salt thereof, having a higher antibacterial activity and a lower renal cytotoxicity than the clinically used polymyxin B and colistin (polymyxin E). The renal cells are selected from the group consisting of human renal tubular epithelial cells (HK-2 cells), human embryonic kidney epithelial cells (HEK293 cells), African green monkey kidney cells (Vero cells), canine kidney cells (MDCK cells), for example, African green monkey kidney cells (Vero cells).

The present invention also provides an antibacterial pharmaceutical composition comprising a therapeutically effective amount of a polymyxin derivative or a pharmaceutically acceptable salt thereof as an active ingredient, which may be the compound itself or its mixture with pharmaceutically acceptable excipient, diluent, etc. The mixture is administered orally in the form of tablets, capsules, granules, powder or syrup, or parenterally in the form of an injection, a spray, an aerosol, an ointment or an eye drop.

The above formulations can be prepared by conventional pharmaceutical methods. Examples of useful pharmaceutically acceptable excipients and diluents include excipients (e.g., saccharide derivatives for example, lactose, sucrose, glucose, mannitol, and sorbitol; starch derivatives for example, corn starch, potato starch, dextrin, and carboxymethyl starch; cellulose derivatives for example, crystalline cellulose, hydroxypropyl cellulose, hydroxymethyl cellulose, calcium hydroxymethyl cellulose, sodium hydroxymethyl cellulose; gum arabic; dextran; silicate derivatives for example, magnesium aluminum metasilicate, phosphate derivatives for example, calcium phosphate; carbonate derivatives for example, calcium carbonate; sulfate derivatives for example, calcium sulfate; and binders for example, gelatin, polyvinylpyrrolidone and polyethylene glycol; Disintegrators (for example, cellulose derivatives for example, sodium carboxymethylcellulose, polyvinylpyrrolidone); lubricants (for example, talc, calcium stearate, magnesium stearate, cetyl, boric acid, sodium benzoate, leucine), stabilizers (methyl *p*-hydroxybenzoate, propyl paraben, etc.); flavoring agents (for example, commonly used sweeteners, sour agents and perfumes); diluents and injection solvents (eg water, ethanol and glycerin, etc).

### Embodiments

Synthetic scheme 1: Using protected position-5 amino acid Fmoc-Dab-OP as the starting amino acid, its structure is as shown in Formula III: 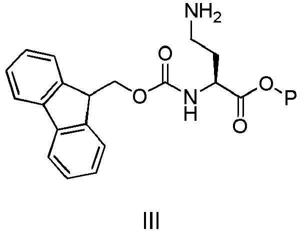 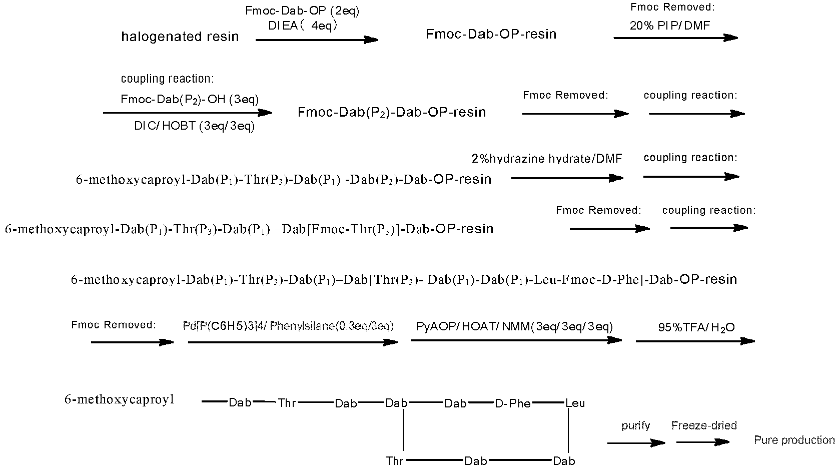
Synthetic scheme 2: Using protected position-8 amino acid Fmoc-Dab-OP as the starting amino acid, its structure is as shown in Formula III: 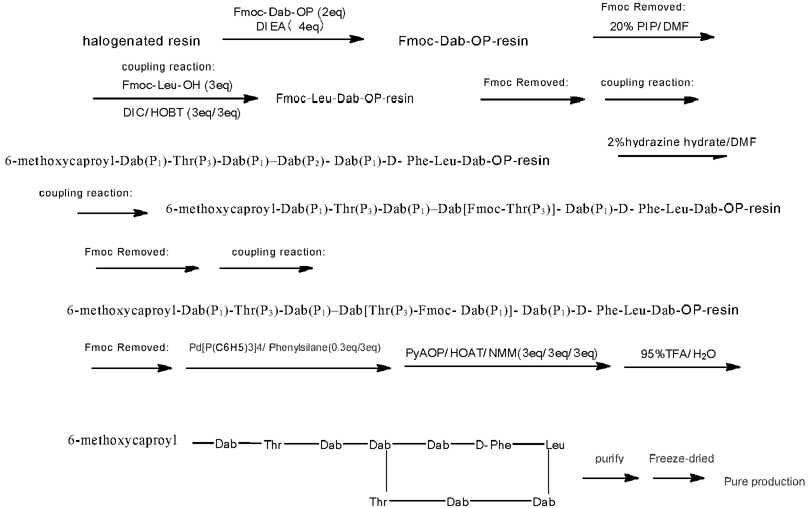
Synthetic scheme 3: Using protected position-9 amino acid Fmoc-Dab-OP as the starting amino acid, its structure is as shown in Formula III: 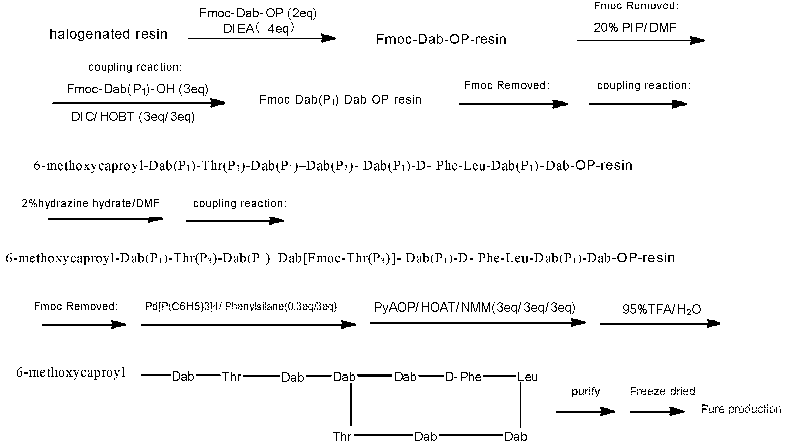
   P stands for: Allyl
   P₁ represents: tert-butoxycarbonyl (Boc)
   P₂ stands for: 1-(4,4-dimethyl-2,6-dioxocylohexylidene)ethyl (Dde), 1-(4,4-dimethyl-2,6-dioxocyclohexylidene)-3-methylbutyl(ivDde)
   P₃ stands for: tert-butyl (tBu)
   Fmoc stands for: 9-fluorenylmethoxycarbonyl

Compared with the existing synthetic methods, the method put forward in this invention has wider application range, is greener and more environmentally friendly, has higher purity of the crude peptide obtained, is easier to be separated and purified, and the total yield is as high as 40%.

### Embodiment 1: Preparation of 6-methoxyhexanoyl-Dab-Thr-Dab-ring(4-10) [Dab-Dab-D-Phe-Leu-Dab-Dab-Thr] (Compound 1)

Synthetic scheme 1: Sequence of addition of the protected amino acid and the side chain carboxylic acid to the synthetic route is Fmoc-Dab-OAllyl, Fmoc-Dab(Dde)-OH, Fmoc-Dab(Boc)-OH, Fmoc-Thr(tBu)-OH, Fmoc-Dab(Boc)-OH, 6-methoxyhexanoic acid, Fmoc-Thr(tBu)-OH, Fmoc-Dab(Boc)-OH, Fmoc-Dab(Boc)-OH, Fmoc-Leu-OH, Fmoc-D-Phe-OH

Synthetic scheme 2: Sequence of addition of the protected amino acid and the side chain carboxylic acid to the synthetic route is Fmoc-Dab-OAllyl, Fmoc-Leu-OH, Fmoc-D-Phe-OH, Fmoc-Dab(Boc)-OH, Fmoc-Dab (Dde)-OH, Fmoc-Dab(Boc)-OH, Fmoc-Thr(tBu)-OH, Fmoc-Dab(Boc)-OH, 6-methoxyhexanoic acid, Fmoc-Thr(tBu)-OH, Fmoc-Dab(Boc)-OH

Synthetic scheme 3: Sequence of addition of the protected amino acid and the side chain carboxylic acid to the synthetic route is Fmoc-Dab-OAllyl, Fmoc-Dab(Boc)-OH, Fmoc-Leu-OH, Fmoc-D-Phe-OH, Fmoc-Dab (Boc)-OH, Fmoc-Dab(Dde)-OH, Fmoc-Dab(Boc)-OH, Fmoc-Thr(tBu)-OH, Fmoc-Dab(Boc)-OH, 6-methoxyhexanoic acid, Fmoc-Thr(tBu)-OH

2-Cl-Trt resin (0.5 mmol, degree of substitution: 0.5 mmol/g) was added into the peptide solid phase synthesis tube to prepare 6-methoxyhexanoyl-Dab-Thr-Dab-ring(4-10)[Dab-Dab-D-Phe -Leu-Dab-Dab-Thr] according to the synthetic scheme 1, 2 and 3, respectively.

Crude peptide obtained: 530 mg, yield: 89.0%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 238 mg product. Yield: 40.0% (calculated on the basis of 0.5 mmol 2-Cl-Trt resin used).

Characterization of the purified peptide: purity (area integral by HPLC profile) > 99.0%; ESI: m/z = 596.36 ([M+2H⁺]²⁺).

Embodiment 2: Preparation of *N*, *N*-dimethylaminovaleryl-Dab-Thr-Dab-ring(4-10)[Dab-Dab-D-Phe-Leu-Dab-Dab-Thr] (Compound 2)

Synthetic scheme 1: Sequence of addition of the protected amino acid and the side chain carboxylic acid to the synthetic route is: Fmoc-Dab-OAllyl, Fmoc-Dab(Dde)-OH, Fmoc-Dab(Boc)-OH, Fmoc-Thr(tBu)-OH, Fmoc-Dab(Boc)-OH, *N*, *N*-dimethylaminopentanoic acid, Fmoc-Thr(tBu)-OH, Fmoc-Dab(Boc)-OH, Fmoc-Dab(Boc)-OH, Fmoc-Leu-OH, Fmoc-D-Phe-OH

Synthetic scheme 2: Sequence of addition of the protected amino acid and the side chain carboxylic acid to the synthetic route is: Fmoc-Dab-OAllyl, Fmoc-Leu-OH, Fmoc-D-Phe-OH, Fmoc-Dab(Boc)-OH, Fmoc-Dab (Dde)-OH, Fmoc-Dab(Boc)-OH, Fmoc-Thr(tBu)-OH, Fmoc-Dab(Boc)-OH, *N*, *N*-dimethylaminopentanoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Leu-OH, Fmoc-D-Phe-OH, Fmoc-Dab (Boc)-OH, Fmoc-Dab(Dde)-OH, Fmoc-Dab(Boc)-OH, Fmoc-Thr(tBu)-OH, Fmoc-Dab(Boc)-OH, *N*, *N*-dimethylaminopentanoic acid, Fmoc-Thr(tBu)-OH

2-Cl-Trt resin (0.5 mmol, degree of substitution: 0.5 mmol/g) was added into the peptide solid phase synthesis tube to prepare

*N, N-*dimethylaminovaleryl-Dab-Thr-Dab-ring(4-10)[Dab-Dab-D-Phe-Leu-Dab-Dab-Thr] according to synthetic scheme 1, 2 and 3, respectively.

Crude peptide obtained: 530 mg, yield: 89.1%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 240 mg product. Yield: 40.3% (calculated on the basis of 0.5 mmol 2-Cl-Trt resin used).

Characterization of the purified peptide: purity (area integral by HPLC profile) > 99.0%; ESI: m/z = 595.87 ([M+2H⁺]²⁺).

### Embodiment 3: Preparation of 3-oxooctanoyl-Dab-Thr-Dab-ring(4-10) [Dab-Dab-D-Phe-Leu--Dab-Dab-Thr] (Compound 5)

Synthetic scheme 1: Sequence of addition of the protected amino acid and the side chain carboxylic acid to the synthetic route is: Fmoc-Dab-OAllyl, Fmoc-Dab(Dde)-OH, Fmoc-Dab(Boc)-OH, Fmoc-Thr(tBu)-OH, Fmoc-Dab(Boc)-OH, 3-oxooctanoic acid, Fmoc-Thr(tBu)-OH, Fmoc-Dab(Boc)-OH, Fmoc-Dab(Boc)-OH, Fmoc-Leu-OH, Fmoc-D-Phe-OH

Synthetic scheme 2: Sequence of addition of the protected amino acid and the side chain carboxylic acid to the synthetic route is: Fmoc-Dab-OAllyl, Fmoc-Leu-OH, Fmoc-D-Phe-OH, Fmoc-Dab(Boc)-OH, Fmoc-Dab (Dde)-OH, Fmoc-Dab(Boc)-OH, Fmoc-Thr(tBu)-OH, Fmoc-Dab(Boc)-OH, 3-oxooctanoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Leu-OH, Fmoc-D-Phe-OH, Fmoc-Dab (Boc)-OH, Fmoc-Dab(Dde)-OH, Fmoc-Dab(Boc)-OH, Fmoc-Thr(tBu)-OH, Fmoc-Dab(Boc)-OH, 3-oxooctanoic acid, Fmoc-Thr(tBu)-OH

2-Cl-Trt resin (0.5 mmol, degree of substitution: 0.5 mmol/g) was added into the peptide solid phase synthesis tube to prepare 3-oxo-octanoyl-Dab-Thr-Dab-ring(4-10)[Dab-Dab-D-Phe-Leu--Dab-Dab-Thr] according to synthetic scheme 1, 2 and 3, respectively.

Crude peptide obtained: 540 mg, yield: 89.8%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 245 mg product. Yield: 40.7% (calculated on the basis of 0.5 mmol 2-Cl-Trt resin used).

Characterization of the purified peptide: purity (area integral by HPLC profile) > 99.0%; ESI: m/z = 602.36 ([M+2H⁺]²⁺).

### Embodiment 4: Preparation of 4-phenoxybenzoyl-Dab-Thr-Dab-ring (4-10) [Dab-Dab-D-Phe-Leu-Dab-Dab-Thr] (Compound 8)

Synthetic scheme 1: Sequence of addition of the protected amino acid and the side chain carboxylic acid to the synthetic route is: Fmoc-Dab-OAllyl, Fmoc-Dab(Dde)-OH, Fmoc-Dab(Boc)-OH, Fmoc-Thr(tBu)-OH, Fmoc-Dab(Boc)-OH, 4-(phenoxy)benzoic acid, Fmoc-Thr(tBu)-OH, Fmoc-Dab(Boc)-OH, Fmoc-Dab(Boc)-OH, Fmoc-Leu-OH, Fmoc-D-Phe-OH

Synthetic scheme 2: Sequence of addition of the protected amino acid and the side chain carboxylic acid to the synthetic route is: Fmoc-Dab-OAllyl, Fmoc-Leu-OH, Fmoc-D-Phe-OH, Fmoc-Dab(Boc)-OH, Fmoc-Dab (Dde)-OH, Fmoc-Dab(Boc)-OH, Fmoc-Thr(tBu)-OH, Fmoc-Dab(Boc)-OH, 4-(phenoxy)benzoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Leu-OH, Fmoc-D-Phe-OH, Fmoc-Dab (Boc)-OH, Fmoc-Dab(Dde)-OH, Fmoc-Dab(Boc)-OH, Fmoc-Thr(tBu)-OH, Fmoc-Dab(Boc)-OH, 4-(phenoxy)benzoic acid, Fmoc-Thr(tBu)-OH

2-Cl-Trt resin (0.5 mmol, degree of substitution: 0.5 mmol/g) was added into the peptide solid phase synthesis tube to prepare 4-phenoxy benzoyl-Dab-Thr-Dab-ring(4-10)[Dab-Dab-D-Phe-Leu--Dab-Dab-Thr] according to synthetic scheme 1, 2 and 3, respectively.

Crude peptide obtained: 570 mg, yield: 90.5%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 280 mg product. Yield: 44.5% (calculated on the basis of 0.5 mmol 2-Cl-Trt resin used).

Characterization of the purified peptide: purity (area integral by HPLC profile) > 99.0%; ESI: m/z = 630.35 ([M+2H⁺]²⁺).

### Embodiment 5: Preparation of (S)-6-methyloctanoyl-Dab-Thr-Dab-ring (4-10) [Dab-Dab-D-Phe(4-Cl)-Leu-Dab-Dab-Thr] (Compound 16)

Synthetic scheme 1: Sequence of addition of the protected amino acid and the side chain carboxylic acid to the synthetic route is: Fmoc-Dab-OAllyl, Fmoc-Dab(Dde)-OH, Fmoc-Dab(Boc)-OH, Fmoc-Thr(tBu)-OH, Fmoc-Dab(Boc)-OH, (S)-6-methyloctanoic acid, Fmoc-Thr(tBu)-OH, Fmoc-Dab(Boc)-OH, Fmoc-Dab(Boc)-OH, Fmoc-Leu-OH, Fmoc-D-Phe(4-Cl)-OH

Synthetic scheme 2: Sequence of addition of the protected amino acid and the side chain carboxylic acid to the synthetic route is: Fmoc-Dab-OAllyl, Fmoc-Leu-OH, Fmoc-D-Phe(4-Cl)-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, (S)-6-methyloctanoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Leu-OH, Fmoc-D-Phe(4-Cl)-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, (S)-6-methyloctanoic acid, Fmoc-Thr(tBu)-OH

2-Cl-Trt resin (0.5 mmol, degree of substitution: 0.5 mmol/g) was added into the peptide solid phase synthesis tube to prepare (S)-6-methyloctanoyl-Dab-Thr-Dab-ring(4-10) [Dab-Dab-D-Phe(4-Cl)-Leu-Dab-Dab-Thr] according to synthetic scheme 1, 2 and 3, respectively.

Crude peptide obtained: 560 mg, yield: 90.5%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 250 mg product. Yield: 40.4% (calculated on the basis of 0.5 mmol 2-Cl-Trt resin used).

Characterization of the purified peptide: purity (area integration by HPLC profile) > 99.0%; ESI: m/z = 619.36 ([M+2H⁺]²⁺).

### Embodiment 6: Preparation of (S)-6-methyloctanoyl-Dab-Thr-Dab-ring(4-10) [Dab-Dab-D-Phe(4-CH₃)-Leu-Dab-Dab-Thr] (Compound 26)

Synthetic scheme 1: Sequence of addition of the protected amino acid and the side chain carboxylic acid to the synthetic route is: Fmoc-Dab-OAllyl, Fmoc-Dab(Dde)-OH, Fmoc-Dab(Boc)-OH, Fmoc-Thr(tBu)-OH, Fmoc-Dab(Boc)-OH, (S)-6-methyloctanoic acid, Fmoc-Thr(tBu)-OH, Fmoc-Dab(Boc)-OH, Fmoc-Dab(Boc)-OH, Fmoc-Leu-OH, Fmoc-D-Phe(4-CH₃)-OH

Synthetic scheme 2: Sequence of addition of the protected amino acid and the side chain carboxylic acid to the synthetic route is: Fmoc-Dab-OAllyl, Fmoc-Leu-OH, Fmoc-D-Phe(4-CH₃)-OH, Fmoc-Dab (Boc)-OH, Fmoc-Dab(Dde)-OH, Fmoc-Dab(Boc)-OH, Fmoc-Thr(tBu)-OH, Fmoc-Dab(Boc)-OH, (S)-6-methyloctanoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Leu-OH, Fmoc-D-Phe(4-CH₃)-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, (S)-6-methyloctanoic acid, Fmoc-Thr(tBu)-OH

2-Cl-Trt resin (0.5 mmol, degree of substitution: 0.5 mmol/g) was added into the peptide solid phase synthesis tube to prepare (S)-6-methyloctanoyl-Dab-Thr-Dab-ring(4-10)[Dab-Dab--D-Phe(4-CH₃)-Leu-Dab-Dab-Thr] according to synthetic scheme 1, 2 and 3, respectively.

Crude peptide obtained: 550 mg, yield: 90.3%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 250 mg product. Yield: 41.1% (calculated on the basis of 0.5 mmol 2-Cl-Trt resin used).

Characterization of the purified peptide: purity (area integral by HPLC profile) > 99.0%; ESI: m/z = 609.39 ([M+2H⁺]²⁺).

### Embodiment 7: Preparation of Octanoyl-Dab-Thr-D-Dab-ring(4-10) [Dab-Dab-D-Leu-Thr-Dab-Dab-Thr] (Compound 32)

Synthetic scheme 1: Sequence of addition of the protected amino acid and the side chain carboxylic acid to the synthetic route is: Fmoc-Dab-OAllyl, Fmoc-Dab(Dde)-OH, Fmoc-D-Dab(Boc)-OH, Fmoc-Thr(tBu)-OH, Fmoc-Dab(Boc)-OH, octanoic acid, Fmoc-Thr(tBu)-OH, Fmoc-Dab(Boc)-OH, Fmoc-Dab(Boc)-OH, Fmoc-Thr(tBu)-OH, Fmoc-D-Leu-OH

Synthetic scheme 2: Sequence of addition of the protected amino acid and the side chain carboxylic acid to the synthetic route is: Fmoc-Dab-OAllyl, Fmoc-Thr(tBu)-OH, Fmoc-D-Leu-OH, Fmoc-Dab(Boc)-OH, Fmoc-Dab(Dde)-OH, Fmoc-D-Dab(Boc)-OH, Fmoc-Thr(tBu)-OH, Fmoc-Dab(Boc)-OH, octanoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Thr(tBu)-OH, Fmoc-D-Leu-OH, Fmoc-Dab(Boc)-OH, Fmoc-Dab(Dde)-OH, Fmoc-D-Dab(Boc)-OH, Fmoc-Thr(tBu)-OH, Fmoc-Dab(Boc)-OH, octanoic acid, Fmoc-Thr(tBu)-OH

2-Cl-Trt resin (0.5 mmol, degree of substitution = 0.5 mmol/g) was added into the peptide solid phase synthesis tube to prepare Octanoyl-Dab-Thr-D-Dab-ring(4-10)[Dab-Dab-D-Leu--Thr-Dab-Dab-Thr]according to synthetic scheme 1, 2 and 3, respectively.

Crude peptide obtained: 520 mg, yield: 91.0%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 248 mg product. Yield: 43.4% (calculated on the basis of 0.5 mmol 2-Cl-Trt resin used).

Characterization of the purified peptide: purity (area integration by HPLC profile) > 99.0%; ESI: m/z = 572.36 ([M+2H⁺]²⁺).

### Embodiment 8: Octanoyl-Dab-Thr-Dab-rin(4-10) [Dab-Dab-D-Phe-Leu-Dab-Dab-Thr] (Compound 44)

Synthetic scheme 1: Sequence of addition of the protected amino acid and the side chain carboxylic acid to the synthetic route is: Fmoc-Dab-OAllyl, Fmoc-Dab(Dde)-OH, Fmoc-Dab(Boc)-OH, Fmoc-Thr(tBu)-OH, Fmoc-Dab(Boc)-OH, octanoic acid, Fmoc-Thr(tBu)-OH, Fmoc-Dab(Boc)-OH, Fmoc-Dab(Boc)-OH, Fmoc-Leu-OH, Fmoc-D-Phe-OH

Synthetic scheme 2: Sequence of addition of the protected amino acid and the side chain carboxylic acid to the synthetic route is: Fmoc-Dab-OAllyl, Fmoc-Leu-OH, Fmoc-D-Phe-OH, Fmoc-Dab(Boc)-OH, Fmoc-Dab (Dde)-OH, Fmoc-Dab(Boc)-OH, Fmoc-Thr(tBu)-OH, Fmoc-Dab(Boc)-OH, octanoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Leu-OH, Fmoc-D-Phe-OH, Fmoc-Dab (Boc)-OH, Fmoc-Dab(Dde)-OH, Fmoc-Dab(Boc)-OH, Fmoc-Thr(tBu)-OH, Fmoc-Dab(Boc)-OH, octanoic acid, Fmoc-Thr(tBu)-OH

2-Cl-Trt resin (0.5 mmol, degree of substitution = 0.5 mmol/g) was added into the peptide solid phase synthesis tube to prepare Octanoyl-Dab-Thr-Dab-ring(4-10)[Dab-Dab-D-Phe-Leu-Dab-Dab-Thr] according to synthetic scheme 1, 2 and 3, respectively.

Crude peptide obtained: 540 mg, yield: 90.8%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 240 mg product. Yield: 40.4% (calculated on the basis of 0.5 mmol 2-Cl-Trt resin used).

Characterization of the purified peptide: purity (area integral by HPLC profile) > 99.0%; ESI: m/z = 595.37 ([M+2H⁺]²⁺).

### Example 9: Octanoyl-Dab-Thr-D-Ser-Ring(4-10) [Dab-Dab-D-Leu-Thr-Dab-Dab-Thr] (Compound 60)

Synthetic scheme 1: Sequence of addition of the protected amino acid and the side chain carboxylic acid to the synthetic route is: Fmoc-Dab-OAllyl, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH, Fmoc-Dab(Boc)-OH, Fmoc-Dab(Boc)-OH, Fmoc-Thr(tBu)-OH, Fmoc-D-Leu-OH

Synthetic scheme 2: Sequence of addition of the protected amino acid and the side chain carboxylic acid to the synthetic route is: Fmoc-Dab-OAllyl, Fmoc-Thr(tBu)-OH, Fmoc-D-Leu-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Thr(tBu)-OH, Fmoc-D-Leu-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH

2-Cl-Trt resin (0.5 mmol, degree of substitution = 0.5 mmol/g) was added into the peptide solid phase synthesis tube to prepare Octanoyl-Dab-Thr-D-Ser-ring(4-10)[Dab-Dab-D-Leu-Thr--Dab-Dab-Thr] according to synthetic scheme 1, 2 and 3, respectively.

Crude peptide obtained: 510 mg, yield: 90.2%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 230 mg product. Yield: 40.7% (calculated on the basis of 0.5 mmol 2-Cl-Trt resin used).

Characterization of the purified peptide: purity (area integral by HPLC profile) > 99.0%; ESI: m/z = 565.85 ([M+2H⁺]²⁺).

### Embodiment 10: Octanoyl-Dab-Thr-Dab-ring(4-10) [Dab-Dab-D-Leu-Leu-Dab-Dab-Thr] (Compound 78)

Synthetic scheme 1: Sequence of addition of the protected amino acid and the side chain carboxylic acid to the synthetic route is: Fmoc-Dab-OAllyl, Fmoc-Dab(Dde)-OH, Fmoc-Dab(Boc)-OH, Fmoc-Thr(tBu)-OH, Fmoc-Dab(Boc)-OH, octanoic acid, Fmoc-Thr(tBu)-OH, Fmoc-Dab(Boc)-OH, Fmoc-Dab(Boc)-OH, Fmoc-Leu-OH, Fmoc-D-Leu-OH

Synthetic scheme 2: Sequence of addition of the protected amino acid and the side chain carboxylic acid to the synthetic route is: Fmoc-Dab-OAllyl, Fmoc-Leu-OH, Fmoc-D-Leu-OH, Fmoc-Dab(Boc)-OH, Fmoc-Dab (Dde)-OH, Fmoc-Dab(Boc)-OH, Fmoc-Thr(tBu)-OH, Fmoc-Dab(Boc)-OH, octanoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Leu-OH, Fmoc-D-Leu-OH, Fmoc-Dab (Boc)-OH, Fmoc-Dab(Dde)-OH, Fmoc-Dab(Boc)-OH, Fmoc-Thr(tBu)-OH, Fmoc-Dab(Boc)-OH, octanoic acid, Fmoc-Thr(tBu)-OH

2-Cl-Trt resin (0.5 mmol, degree of substitution = 0.5 mmol/g) was added into the peptide solid phase synthesis tube to prepare Octanoyl-Dab-Thr-Dab-ring(4-10)[Dab-Dab-D-Leu-Leu-Dab-Dab-Thr] according to synthetic scheme 1, 2 and 3, respectively.

Crude peptide obtained: 540 mg, yield: 93.5%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 240 mg product. Yield: 41.5% (calculated on the basis of 0.5 mmol 2-Cl-Trt resin used).

Characterization of the purified peptide: purity (area integral by HPLC profile) > 99.0%; ESI: m/z = 578.38 ([M+2H⁺]²⁺).

### Embodiment 11: Preparation of Octanoyl-Dab-Thr-Dab-ring(4-10) [Dab-Dab-D-Leu-Thr--Dab-Dab-Thr] (Compound 104)

Synthetic scheme 1: Sequence of addition of the protected amino acid and the side chain carboxylic acid to the synthetic route is: Fmoc-Dab-OAllyl, Fmoc-Dab(Dde)-OH, Fmoc-Dab(Boc)-OH, Fmoc-Thr(tBu)-OH, Fmoc-Dab(Boc)-OH, octanoic acid, Fmoc-Thr(tBu)-OH, Fmoc-Dab(Boc)-OH, Fmoc-Dab(Boc)-OH, Fmoc-Thr(tBu)-OH, Fmoc-D-Leu-OH

Synthetic scheme 2: Sequence of addition of the protected amino acid and the side chain carboxylic acid to the synthetic route is: Fmoc-Dab-OAllyl, Fmoc-Thr(tBu)-OH, Fmoc-D-Leu-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, octanoic acid, Fmoc-Thr(tBu)-OH, Fmoc-Dab (Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Thr(tBu)-OH, Fmoc-D-Leu-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, octanoic acid, Fmoc-Thr (tBu)-OH

2-Cl-Trt resin (0.5 mmol, degree of substitution = 0.5 mmol/g) was added into the peptide solid phase synthesis tube to prepare Octanoyl-Dab-Thr-Dab-ring(4-10)[Dab-Dab-D-Leu-Thr-Dab-Dab-Thr] according to synthetic scheme 1, 2 and 3 respectively.

Crude peptide obtained: 525 mg, yield: 91.8%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 250 mg product. Yield: 43.7% (calculated on the basis of 0.5 mmol 2-Cl-Trt resin used).

Characterization of the purified peptide: purity (area integration by HPLC profile) > 99.0%; ESI: m/z = 572.36 ([M+2H⁺]²⁺).

### Embodiment 12: Preparation of octanoyl-Dab-Thr-D-Ser-Ring (4-10) [Dab-Dab-D-Phe-Thr--Dab-Dab-Thr] (Compound 117)

Synthetic scheme 1: Sequence of addition of the protected amino acid and the side chain carboxylic acid to the synthetic route is: Fmoc-Dab-OAllyl, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH, Fmoc-Dab(Boc)-OH, Fmoc-Dab(Boc)-OH, Fmoc-Thr(tBu)-OH, Fmoc-D-Phe-OH

Synthetic scheme 2: Sequence of addition of the protected amino acid and the side chain carboxylic acid to the synthetic route is: Fmoc-Dab-OAllyl, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH

2-Cl-Trt resin (0.5 mmol, degree of substitution = 0.5 mmol/g) was added into the peptide solid phase synthesis tube to prepare Octanoyl-Dab-Thr-D-Ser-ring(4-10)[Dab-Dab-D-Phe--Thr-Dab-Dab-Thr] according to synthetic scheme 1, 2 and 3, respectively.

Crude peptide obtained: 530 mg, yield: 91.0%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 240 mg product. Yield: 41.2% (calculated on the basis of 0.5 mmol 2-Cl-Trt resin used).

Characterization of the purified peptide: purity (area integration by HPLC profile) > 99.0%; ESI: m/z = 582.84 ([M+2H⁺]²⁺).

### Embodiment 13: Preparation of octanoyl-Dab-Thr-Dab-ring(4-10) [Dab-Dab-D-Phe-Leu--Dab-Dab-Leu] (Compound 129)

Synthetic scheme 1: Sequence of addition of the protected amino acid and the side chain carboxylic acid to the synthetic route is: Fmoc-Dab-OAllyl, Fmoc-Dab(Dde)-OH, Fmoc-Dab(Boc)-OH, Fmoc-Thr(tBu)-OH, Fmoc-Dab(Boc)-OH, octanoic acid, Fmoc-Leu-OH, Fmoc-Dab(Boc)-OH, Fmoc-Dab(Boc)-OH, Fmoc-Leu-OH, Fmoc-D-Phe-OH

Synthetic scheme 2: Sequence of addition of the protected amino acid and the side chain carboxylic acid to the synthetic route is: Fmoc-Dab-OAllyl, Fmoc-Leu-OH, Fmoc-D-Phe-OH, Fmoc-Dab(Boc)-OH, Fmoc-Dab (Dde)-OH, Fmoc-Dab(Boc)-OH, Fmoc-Thr(tBu)-OH, Fmoc-Dab(Boc)-OH, octanoic acid, Fmoc-Leu-OH, Fmoc-Dab(Boc)-OH

Synthetic scheme 3: Sequence of addition of the protected amino acid and the side chain carboxylic acid to the synthetic route is: Fmoc-Dab-OAllyl, Fmoc-Dab(Boc)-OH, Fmoc-Leu-OH, Fmoc-D-Phe-OH, Fmoc-Dab (Boc)-OH, Fmoc-Dab(Dde)-OH, Fmoc-Dab(Boc)-OH, Fmoc-Thr(tBu)-OH, Fmoc-Dab(Boc)-OH, octanoic acid, Fmoc-Leu-OH

2-Cl-Trt resin (0.5 mmol, degree of substitution = 0.5 mmol/g) was added into the peptide solid phase synthesis tube to prepare Octanoyl-Dab-Thr-Dab-ring(4-10) [Dab-Dab-D-Phe--Leu-Dab-Dab-Leu] according to synthetic scheme 1, 2 and 3, respectively.

Crude peptide obtained: 550 mg, yield: 91.6%. The crude peptide obtained was dissolved in water, filtered through a 0.22 µm pore size filter, purified using preparative high performance liquid chromatography. Stationary phase: 10 µm reversed phase C₁₈, mobile phase A: 0.1% TFA/water solution, mobile phase B: 0.1% TFA/acetonitrile solution, column dimentions: 22mm × 250mm, mobile phase flow rate: 10mL / min, detection wavelength: 215 nm, gradient elution and cycle injection purification were used. The crude solution was injected into the column, started elution, collecting the fraction corresponding to the main peak in the chromatogram. Acetonitrile was evaporated from the solution to obtain an aqueous solution of the polymyxin derivative. The solution was lyophilized to obtain 250 mg product. Yield: 41.6% (calculated on the basis of 0.5 mmol 2-Cl-Trt resin used).

Characterization of the purified peptide: purity (area integration by HPLC profile) > 99.0%; ESI: m/z = 601.39 ([M+2H⁺]²⁺).

### Experimental example 1: Experiments on Antibacterial Activity

The minimum inhibitory concentration (MIC) was determined by means of dish double dilution method using a Multipoint inoculator according to the CLSI recommended method. The compounds of the present invention (for example, the compounds prepared in the Embodiments) and the reference substances were diluted twice each time with the broth into various desired concentrations, and appropriate amounts were added to the dishes. Agar medium is melted, and then quantitatively injected into the dish containing the drug solution, and mixed. The final concentrations of the compounds of the invention (e.g., the compounds prepared in the Embodiments) and the controls were 0.03, 0.06, 0.125, 0.25... 128 µg/mL, respectively. The test bacteria were cultured overnight with nutrient broth, brain heart infusion or HTM broth. During the tests, the bacterial solutions were diluted appropriately, and the test bacteria (inoculation amount 10⁴ CFU/dot) were inoculated on the surface of the drug-containing agar by a multi-point inoculator. After drying, the bacterial was incubated for 18 to 24 hours at 35°C, the results were observed, the minimum concentrations of the compounds of the present invention (for example, the compounds prepared in the Embodiments) and the controls contained in the dishes with no growth of colonies were MICs.

The strains used in the antibacterial activity experiments were from the American Type Culture Collection (ATCC) and clinical isolates.

The strains used for the experiments on antibacterial activity included Escherichia coli ATCC 25922, Klebsiella pneumoniae ATCC BAA-2146 (NDM-1), Pseudomonas aeruginosa ATCC 27853, Acinetobacter baumannii ATCC 19606 and Staphylococcus epidermidis ATCC 12228.
Tested samples: polymyxin derivatives prepared according to the technical scheme of the present invention;
Controls: polymyxin B sulfate and colistin (polymyxin E sulfate).

**Table 2 Activity of some compounds prepared accrding to this invention against Gram-negative and-positive bacteria (MIC, unit µg/mL)**

| Compound (µg/mL) | E. coli ATCC 25922 | K. pneumoniae ATCC BAA-2146 (NDM-1) | P. aeruginosa ATCC 27853 | A. baumannii ATCC 19606 | S. epidermidis ATCC 12228 |
|---|---|---|---|---|---|
| 1 | 1 | 4 | 1 | 2 | >128 |
| 2 | 16 | 128 | 1 | 8 | >128 |
| 3 | 0.5 | 2 | 2 | 8 | 128 |
| 4 | 0.5 | 2 | 2 | 2 | 128 |
| 5 | 0.5 | 0.5 | 1 | 2 | 32 |
| 6 | 1 | 1 | 2 | 2 | 16 |
| 7 | 2 | 2 | 4 | 2 | 32 |
| 8 | 4 | 4 | 8 | 2 | 16 |
| 9 | 4 | 4 | 4 | 4 | 8 |
| 11 | 1 | 2 | 2 | 1 | 128 |
| 12 | 2 | 2 | 1 | 2 | 128 |
| 13 | 0.5 | 1 | 2 | 0.25 | 16 |
| 14 | 1 | 2 | 2 | 1 | 8 |
| 16 | 1 | 2 | 2 | 1 | 16 |
| 18 | 1 | 2 | 2 | 0.5 | 4 |
| 19 | 1 | 2 | 2 | 0.5 | 4 |
| 23 | 1 | 2 | 2 | 1 | 8 |
| 24 | 1 | 2 | 2 | 1 | 16 |
| 25 | 1 | 2 | 2 | 2 | 16 |
| 26 | 1 | 2 | 2 | 0.5 | 8 |
| 27 | 2 | 4 | 4 | 8 | 8 |
| 29 | 2 | 2 | 4 | 1 | 16 |
| 30 | 0.5 | 1 | 1 | 1 | >128 |
| 31 | 0.5 | 0.5 | 1 | 0.5 | >128 |
| 32 | 0.5 | 0.5 | 1 | 0.5 | >128 |
| 42 | 0.5 | 0.5 | 2 | 1 | 32 |
| 43 | 0.5 | 1 | 2 | 0.5 | 64 |
| 44 | 0.5 | 1 | 1 | 0.5 | 32 |
| 45 | 0.5 | 0.5 | 1 | 0.5 | 128 |
| 46 | 1 | 2 | 2 | 1 | 32 |
| 47 | 1 | 1 | 2 | 1 | 128 |
| 54 | 0.5 | 1 | 1 | 0.5 | 64 |
| 58 | 0.12 | 0.5 | 2 | 0.06 | >128 |
| 59 | 0.25 | 0.5 | 2 | 0.12 | >128 |
| 60 | 0.12 | 0.5 | 2 | 0.06 | >128 |
| 70 | 1 | 1 | 2 | 1 | 64 |
| 71 | 1 | 1 | 2 | 1 | 128 |
| 72 | 0.5 | 0.5 | 2 | 0.5 | 128 |
| 73 | 1 | 1 | 1 | 1 | 128 |
| 74 | 0.5 | 1 | 2 | 0.5 | 64 |
| 75 | 1 | 1 | 2 | 1 | 128 |
| 76 | 0.5 | 0.5 | 2 | 1 | >128 |
| 77 | 0.5 | 0.5 | 1 | 0.5 | >128 |
| 78 | 0.5 | 0.5 | 1 | 0.5 | 32 |
| 79 | 0.5 | 1 | 1 | 0.5 | >128 |
| 80 | 0.5 | 0.5 | 1 | 1 | >128 |
| 81 | 0.5 | 1 | 1 | 1 | >128 |
| 82 | 1 | 0.5 | 1 | 2 | >128 |
| 83 | 0.5 | 1 | 1 | 1 | >128 |
| 86 | 1 | 1 | 2 | 1 | 64 |
| 98 | 0.5 | 1 | 1 | 0.5 | >128 |
| 102 | 1 | 0.25 | 1 | 0.5 | >128 |
| 103 | 0.5 | 0.5 | 0.5 | 1 | >128 |
| 104 | 0.25 | 0.5 | 1 | 0.5 | 64 |
| 109 | 1 | 2 | 1 | 16 | >128 |
| 111 | 0.5 | 0.5 | 1 | 1 | 64 |
| 115 | 0.25 | 0.5 | 2 | 0.12 | >128 |
| 116 | 0.25 | 1 | 2 | 0.25 | >128 |
| 117 | 0.5 | 0.5 | 2 | 0.25 | >128 |
| 127 | 4 | 4 | 4 | 2 | 8 |
| 128 | 4 | 4 | 4 | 4 | 16 |
| 129 | 4 | 4 | 4 | 4 | 8 |
| 143 | 0.5 | 1 | 1 | 1 | >128 |
| 144 | 0.5 | 1 | 1 | 1 | >128 |
| polymyxin B | 1 | 1 | 2 | 0.5 | 64 |
| colistin | 1 | 1 | 2 | 2 | 64 |

### Experimental Example 2: Nephrotoxicity test

African green monkey kidney cells (Vero cells) were cultured in MEM medium (Hyclone), 10% fetal calf serum (Invitrogen) was added before use, cultured at 37°C under 5% CO2.

Experiment was carried out using MTT method. After digestion, cells in logarithmic growth phase were counted, then the cells were inoculated in a 96-well culture plate. After incubation for 24 h to be adherent, cells were treated with a concentration gradient of a compound of the invention (e.g., a compound prepared in the Embodiments) and the controls. After 72 h, the culture solution was removed, 100 uL of MTT reagent at a concentration of 0.5 mg/ml was added, the medium was removed after incubating for 3 hours in a 37°C incubator. Add 150 µL DMSO solvent to each well, mix for 3 min, after whcih the absorbance at 570 nm (A) was measured with a microplate reader.

Cell viability%=(A_{dosed cell}-A_{background})/(A_{control cell}-A_{background})×100%. The average value of 3 parallel wells was taken for each detection point, and the inhibition curve was drawn to calculate the IC₅₀ value.

The African green monkey kidney cells (Vero cells) used in the experiment were from the Cell Resource Center of the Institute of Basic Medicine, Chinese Academy of Medical Sciences.

**Table 3 Renal cytotoxicity (IC₅₀, µg/mL) of part of the compounds prepared in this invention**

| compound(µg/mL) | Vero cells |
|---|---|
| 4 | 109.61 ±9.08 |
| 5 | 93.70 ±7.38 |
| 11 | 166.38 ±15.67 |
| 13 | 185.75 ±11.31 |
| 30 | 144.90 ±12.09 |
| 31 | 287.90 ±23.28 |
| 42 | 71.29 ± 6.08 |
| 43 | 159.10 ± 14.14 |
| 44 | 86.40 ± 8.31 |
| 45 | 160.05 ± 13.59 |
| 46 | 33.15 ±2.64 |
| 47 | 74.72 ±6.13 |
| 58 | 189.34 ± 11.34 |
| 59 | 318.00 ± 25.55 |
| 71 | 176.2 ±14.72 |
| 72 | 130.9 ±8.69 |
| 73 | 108.5 ±9.16 |
| 75 | 198.7 ±13.07 |
| 76 | 225.0 ±17.02 |
| 77 | 168.4 ±12.46 |
| 82 | 215.9 ±16.09 |
| 86 | 87.03 ±6.98 |
| 103 | 276.10 ± 17.72 |
| 115 | 160.31 ± 23.39 |
| 116 | > 500 |
| 127 | 17.19 ± 2.16 |
| 128 | 30.07 ±2.59 |
| 143 | 141.50 ±15.19 |
| polymyxin B | 71.65±5.85 |
| colistin | 128.13 ± 14.66 |

In summary, part of the polymyxin derivatives prepared by the invention have low nephrotoxicity and high antibacterial activity, they are quite possible to become a new class of clinical antibiotics.

## Claims

1. The polymyxin derivative or a pharmaceutically acceptable salt thereof, the said polymyxin derivative is the following compound:
(116) 6-methylheptanoyl-Dab-Thr-D-Ser-ring(4-10)[Dab-Dab-D-Phe-Thr-Dab-Dab-Thr]

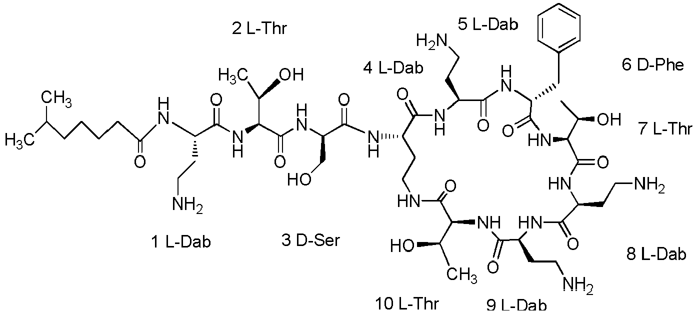

2. A pharmaceutical composition comprising a polymyxin derivative according to claim 1, or a pharmaceutically acceptable salt thereof, and a pharmaceutically acceptable carrier or excipient.

3. A polymyxin derivative or a pharmaceutically acceptable salt thereof according to claim 1 for use as antibacterial agent in therapy, in particular against "superbugs" carrying the NDM-1 gene.

4. A method of preparing a compound according to claim 1, comprising the steps of:
(1) the side chain free amino group in the protected basic amino acid Fmoc-Dab-OP and halogenated resin are reacted to obtain Fmoc-AA-OP-resin; P is a carboxyl protecting group, the structure of the Fmoc-Dab-OP is as shown in Formula III: 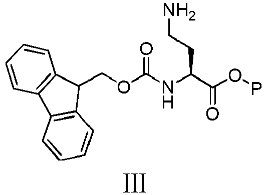
(2) Fmoc-AA-OP-resin is coupled one by one to obtain a linear polypeptide-resin;
(3) the protecting group is selectively removed from the linear polypeptide-resin, cyclized in solid phase to obtain a cyclic polypeptide-resin;
(4) the cyclic polypeptide-resin is acid hydrolysed to obtain a crude cyclic polypeptide and
(5) the crude cyclic polypeptide is purified and/or salified, and lyophilized to obtain a pure cyclic polypeptide.

5. The method according to claim 4, wherein DIC/HOBT is used as a condensing agent without adding a base as a catalyst.

6. The method according to claim 4, wherein P is allyl group or benzyl group.

## Patentansprüche

1. Polymyxinderivat oder ein pharmazeutisch annehmbares Salz davon, wobei das Polymyxinderivat die folgende Verbindung ist:
(116) 6-Methylheptanoyl-Dab-Thr-D-Ser-ring(4-10)[Dab-Dab-D-Phe-Thr-Dab-Dab-Thr]

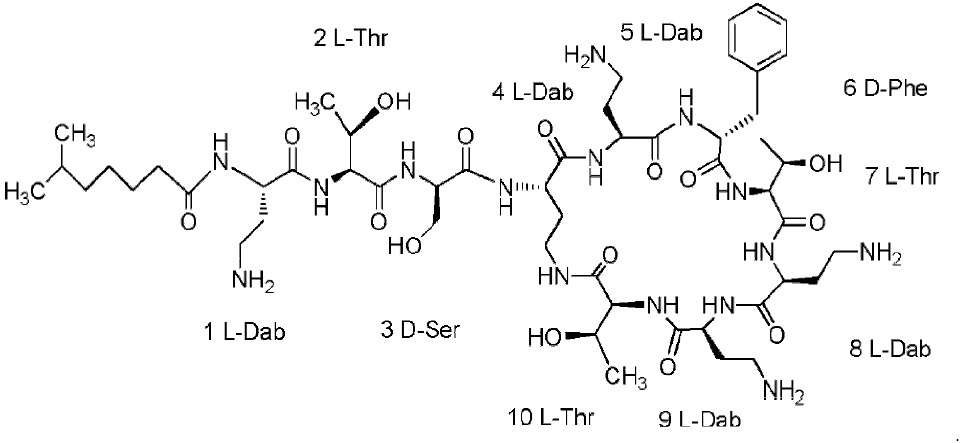

2. Pharmazeutische Zusammensetzung, umfassend ein Polymyxinderivat nach Anspruch 1 oder ein pharmazeutisch annehmbares Salz davon, und einen pharmazeutisch annehmbaren Träger oder Hilfsstoff.

3. Polymyxinderivat oder ein pharmazeutisch annehmbares Salz davon nach Anspruch 1 zur Verwendung als antibakterielles Mittel in der Therapie, insbesondere gegen "Superbugs", die das NDM-1-Gen tragen.

4. Verfahren zum Herstellen einer Verbindung nach Anspruch 1, umfassend die folgenden Schritte:
(1) die freie Aminogruppe der Seitenkette in der geschützten basischen Aminosäure Fmoc-Dab-OP und halogeniertes Harz werden umgesetzt, um Fmoc-AA-OP-Harz zu erhalten; P ist eine Carboxylschutzgruppe, die Struktur des Fmoc-Dab-OP ist wie in Formel III gezeigt: 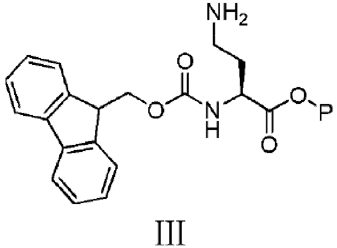
(2) Fmoc-AA-OP-Harz wird einzeln gekoppelt, um ein lineares Polypeptid-Harz zu erhalten;
(3) die Schutzgruppe wird selektiv von dem linearen Polypeptidharz entfernt, und in fester Phase zyklisiert, um ein zyklisches Polypeptidharz zu erhalten;
(4) das zyklische Polypeptidharz wird säurehydrolysiert, um ein rohes zyklisches Polypeptid zu erhalten, und
(5) das rohe zyklische Polypeptid wird gereinigt und/oder in ein Salz überführt und lyophilisiert, um ein reines zyklisches Polypeptid zu erhalten.

5. Verfahren nach Anspruch 4, wobei DIC/HOBT als Kondensationsmittel verwendet wird, ohne eine Base als Katalysator hinzuzufügen.

6. Verfahren nach Anspruch 4, wobei P eine Allylgruppe oder Benzylgruppe ist.

## Revendications

1. Dérivé de polymyxine ou sel pharmaceutiquement acceptable de celui-ci, ledit dérivé de polymyxine est le composé suivant :
(116) 6-méthylheptanoyl-Dab-Thr-D-Ser-cycle(4-10)[Dab-Dab-D-Phe-Thr-Dab-Dab-Thr]

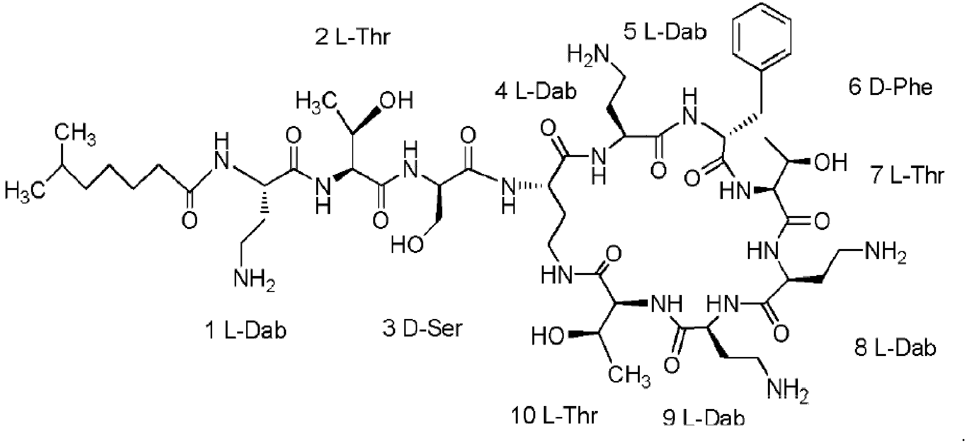

2. Composition pharmaceutique comprenant un dérivé de polymyxine selon la revendication 1, ou un sel pharmaceutiquement acceptable de celui-ci, et un véhicule ou excipient pharmaceutiquement acceptable.

3. Dérivé de polymyxine ou sel pharmaceutiquement acceptable de celui-ci selon la revendication 1 pour une utilisation comme agent antibactérien en thérapie, en particulier contre les « superbactéries » porteuses du gène NDM-I.

4. Procédé de préparation d'un composé selon la revendication 1, comprenant les étapes de :
(1) mise en réaction du groupe aminé libre à chaîne latérale dans l'acide aminé basique protégé Fmoc-Dab-OP et de la résine halogénée pour obtenir la résine Fmoc-AA-OP ; P est un groupe protecteur de carboxyle, la structure du Fmoc-Dab-OP est celle représentée dans la formule III : 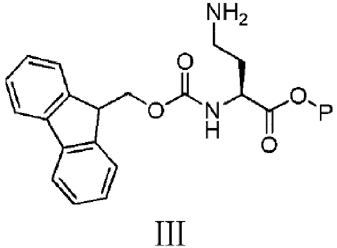
(2) couplage de la résine Fmoc-AA-OP une par une pour obtenir une résine polypeptidique linéaire ;
(3) élimination sélective du groupe protecteur de la résine polypeptidique linéaire, et cyclisation en phase solide pour obtenir une résine polypeptidique cyclique ;
(4) hydrolyse acide de la résine polypeptidique cyclique pour obtenir un polypeptide cyclique brut et
(5) purification et/ou salification, et lyophilisation du polypeptide cyclique brut pour obtenir un polypeptide cyclique pur.

5. Procédé selon la revendication 4, dans lequel du DIC/HOBT est utilisé comme agent de condensation sans ajouter une base comme catalyseur.

6. Procédé selon la revendication 4, dans lequel P est un groupe allyle ou un groupe benzyle.
